# EUROPEAN PATENT SPECIFICATION

(11) **EP 3 772 927 B1**
(45) Date of publication and mention of the grant of the patent: **15.01.2025**
(21) Application number: 19716639.0
(22) Date of filing: 22.03.2019
(51) Int. Cl.: A01K 67/0275, C07K 16/00, C07K 14/74, C07K 16/28

(54) **GENETICALLY MODIFIED MICE OR RATS FOR GENERATING THERAPEUTIC ANTIBODIES AGAINST PEPTIDE-MHC COMPLEXES, METHODS OF MAKING AND USES THEREOF**
GENETISCH MODIFIZIERTE MÄUSE ODER RATTEN ZUR ERZEUGUNG THERAPEUTISCHER ANTIKÖRPER GEGEN PEPTID-MHC-KOMPLEXE, VERFAHREN ZU DEREN HERSTELLUNG UND DEREN VERWENDUNGEN
SOURIS OU RATS GÉNÉTIQUEMENT MODIFIÉS POUR PRODUIRE DES ANTICORPS THÉRAPEUTIQUES CONTRE DES COMPLEXES PEPTIDE-MHC, PROCÉDÉS DE FABRICATION ET UTILISATIONS DE CEUX-CI

(30) Priority: 24.03.2018 US 201862647720 P; 24.03.2018 US 201862647724 P
(43) Date of publication of application: 17.02.2021
(60) Divisional application: 24218668.2
(73) Proprietor: Regeneron Pharmaceuticals, Inc., Tarrytown, NY 10591 (US)
(72) Inventor: MURPHY, Andrew J., New York 10591 (US)
(74) Representative: J A Kemp LLP
(86) International application number: PCT/US2019/023609
(87) International publication number: WO 2019/190922

(56) References cited:
- WO-A1-2006/056769
- WO-A1-2013/096142
- WO-A1-2013/116609
- WO-A1-2016/044745
- WO-A1-2016/149678
- JUSTIN A. SPANIER ET AL: "Efficient generation of monoclonal antibodies against peptide in the context of MHCII using magnetic enrichment", NATURE COMMUNICATIONS, vol. 7, no. 1, September 2016 (2016-09-01), XP055590261, DOI: 10.1038/ncomms11804
- LAURENT VERKOCZY ET AL: "Human Ig knockin mice to study the development and regulation of HIV-1 broadly neutralizing antibodies", IMMUNOLOGICAL REVIEWS., vol. 275, no. 1, 2017, US, pages 89 - 107, XP055371222, ISSN: 0105-2896, DOI: 10.1111/imr.12505

## Description

### TECHNICAL FIELD

Disclosed herein are genetically modified rodents that are tolerized to a human(ized) MHC molecule (e.g., but not limited to an empty human(ized) MHC molecule), or the peptide binding portion thereof (e.g., the peptide binding groove of an MHC molecule), such that the rodents may generate a robust B cell response to the human(ized) MHC molecule when it is presenting, e.g., is complexed with, a peptide that is foreign to the rodent, e.g., when it is part of a peptide/MHC (pMHC) complex, wherein the peptide is heterologous to the rodent. Such animals may be useful in generating therapeutic antigen-binding proteins against pathogenic pMHC complexes, e.g., autoimmunogenic human self-peptides presented in the context of human HLA.

### RELATED APPLICATIONS

This application claims the benefit of priority to U.S. Provisional Patent Application Ser. No. 62/647,720, filed March 24, 2018, and U.S. Provisional Patent Application Ser. No. 62/647,724, filed March 24, 2018.

### BACKGROUND OF THE INVENTION

Although T cells play important roles in adaptive anti-infection or antitumor responses, they also play roles in maladaptive immune responses such as autoimmune and transplant rejection responses. Generally, a T-cell mediated immune response involves close contact between a T cell and an antigen presenting cell (APC). The pairing of several molecules are involved in the formation of the immunological synapse that triggers T-cell activity, including, but not limited to, (a) a T-cell receptor (TCR) on a T cell, which specifically binds to a peptide presented in the peptide binding groove of a major histocompatibility complex (MHC) molecule on an APC, and (b) CD28 (on the T cell), which pairs with a B7 molecule on the APC. A TCR, together with CD3 molecules, form a TCR complex, and upon pairing of the TCR to the peptide-MHC (pMHC) complex, a signal is sent through CD3. Signaling through both the TCR complex and CD28 on the T cell results in activation of the T cell.

Immunotherapeutic approaches to treating diseases work to regulate T cell activity *in vivo,* e.g., to downregulate autoimmune and transplant rejection responses, etc. However, such methods typically lack specificity since many immunotherapies target signaling by the TCR complex by binding CD3 and/or the pairing of costimulatory molecules, etc. Such approaches often result in undesirable side effects, e.g., a hyperactive immune response or generalized immune suppression. Accordingly, therapies that take advantage of the unique interaction between a TCR and pMHC complex may provide the ability to modulate the activity of specific T cells *in vivo,* and provide new treatments based on T cell modulation.

### SUMMARY OF THE INVENTION

Disclosed are non-human animals (i.e. rats or mice) that are genetically modified and tolerized against human HLA molecules and/or β2 microglobulin and are also capable of producing human or humanized antibodies. Both (1) the tolerance by these rats or mice to human HLA molecules, and (2) the ability of these rats or mice to provide humanized antigen-binding proteins allow them to be used as a unique platform for the isolation of cells that may be useful in *in vitro* studies, e.g., of allogeneic reactions, and/or for the production of human or humanized antigen-binding proteins that bind specifically to a peptide-MHC complex of interest, which antigen-binding proteins are uniquely poised to be useful therapeutics. Accordingly, also described herein are methods of making and using the rats or mice disclosed herein, and cells, tissues, nucleic acids, and antigen-binding proteins isolated therefrom. The term "non-human animal" used throughout the description must be construed as meaning a rat or a mouse in the context of the invention.

The present invention provides a genetically modified rodent whose germline genome comprises:
(a) a nucleotide sequence encoding a human or humanized MHC molecule that comprises at least a human peptide-binding groove of a human leukocyte antigen (HLA) molecule or at least the human peptide-binding groove thereof; and
(b)
   (i) an unrearranged human or humanized immunoglobulin heavy chain locus and/or
   (ii) an unrearranged human or humanized immunoglobulin light chain locus,

   wherein the genetically modified rodent expresses the human or humanized MHC molecule or at least the human peptide-binding groove thereof,
   wherein the genetically modified rodent expresses in a B-cell a human or humanized antibody comprising:
      (i) a human or humanized heavy chain variable domain, expressed from a rearranged immunoglobulin heavy chain variable region locus formed from said unrearranged human or humanized immunoglobulin heavy chain locus; and/or
      (ii) a human or humanized light chain variable domain, expressed from a rearranged immunoglobulin light chain variable region locus formed from said unrearranged human or humanized immunoglobulin light chain locus,
   wherein the genetically modified rodent is tolerized to:
      the human or humanized MHC molecule or at least the human peptide-binding groove thereof, such that it generates a specific B-cell response that generates a human or humanized antibody that:
      (A) specifically binds an antigenic peptide-MHC (pMHC) complex that comprises
         (i) a peptide that is heterologous to the genetically modified rodent complexed with
         (ii) the HLA molecule or at least the human peptide-binding groove thereof, and
      (B) does not bind an empty HLA molecule from which the human peptide-binding groove is derived,
      when immunized with said antigenic pMHC complex, and
   wherein the genetically modified rodent further comprises:
(c) said antigenic pMHC complex that comprises
   (i) said peptide that is heterologous to the genetically modified rodent complexed with
   (ii) said HLA molecule or at least the human peptide-binding groove thereof, and
(d) said human or humanized antibody that specifically binds said antigenic pMHC complex and does not bind the empty HLA molecule, and
wherein the genetically modified rodent is a rat or a mouse.

The invention further provides a method of making a genetically modified rodent comprising:
(1) modifying the genome of a rodent to comprise:
   (a) a nucleotide sequence encoding a human or humanized MHC molecule that comprises at least a human peptide-binding groove of an HLA molecule or at least the human peptide-binding groove thereof, and
   (b) an unrearranged human or humanized immunoglobulin heavy chain locus and/or an unrearranged human or humanized immunoglobulin light chain locus, and
(2) administering to the rodent an antigenic pMHC complex that comprises a peptide heterologous to the rodent associated with said HLA molecule or at least the human peptide-binding groove thereof,

wherein the genetically modified rodent is:
   A. tolerized to the human or humanized MHC molecule or at least the human peptide-binding groove thereof such that it generates a specific B-cell response that generates a human or humanized antibody that (A) specifically binds an antigenic pMHC complex that comprises (i) a peptide that is heterologous to the genetically modified rodent complexed with (ii) the HLA molecule or at least the human peptide-binding groove thereof, and (B) and does not bind an empty HLA molecule from which the human peptide-binding groove is derived, when immunized with said antigenic pMHC complex, and
   B. capable of expressing from a B-cell said human or humanized antibody which comprises
      (i) a human or humanized heavy chain variable domain, expressed from a rearranged immunoglobulin heavy chain variable region locus formed from said unrearranged human or humanized immunoglobulin heavy chain locus; and/or
      (ii) a human or humanized light chain variable domain, expressed from a rearranged immunoglobulin light chain variable region locus formed from said unrearranged human or humanized immunoglobulin light chain locus, and
wherein the rodent is a rat or a mouse.

The invention additionally provides a method of generating a human or humanized antibody that specifically binds an antigenic pMHC complex of interest or a nucleic acid sequence encoding the same comprising maintaining the genetically modified rodent according to the invention or made according to the method of the invention in conditions sufficient for the genetically modified rodent to mount an immune response to the antigenic pMHC complex of interest, wherein the antigenic pMHC complex of interest comprises a peptide that is heterologous to the genetically modified rodent and is presented in the context of the HLA molecule or at least the human peptide-binding groove thereof, optionally

comprising as a first step(s) immunizing the genetically modified rodent with the antigenic pMHC complex of interest, and optionally further boosting the immune response of the immunized genetically modified rodent, optionally wherein immunizing and/or boosting comprises administering to the genetically modified rodent the antigenic pMHC complex of interest linked to a helper T cell epitope, optionally wherein the helper T cell epitope comprises PADRE set forth as SEQ ID NO: 28.

The invention further provides a method of obtaining a nucleic acid encoding a human immunoglobulin heavy chain variable domain and/or a human immunoglobulin light chain variable domain, comprising:
isolating from a genetically modified rodent according the invention or made according to the method of the invention, a nucleic acid comprising a rearranged human immunoglobulin variable region gene sequence, optionally wherein the rearranged human immunoglobulin variable region gene sequence comprises at least one somatic hypermutation, that encodes a human immunoglobulin variable domain expressed by a lymphocyte of the genetically modified rodent, or a hybridoma produced from the lymphocyte,
wherein the human immunoglobulin variable domain expressed by the lymphocyte, or hybridoma produced therefrom, associates with its cognate variable domain to form an antigen-binding domain specific for the antigenic pMHC complex.

The invention also provides a method of obtaining a cell that expresses a human immunoglobulin heavy chain variable domain and/or a human immunoglobulin light chain variable domain comprising:
isolating a lymphocyte from a genetically modified rodent according to the invention or made according to the method of the invention,
wherein the lymphocyte expresses a human immunoglobulin variable domain that forms an antigen-binding domain specific for the antigenic pMHC complex.

The invention further provides an *in vitro* method of making a human immunoglobulin variable domain comprising:
expressing, in a cell, a first nucleic acid comprising a rearranged human immunoglobulin variable region gene sequence that encodes a human immunoglobulin variable domain expressed by a lymphocyte of a genetically modified rodent according to the invention or made according to the method of the invention, or a hybridoma produced from the lymphocyte,
wherein the human immunoglobulin variable domain expressed by the lymphocyte, or hybridoma produced therefrom, associates with its cognate variable domain to form an antigen-binding domain specific for the antigenic pMHC complex,
optionally wherein the first nucleic acid further comprises a human immunoglobulin constant region gene sequence operably linked to the rearranged human immunoglobulin variable region gene sequence,
optionally wherein the human immunoglobulin constant region gene sequence is a heavy chain constant region gene sequence and comprises a modification that increases an affinity of a C_{H}2-C_{H}3 region of an IgG heavy chain constant region amino acid sequence to neonatal Fc receptor (FcRn) at a pH ranging from 5.5 to 6.0, wherein the modification is a mutation in the IgG heavy chain constant region amino acid sequence selected from the group consisting of M428L, N434S, V259I, V308F, N434A, M252Y, S254T, T256E, T250Q, H433K, N434Y, and a combination thereof.

In some aspects described herein, a genetically modified non-human animal comprises (a) a nucleotide sequence encoding a human or humanized MHC molecule or at least a peptide binding portion thereof, and (b) an (un)rearranged human or humanized immunoglobulin heavy locus and/or an (un)rearranged human or humanized immunoglobulin light chain locus, optionally wherein at least one of the (un)rearranged human or humanized immunoglobulin heavy locus and/or an (un)rearranged human or humanized immunoglobulin light chain locus is unrearranged, wherein the genetically modified non-human animal expresses the human or humanized MHC molecule or at least a peptide binding portion thereof, wherein the genetically modified non-human animal expresses immunoglobulins comprising a human or humanized heavy chain variable domain and/or a human or humanized light chain variable domain, and wherein the non-human animal is tolerized to the human or humanized MHC molecule or at least a peptide binding portion thereof such that it generates a specific B cell response when immunized with an antigenic peptide-MHC (pMHC) complex that comprises (i) a peptide that is heterologous to the non-human animal complexed with (ii) human HLA molecule from which the human or humanized MHC molecule is derived or a portion thereof. In some aspects, the genetically modified non-human animal further comprises an antigenic peptide-MHC (pMHC) complex that comprises a peptide heterologous to the non-human animal associated with a human HLA molecule from which the human or humanized MHC molecule is derived. In some aspects, the genetically modified non-human animal further comprises (c) an antigenic peptide-MHC (pMHC) complex that comprises (i) a peptide heterologous to the non-human animal associated with (ii) a human HLA molecule from which the human or humanized MHC molecule is derived or a portion thereof, and (d) a human or humanized antigen-binding protein that specifically binds the antigenic pMHC and does not bind the human HLA molecule from which the human or humanized MHC molecule is derived.

In some embodiments, the human or humanized MHC molecule is selected from the group consisting of a human or humanized MHC class I molecule, a human or humanized MHC class II α molecule, a human or humanized MHC class II β molecule, or any combination thereof. In some embodiments, the human or humanized MHC molecule is a human or humanized MHC class I molecule. In some embodiments, the human or humanized MHC molecule is derived from an HLA class I molecule selected from the group consisting of an HLA-A molecule, an HLA-B molecule, an HLA-C molecule, and any combination thereof. In some embodiments, the genetically modified non-human animal further comprises in its genome a nucleotide sequence encoding a human or humanized β2 microglobulin, optionally at an endogenous β2 microglobulin locus, wherein the non-human animal expresses the human or humanized β2 microglobulin such that the non-human animal is tolerized to the β2 microglobulin by itself or in association with the human or humanized class I molecule.

In some embodiments, the human or humanized MHC molecule is a human or humanized MHC class II molecule, optionally wherein the human or humanized MHC molecule is derived from the α and/or β chains, or at least peptide binding groove of, an HLA class II molecule selected from the group consisting of HLA-DP, HLA-DQ, HLA-DR molecule, and any combination thereof.

In some embodiments, the human or humanized MHC molecule is a human HLA molecule. In some embodiments, the non-human animal comprises at an endogenous MHC locus a nucleotide sequence encoding a human HLA molecule, optionally wherein the nucleotide sequence replaces an endogenous nucleic acid sequence encoding an endogenous MHC molecule. In some embodiments, the non-human animal comprises at an ectopic locus a nucleotide sequence encoding a human HLA molecule. In some embodiments, the non-human animal comprises at a ROSA26 locus a nucleotide sequence encoding a human HLA molecule. In some embodiments, the non-human animal is homozygous for the nucleotide sequence at the endogenous MHC locus, the ectopic locus, or the ROSA26 locus. In some embodiments, the non-human animal is heterozygous for the nucleotide sequence at the endogenous MHC locus, the ectopic locus, or the ROSA26 locus.

In some embodiments, the human or humanized MHC molecule is a humanized, e.g., chimeric, MHC molecule. In some embodiments, the non-human animal comprises at an endogenous MHC locus a nucleotide sequence encoding the chimeric MHC molecule, optionally wherein the nucleotide sequence replaces an endogenous nucleic acid sequence encoding an endogenous MHC molecule. In some embodiments, the non-human animal comprises at an ectopic locus a nucleotide sequence encoding the chimeric MHC molecule. In some embodiments, the non-human animal comprises at a ROSA26 locus the nucleotide sequence encoding the chimeric MHC molecule. In some embodiments, the non-human animal is homozygous for the nucleotide sequence encoding the chimeric MHC molecule at the endogenous MHC locus, the ectopic locus, or the ROSA26 locus. In some embodiments, the non-human animal is heterozygous for the nucleotide sequence encoding the chimeric MHC molecule at the endogenous MHC locus, the ectopic locus, or the ROSA26 locus.

In some embodiments, the nucleotide sequence encodes a chimeric human/non-human MHC molecule comprising the extracellular domains of a human HLA molecule operably linked to transmembrane and cytoplasmic domains of an endogenous MHC molecule. In some embodiments, the nucleotide sequence encodes (i) a chimeric human/non-human MHC class I molecule comprising the α1, α2, and α3 domains of a human MHC class I molecule selected from the group consisting of HLA-A, HLA B, and HLA-C operably linked to the transmembrane and cytoplasmic domains of an endogenous non-human MHC class molecule, such as an endogenous murine H-2K polypeptide, an endogenous murine H-2D polypeptide, or an endogenous murine H-2L polypeptide, and/or (ii) a chimeric human/non-human MHC class II molecule comprising the α1 and α2 domains of a human HLA class II α polypeptide operably linked to the transmembrane and cytoplasmic domains of an endogenous non-human MHC class II α molecule, such as an endogenous murine H-2A α polypeptide or endogenous murine H-2E α polypeptide, and/or the β1 and β2 domains of a human HLA class II β polypeptide operably linked to the transmembrane and cytoplasmic domains of an endogenous non-human MHC class II β molecule, such as an endogenous murine H-2A β polypeptide or endogenous murine H-2E β polypeptide.

In some embodiments, the nucleotide sequence encoding the human or humanized MHC molecule does not disrupt an endogenous non-human MHC locus. In some embodiments, the nucleotide sequence encoding the human or humanized MHC molecule is integrated into a locus outside the endogenous MHC locus. In some embodiments, such integration does not disrupt the functionality of any other endogenous gene. In one embodiment, the nucleotide sequence is placed into an endogenous ROSA26 locus.

In some embodiments, the non-human animal is heterozygous for the nucleotide sequence encoding a human or humanized MHC molecule or at least the human peptide binding groove thereof.

In some embodiments, the genetically modified non-human animal comprises at an endogenous heavy chain locus an unrearranged human(ized) immunoglobulin heavy chain variable region in operable linkage to an endogenous heavy chain constant region. In some embodiments, the genetically modified non-human animal comprises at an endogenous heavy chain locus a restricted unrearranged human(ized) heavy chain variable region in operable linkage to an endogenous heavy chain constant region. In some embodiments, the genetically modified non-human animal comprises at an endogenous heavy chain locus a common heavy chain encoding sequence. In some embodiments, the genetically modified non-human animal comprises at an endogenous heavy chain locus a histidine modified unrearranged human(ized) heavy chain variable region in operable linkage to an endogenous heavy chain constant region. In some embodiments, the genetically modified non-human animal comprises at an endogenous heavy chain locus a heavy chain only immunoglobulin encoding sequence. In some embodiments, the genetically modified non-human animal comprises at an endogenous heavy chain locus an unrearranged human(ized) hybrid heavy chain sequence encoding a hybrid immunoglobulin chain. In some embodiments, the genetically modified non-human animal comprises at an endogenous light chain locus an unrearranged human(ized) immunoglobulin light chain variable region in operable linkage to an endogenous light chain constant region. In some embodiments, the genetically modified non-human animal comprises at an endogenous light chain locus a common light chain encoding sequence. In some embodiments, the genetically modified non-human animal comprises at an endogenous light chain locus a restricted unrearranged human(ized) light chain variable region in operable linkage to an endogenous light chain constant region. In some embodiments, the genetically modified non-human animal comprises at an endogenous light chain locus a histidine modified unrearranged human(ized) light chain variable region in operable linkage to an endogenous light chain constant region. In some embodiments, the genetically modified non-human animal comprises at an endogenous light chain locus a histidine modified rearranged human(ized) light chain variable region in operable linkage to an endogenous light chain constant region.

In some embodiments a genetically modified non-human animal comprises a functional ADAM6 gene, optionally wherein the functional ADAM6 gene is an endogenous ADAM6 gene.

In some embodiments a genetically modified non-human animal expresses an exogenous terminal deoxynucleotidyl transferase (TdT) gene.

In some aspects described herein, a method of making the genetically modified non-human animal comprises modifying the genome of the non-human animal to comprise (a) a nucleotide sequence encoding a human or humanized MHC molecule or at least a peptide binding portion thereof, and (b) an (un)rearranged human or humanized immunoglobulin heavy locus and/or an (un)rearranged human or humanized immunoglobulin light chain locus, optionally wherein at least one of the (un)rearranged human or humanized immunoglobulin heavy locus and/or an (un)rearranged human or humanized immunoglobulin light chain locus is unrearranged wherein the genetically modified non-human animal is tolerized to the human or humanized MHC molecule or at least a peptide binding portion thereof such that it generates a specific B cell response when immunized with a peptide-MHC complex that comprises (i) a peptide that is heterologous to the non-human animal complexed with (ii) human HLA molecule from which the human or humanized MHC molecule is derived or a portion thereof, and capable of providing human or humanized antigen-binding proteins comprising a human or humanized heavy chain variable domain and/or a human or humanized light chain variable domain.

In some embodiments, the method of making the genetically modified non-human animal of the invention comprises
(a)
   (i) inserting a nucleotide sequence encoding a human or humanized MHC molecule that comprises at least a human peptide-binding groove of a human leukocyte antigen (HLA) molecule or at least the human peptide binding groove thereof into a first ectopic locus, or
   (ii) replacing at an endogenous non-human animal MHC I locus a nucleotide sequence encoding a non-human animal MHC I polypeptide with a nucleotide sequence encoding a human(ized) MHC I polypeptide and/or at an endogenous non-human animal MHC II locus a nucleotide sequence encoding a non-human animal MHC II molecule with a nucleotide sequence encoding a human(ized) MHC II molecule,

   optionally wherein the human(ized) MHC I molecule comprises α1, α2, and α3 domains of a human MHC I and at least transmembrane and cytoplasmic domains of an endogenous non-human MHC I polypeptide,
   optionally wherein the human(ized) MHC II molecule comprises α1, α2, β1, and β2 domains of a human MHC II and at least transmembrane and cytoplasmic domains of an endogenous rodent MHC II polypeptide, and
(b)
   (i) inserting an (un)rearranged human or humanized immunoglobulin heavy chain locus and/or an (un)rearranged human or humanized immunoglobulin light chain locus into a second ectopic locus or
   (ii) replacing

   (A) at an endogenous non-human heavy chain locus an endogenous non-human immunoglobulin variable (V_{H}) gene segment with an unrearranged human immunoglobulin variable (V_{H}) gene segment, and optionally replacing an endogenous non-human immunoglobulin diversity (D_{H}) and/or an endogenous non-human joining (J_{H}) gene segment with an unrearranged human immunoglobulin diversity (D_{H}) gene segment and/or an unrearranged human immunoglobulin joining (J_{H}) gene segment, respectively, wherein the unrearranged human V_{H}, and optional D_{H} and J_{H} gene segments are operably linked to an endogenous heavy chain constant region gene sequence, and/or
   (B) at an endogenous non-human light chain locus an endogenous non-human light chain variable (V_{L}) gene segment and an endogenous non-human light chain joining (J_{L}) gene segment with a human light chain variable (V_{L}) gene segment and a human light chain joining (J_{L}) gene segment, which are optionally rearranged to form a V_{L}/J_{L} gene sequence, wherein the human V_{L} and joining J_{L} gene segments are operably linked to an endogenous light chain constant region gene sequence
   wherein the (a) nucleotide sequence respectively encoding a non-human MHC I and/or a non-human MHC II molecule and (b) V_{H}, D_{H}, J_{H}, V_{L}, and J_{L} gene segments are either
   (I) inserted or replaced by sequential homologous recombination in a single non-human embryonic stem (ES) cell or
   (II) in a first and a second ES cell respectively used to generate a first and second non-human animals.

In some aspects, the method of making a genetically modified non-human animal comprises administering to the non-human animal an antigenic pMHC complex that comprises a peptide heterologous to the non-human animal associated with a human HLA molecule from which the human or humanized MHC molecule is derived. In some embodiments, the antigenic pMHC complex is linked to a helper T cell epitope. In some embodiments, the helper T cell epitope comprises PADRE, e.g., as set forth as SEQ ID NO:28.

In some embodiments, a method of generating an antigen binding protein that specifically binds an antigenic pMHC complex of interest or a nucleic acid sequence encoding same comprises maintaining a genetically modified non-human animal of the invention in conditions sufficient for the non-human animal to mount an immune response to the antigenic pMHC complex of interest, wherein the antigenic pMHC complex of interest comprises a peptide that is heterologous to the non-human animal and is presented in the context of a human HLA from which the human or humanized MHC molecule is derived, or a portion thereof. In some embodiments, the method comprises as a first step(s) immunizing the non-human animal with the antigenic pMHC complex of interest, and optionally boosting the immune response of the immunized non-human animal, optionally wherein immunizing and/or boosting comprises administering to the non-human animal with the antigenic pMHC complex of interest linked to a helper T cell epitope, e.g., PADRE (SEQ ID NO:28).

In some embodiments, a method of obtaining a nucleic acid encoding a human immunoglobulin heavy chain variable domain and/or a human immunoglobulin light chain variable domain comprises: isolating from a non-human animal of the invention a nucleic acid comprising a rearranged human immunoglobulin variable region gene sequence that encodes a human immunoglobulin variable domain expressed by a lymphocyte of the non-human animal, or a hybridoma produced from the lymphocyte, wherein the human immunoglobulin variable domain expressed by the lymphocyte, or hybridoma produced therefrom, associates with its cognate variable domain to form an antigen binding domain specific for the antigenic pMHC complex. In some embodiments, the method further comprises immunizing the non-human animal with an antigenic pMHC complex of interest and allowing the non-human animal to mount an immune response to the antigen before obtaining the nucleic acid. In some embodiments, the obtained rearranged human immunoglobulin variable region gene sequence comprises at least one somatic hypermutation.

In some aspects of the disclosure, a nucleic acid described herein comprises a human constant region gene sequence operably linked to the rearranged human immunoglobulin variable region gene sequence. In some embodiments, the human heavy chain constant region gene sequence comprises a modification that increases an affinity of a CH2-CH3 region of an IgG heavy chain constant region amino acid sequence to neonatal Fc receptor (FcRn) at a pH ranging from 5.5 to 6.0, wherein the modification is a mutation in the IgG heavy chain constant region amino acid sequence selected from the group consisting of M428L, N434S, V259I, V308F, N434A, M252Y, S254T, T256E, T250Q, H433K, N434Y, and a combination thereof. Also described herein are mammalian host cells, e.g., for expressing a nucleic acid encoding human immunoglobulin heavy and/or light chain specific for the antigen pMHC complex.

In some embodiments, a method of obtaining a cell that expresses a human immunoglobulin heavy chain variable domain and/or a human immunoglobulin light chain variable domain comprises isolating a lymphocyte from a non-human animal of the invention, wherein the lymphocyte expresses a human immunoglobulin variable domain that forms an antigen binding domain specific for the antigenic pMHC complex. In some embodiments, the method comprises producing a hybridoma from the isolated lymphocyte.

In some aspects described herein, an isolated cell, e.g., a germ cell, an embryonic stem cell, a somatic cell (e.g., a B cell) as described herein comprises (a) a nucleotide sequence encoding a human or humanized MHC molecule or at least a peptide binding portion thereof, and (b) an (un)rearranged human or humanized immunoglobulin heavy locus and/or an (un)rearranged human or humanized immunoglobulin light chain locus. In some embodiments, at least one of the (un)rearranged human or humanized immunoglobulin heavy locus and/or an (un)rearranged human or humanized immunoglobulin light chain locus is unrearranged. In some aspects, an isolated cell is obtained according to a method described herein.

In some embodiments, an *in vitro* method of making a human immunoglobulin variable domain comprises expressing in a cell a first nucleic acid comprising a rearranged human immunoglobulin variable region gene sequence that encodes a human immunoglobulin variable domain expressed by a lymphocyte of a non-human animal of the invention, or a hybridoma produced from the lymphocyte, wherein the human immunoglobulin variable domain expressed by the lymphocyte, or hybridoma produced therefrom, associates with its cognate variable domain to form an antigen binding domain specific for the antigenic pMHC complex. In some embodiments, the first nucleic acid further comprises a human immunoglobulin constant region gene sequence operably linked to the rearranged human immunoglobulin variable region gene sequence. In some embodiments, the human immunoglobulin constant region gene sequence is a heavy chain constant region gene sequence and comprises a modification that increases an affinity of a CH2-CH3 region of an IgG heavy chain constant region amino acid sequence to neonatal Fc receptor (FcRn) at a pH ranging from 5.5 to 6.0, wherein the modification is a mutation in the IgG heavy chain constant region amino acid sequence selected from the group consisting of M428L, N434S, V259I, V308F, N434A, M252Y, S254T, T256E, T250Q, H433K, N434Y, and a combination thereof.

### BRIEF DESCRIPTION OF THE DRAWINGS

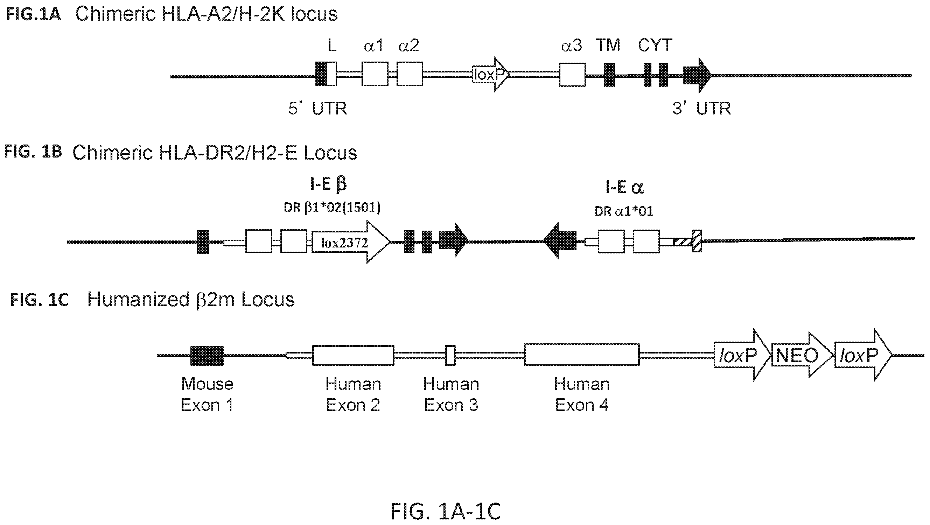

**FIGs. 1A-1C** are exemplary aspects of the present disclosure, which provide schematic representations (not to scale) of chimeric MHC I and MHC II loci, e.g., a chimeric HLA-A2/H-2K locus, e.g., at an endogenous H-2K locus, (**FIG. 1A**), a chimeric HLA-DR2/H-2E locus, e.g., at an endogenous H-2E locus, (**FIG. 1B**), and a humanized β2M locus, e.g., at an endogenous β2M locus (**FIG. 1C**). Unless otherwise indicated, human sequences are depicted as empty shapes and mouse sequences are depicted as filled shapes. The striped shapes represent exon 1 and a portion of the intron downstream thereof of an H-2E gene sequence derived from a different mouse strain than the endogenous locus. Floxed neomycin phosphotransferase cassette(s) before Cre-mediated removal of the cassette (**FIG. 1C**) and after Cre-removal of the cassette (**FIGs. 1A** and **1B**) are depicted with arrows labeled accordingly.

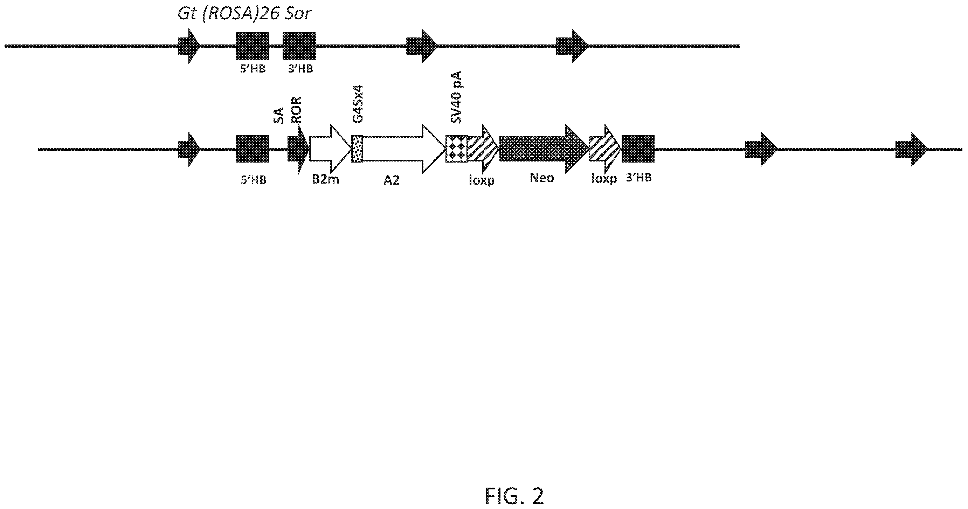

**FIG. 2** provides a schematic representation (not to scale) of an exemplary transgene (SEQ ID NO:22) of the present disclosure encoding a single chain MHC molecule (SEQ ID NO:23) comprising the full-length of a mature HLA-A2 (A2) polypeptide (e.g., amino acids 25-365 of HLA-A2) associated with human β2 microglobulin (B2m) at the ROSA26 (Gt (ROSA)26 Sor) locus. Unless otherwise indicated, human sequences are depicted as empty shapes, mouse sequences are depicted as filled shapes, and sequences that are neither human or mouse sequences are depicted with various patterns. Filled arrows indicate exons of the endogenous mouse ROSA26 locus. 5'HB and 3'HB: homology boxes in the ROSA 26 gene used for insertion of the B2m-G4Sx4-HLA-A2 transgene (SEQ ID NO:22) that encodes the single chain HLA-A2/β2M complex (SEQ ID NO:23) by homologous recombination. SA: consensus Splice Acceptor. ROR: mouse ROR signal sequence. G4Sx4: GGGS linker (SEQ ID NO:21), SV40PA: polyadenylation signal from SV40 virus. LoxP-New-LoxP: floxed neomycin phosphotransferase cassette before Cre-mediated removal of the cassette.

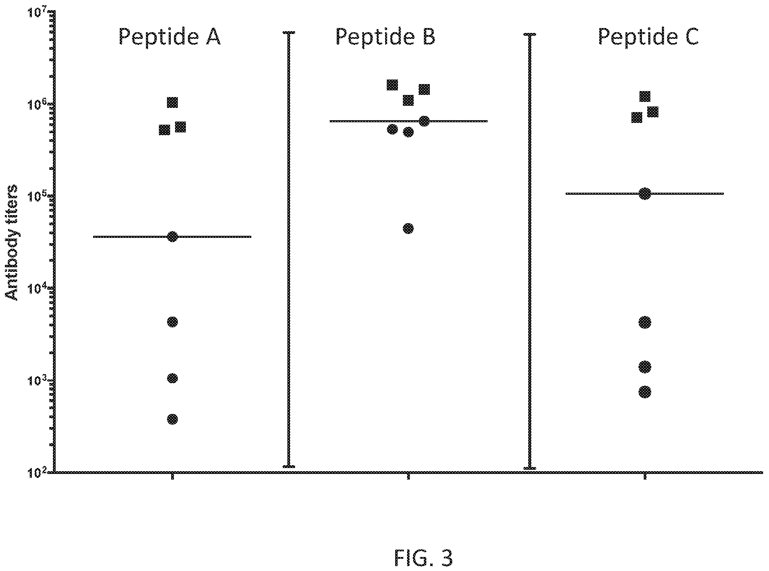

**FIG. 3** shows results from exemplary embodiments of the invention, where sera from test mice (comprising nucleotide sequences encoding a humanized MHC I molecule (HLA-A2/H-2K), a humanized β2 microglobulin, an unrearranged humanized immunoglobulin heavy chain locus, and a humanized common light chain locusVκ1-39/Jκ; •) or control mice (comprising a functional (e.g., murine) ADAM6 gene and humanized heavy and light chain loci; ▪) immunized with a nucleotide sequence encoding an immunogen comprising peptide B presented in the peptide binding groove of HLA-A associated with human β2 microglobulin were tested for titers (y-axis) of antibodies that bind irrelevant or relevant peptide (peptide A (irrelevant), peptide B (relevant), or peptide C (irrelevant)) presented in the peptide binding groove of HLA-A associated with human β2 microglobulin as a single chain pMHC complex. The titer of antibodies in the sera that bind the individual pMHC complexes is calculated as the interpolated serum dilution factor of which the binding signal is 2-fold over background.

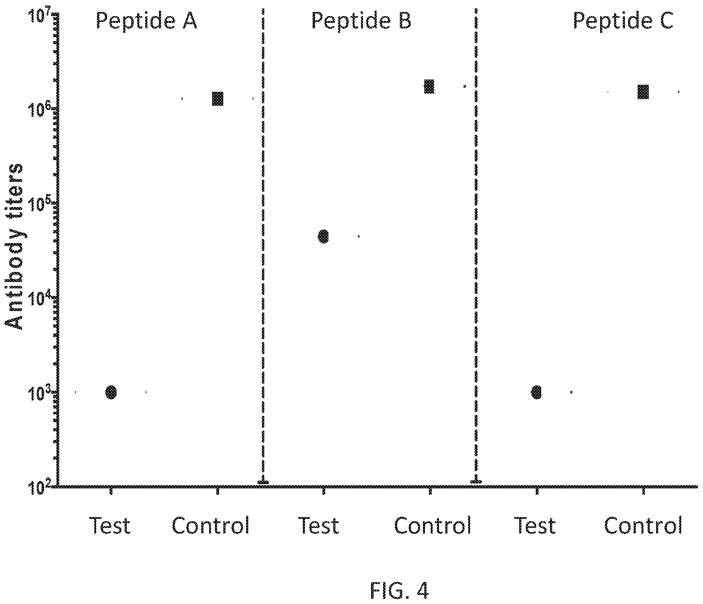

**FIG. 4** shows results from exemplary embodiments of the invention, where sera from a test mouse (comprising nucleotide sequences encoding a humanized MHC I molecule (HLA-A2/H-2K), a humanized β2 microglobulin, an unrearranged humanized immunoglobulin heavy chain locus, and a humanized common light chain locusVκ1-39/Jκ; •) or a control mouse (comprising a functional (e.g., murine) ADAM6 gene and humanized heavy and light chain loci; ▪) immunized with a single-chain pMHC complex immunogen comprising peptide B presented in the peptide binding groove of HLA-A associated with human β2 microglobulin was tested for titers (y-axis) of antibodies that bind irrelevant or relevant peptide (peptide A (irrelevant), peptide B (relevant), or peptide C (irrelevant)) presented in the peptide binding groove of HLA-A associated with human β2 microglobulin as a single chain pMHC complex. The titer (y-axis) of antibodies in the sera that bind the individual pMHC complexes is calculated as the interpolated serum dilution factor of which the binding signal is 2-fold over background.

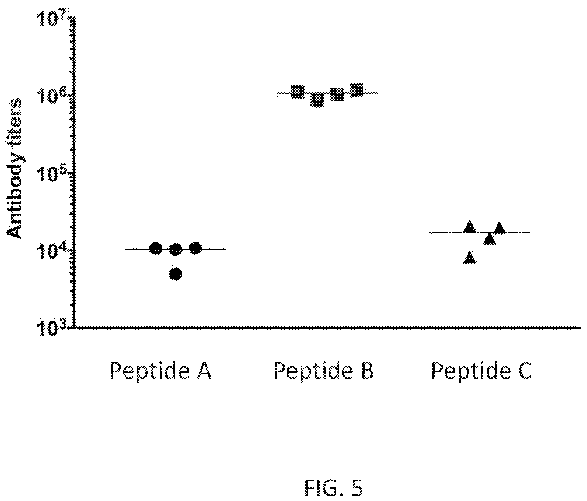

**FIG. 5** shows results from exemplary embodiments of the invention, where sera from test mice (comprising nucleotide sequences encoding a humanized MHC I molecule (HLA-A2/H-2K), a humanized β2 microglobulin, an unrearranged humanized immunoglobulin heavy chain locus and a humanized common light chain locus Vκ1-39/Jκ) immunized with an immunogen comprising peptide B presented in the peptide binding groove of HLA-A associated with human β2 microglobulin and provided boosters with another recombinant polypeptide comprising peptide B presented in the peptide binding groove of HLA-A associated with human β2 microglobulin and a helper T cell epitope (PADRE) were tested for binding to irrelevant or relevant peptide (peptide A (irrelevant), peptide B (relevant), or peptide C (irrelevant)) presented in the peptide binding groove of HLA-A associated with human β2 microglobulin as a single chain pMHC complex. The antibody titer (y-axis) of antibodies in the sera that bind the individual pMHC complexes is calculated as the interpolated serum dilution factor of which the binding signal is 2-fold over background.

### DESCRIPTION

As shown herein, control mice or rats that are not tolerized to a human HLA and human β2 microglobulin molecules but are immunized with a peptide of interest presented in the context of the HLA molecule, i.e., a pMHC complex of interest, elicit antibody titers to the pMHC complex of interest. **FIGs. 3-4** (Square symbols). However, the sera of the non-tolerized and immunized control animals also comprised comparable antibody titers to irrelevant pMHC complexes (e.g., irrelevant peptide presented in the same context) against which the non-human animals were not immunized, suggesting that response generated against the pMHC complex of interest is not a specific response. See, **FIGs. 3-4** (Square symbols).

In contrast, mice or rats that are tolerized to human HLA and human β2 microglobulin molecules, or at least the peptide-binding groove thereof (e.g., the extracellular portion thereof) and immunized with a pMHC complex of interest derived from the human HLA molecule elicit larger antibody titers to the pMHC complex of interest than to irrelevant pMHC complexes. See, **FIGs. 3-5.** Accordingly, it is shown herein that non-human animals that are tolerized to a human HLA and human β2 microglobulin molecules (or at least the peptide-binding groove thereof (e.g., the extracellular portion thereof)) provide an *in vivo* platform for the generation of a specific immune response to a pMHC of interest, from which a lead compound or compounds may be selected. Such platform is improved over a platform involving non-tolerized animals that generate non-specific immune responses to a pMHC of interest. Accordingly, described herein is a non-human animal genetically modified to be tolerized to a human HLA molecule such that, when immunized with a pMHC complex of interest that comprises a peptide of interest presented in the context of the human HLA molecule, the non-human animal is able to generate a specific B cell response to the pMHC complex of interest.

In exemplary aspects, non-human animals are genetically modified to be tolerized to a human HLA molecule, a portion thereof, and/or a single chain derivative thereof, but not to an antigenic peptide-MHC (pMHC) complex comprising an antigenic peptide (e.g., a peptide heterologous to the genetically modified non-human animal) in association with (e.g., presented by) the human HLA molecule to which the non-human animal is tolerized. Since the non-human animal is not tolerized to such antigenic pMHC complex, an animal genetically modified as disclosed herein may be useful not only for isolation of immune cells that are non-responsive to a human HLA molecule, a portion thereof and/or a single chain derivative thereof, e.g., for *in vitro* studies, but also for the generation of antigen-binding proteins, particularly human or humanized antigen-binding proteins, that specifically bind to an antigenic pMHC complex of interest. Such specific (human or humanized) antigen-binding proteins may be useful in therapies for treating human diseases, e.g., in preventing the formation of an immunological synapse between an autoreactive TCR and a pMHC complex comprising an autoreactive self-antigen (e.g., for the prevention and/or treatment of autoimmune disorders, graft-versus-host disease, transplant rejection, etc.), targeting cells infected with a pathogen (e.g., a virus) and presenting a viral peptide via a pMHC complex expressed on the cell surface, etc. In addition to describing non-human animals and methods of making such non-human animals that are tolerized to a human or humanized HLA molecule, a portion thereof, and/or a single chain derivative thereof; methods of administering the antigenic pMHC complex to the non-human animals for the generation of an anti-pMHC antibody response as well as making such antigenic pMHC complexes are also disclosed.

### Definitions

Unless defined otherwise, all technical and scientific terms used herein have the same meaning as commonly understood by one of ordinary skill in the art.

Singular forms "a", "an", and "the" include plural references unless the context clearly dictates otherwise. Thus, for example, a reference to "a method" includes one or more methods, and/or steps of the type described herein and/or which will become apparent to those persons skilled in the art upon reading this disclosure.

The term "about" or "approximately" includes being within a meaningful range of a value. The allowable variation encompassed by the term "about" or "approximately" depends on the particular system under study, and can be readily appreciated by one of ordinary skill in the art.

The terms "major histocompatibility complex," and "MHC" encompass the terms "human leukocyte antigen" or "HLA" (the latter two of which are generally reserved for human MHC molecules), naturally occurring MHC molecules, individual chains of MHC molecules (e.g., MHC class I α (heavy) chain, β2 microglobulin, MHC class II α chain, and MHC class II β chain), individual subunits of such chains of MHC molecules (e.g., α1, α2, and/or α3 subunits of MHC class I α chain, α1-α2 subunits of MHC class II α chain, β1- β2 subunits of MHC class II β chain) as well as portions (e.g., the peptide-binding portions, e.g., the peptide-binding grooves), mutants and various derivatives thereof (including fusions proteins), wherein such portion, mutants and derivatives retain the ability to display an antigenic peptide for recognition by a T-cell receptor (TCR), e.g., an antigen-specific TCR. An MHC class I molecule comprises a peptide binding groove formed by the α1 and α2 domains of the heavy α chain that can stow a peptide of around 8-10 amino acids. Despite the fact that both classes of MHC bind a core of about 9 amino acids (e.g., 5 to 17 amino acids) within peptides, the open-ended nature of MHC class II peptide binding groove (the α1 domain of a class II MHC α polypeptide in association with the β1 domain of a class II MHC β polypeptide) allows for a wider range of peptide lengths. Peptides binding MHC class II usually vary between 13 and 17 amino acids in length, though shorter or longer lengths are not uncommon. As a result, peptides may shift within the MHC class II peptide binding groove, changing which 9-mer sits directly within the groove at any given time. Conventional identifications of particular MHC variants are used herein. According to the invention, a genetically modified non-human animal comprises a nucleotide sequence encoding a human(ized) MHC molecule that comprises at least a human peptide binding groove (e.g., the peptide-binding portion) and in further embodiments, at least the extracellular domains of a human HLA class I/human β2 microglobulin molecule and/or a human HLA class II molecule.

The term "tolerized," "tolerance," "tolerization" and the like refers to the inability or reduced ability of an animal, e.g., a genetically modified non-human animal as disclosed herein, to mount an immune response to a substance. Generally, an animal is tolerized, exhibits tolerance, undergoes tolerization, etc., to its own proteins or molecules expressed during embryogenesis and/or at birth, e.g., an animal fails to or is unlikely able to mount an immune response to its own proteins or molecules expressed from its genome, e.g., its germline genome. By genetically modifying an animal to comprise in its genome, e.g., germline genome, a human(ized) MHC molecule, upon expression of the "empty" human(ized) MHC molecule, the animal becomes tolerized, exhibits tolerance, undergoes tolerization, etc., to the empty human(ized) MHC molecule as if the human(ized) MHC molecule is its own protein. "Empty" in the context of an HLA molecule, MHC molecule, human(ized) MHC molecule, and the like includes an HLA molecule, MHC molecule, human(ized) MHC molecule, and the like expressed either without a peptide within its peptide binding groove, or with a peptide that is endogenous to the animal expressing the HLA molecule, MHC molecule, human(ized) MHC molecule, and the like. For example, an empty MHC molecule may include in an animal a human(ized) MHC molecule that is expressed from the genome, e.g., germline genome, of the animal and presents an endogenous animal self-protein or portion thereof.

A genome of a non-human animal may be considered a "somatic genome," e.g., may be the genome found in the somatic cells of the non-human animal, or may be considered a "germline genome," e.g., may be the genome that is found in the germ cells of the non-human animal and is passed on to the offspring of the non-human animal. A skilled artisan will readily recognize that immunoglobulin heavy and/or light chain variable region loci that are unrearranged in the germline genome are capable of rearranging in a somatic cell (e.g., B cell) of the non-human animal to form a rearranged immunoglobulin variable region locus that encodes an immunoglobulin variable domain. Accordingly, an unrearranged heavy and/or light chain locus in exemplary aspects may be found in the germline genome of the non-human animal, and the rearranged sequence derived therefrom may be found, e.g., in a B cell of the non-human animal.

The term "non-human animal" and the like refers to a rat or a mouse.

The term "humanized," "chimeric," "human/non-human" and the like refers to a molecule (e.g., a nucleic acid, protein, etc.) that was non-human in origin and for which a portion has been replaced with a corresponding portion of a corresponding human molecule in such a manner that the modified (e.g., humanized, chimeric, human/non-human, etc.) molecule retains its biological function and/or maintains the structure that performs the retained biological function. A humanized molecule may be considered derived from a human molecule where the humanized molecule is encoded by a nucleotide comprising a nucleic acid sequence that encodes the human molecule (or a portion thereof). In contrast "human" and the like encompasses molecules having only a human origin, e.g., human nucleotides or protein comprising only human nucleotide and amino acid sequences respectively. The term "human(ized)" is used to reflect that the human(ized) molecule may be (a) a human molecule or (b) a humanized molecule.

According to the invention, a human(ized) MHC molecule comprises, or a non-human animal expresses and is tolerized to a human(ized) MHC molecule comprising, at least the human peptide-binding groove of a human HLA molecule, where the human(ized) MHC molecule retains the ability to present antigen in the human peptide-binding groove and/or the human(ized) MHC molecule maintains the structure of the human peptide-binding groove of the human HLA molecule. In some embodiments, a human(ized) MHC molecule comprises, or a non-human animal expresses and is tolerized to a human(ized) MHC molecule comprising, at least the human extracellular domain of a human HLA molecule, where the human(ized) MHC molecule retains the ability to present antigen and/or the human(ized) MHC molecule maintains the structure of the human extracellular domain of the human HLA molecule that allows a peptide-binding groove to form. In some embodiments, a human(ized) MHC molecule comprises, or a non-human animal expresses and is tolerized to a human(ized) MHC molecule comprising, the human peptide-binding groove of a human HLA class I polypeptide (e.g., at least the α1 and α2 domains of a human HLA class I polypeptide, e.g., the extracellular portion of a human HLA class I polypeptide, e.g., at least the full-length mature human HLA class I polypeptide), where the human(ized) MHC class I polypeptide retains the ability to present antigen and/or the structure necessary to present antigen in the human peptide binding groove. In some embodiments, a human(ized) MHC molecule comprises, or a non-human animal expresses and is tolerized to a human(ized) MHC molecule comprising, the human peptide-binding groove of a human HLA class I polypeptide (e.g., at least the α1 and α2 domains of a human HLA class I polypeptide, e.g., the extracellular portion of a human HLA class I polypeptide, e.g., at least the full-length mature human HLA class I polypeptide), where the human(ized) MHC class I molecule retains the ability to present antigen and/or the structure necessary to present antigen in the human peptide binding groove, and where the human(ized) MHC class I molecule further comprises a human or humanized β2 microglobulin that stabilizes the MHC class I molecule.

The term "antigen" refers to any agent (e.g., protein, peptide, polysaccharide, glycoprotein, glycolipid, nucleotide, portions thereof, or combinations thereof) that, when introduced into an immunocompetent host is recognized by the immune system of the host and elicits an immune response by the host. The T-cell receptor recognizes a peptide presented in the context of a major histocompatibility complex (MHC) as part of an immunological synapse. The peptide-MHC (pMHC) complex is recognized by TCR, with the peptide (antigenic determinant) and the TCR idiotype providing the specificity of the interaction. Accordingly, the term "antigen" encompasses peptides presented in the context of MHCs, e.g., peptide-MHC complexes, e.g., pMHC complexes. The peptide displayed on MHC may also be referred to as an "epitope" or an "antigenic determinant". The terms "peptide," "antigenic determinant," "epitopes," etc., encompass not only those presented naturally by antigen-presenting cells (APCs), but may be any desired peptide so long as it is recognized by an immune cell of a genetically modified non-human animal, e.g., when presented appropriately to the cells of an immune system. For example, a peptide having an artificially prepared amino acid sequence may also be used as the epitope.

"Peptide-MHC complex," "pMHC complex," "peptide-in-groove," and the like includes
(i) an MHC molecule, e.g., a human and/or humanized (e.g."human(ized)") MHC molecule, or portion thereof (e.g., the peptide-binding groove thereof, and e.g., the extracellular portion thereof), and
(ii) an antigenic peptide,
where the MHC molecule and the antigenic peptide are complexed in such a manner that the pMHC complex can specifically bind a T-cell receptor. A pMHC complex encompasses cell surface expressed pMHC complexes and soluble pMHC complexes. In exemplary aspects, the non-human animal comprising in its genome, e.g., germline genome, a nucleotide sequence encoding a human(ized) MHC molecule, or at least a human peptide binding groove thereof, becomes tolerized to the empty human(ized) MHC molecule, or the empty human peptide binding groove thereof. Upon administration to the non-human animal of an antigenic pMHC complex, e.g., a complex comprising the human(ized) MHC molecule complexed with a peptide that is foreign to the host non-human animal into which the pMHC complex is administered, the non-human animal is capable of generating an antibody response to the antigenic pMHC complex. Such specific antigen-binding proteins may then be isolated and used as therapeutics to specifically modulate the specific T-cell receptor interaction with the antigenic pMHC. In exemplary embodiments, a soluble pMHC complex comprising a peptide (which is foreign to the host non-human animal into which the pMHC complex is administered) complexed with an MHC molecule to which the non-human animal is tolerized may not elicit a T-cell immune response due to the soluble nature of the administered pMHC complex. However, such soluble pMHC complex may still be considered antigenic in that it may elicit a B cell mediated immune response that generates antigen-binding proteins that specifically bind the soluble pMHC complex.

The phrase "gene segment," or "segment" includes reference to a variable (V) gene segment (e.g., an immunoglobulin light chain variable (V_{L}) gene segment or an immunoglobulin heavy chain variable (V_{H}) gene segment), an immunoglobulin heavy chain diversity (D_{H}) gene segment, or a joining (J) gene segment, e.g., an immunoglobulin light chain joining (J_{L}) gene segment or an immunoglobulin heavy chain joining (J_{H}) gene segment, which includes unrearranged sequences at immunoglobulin loci that can participate in rearrangement (mediated by, e.g., endogenous recombinases) to form a rearranged light chain V_{L}/J_{L} or rearranged heavy chain V_{H}/D_{H}/J_{H} sequence. Unless indicated otherwise, the unrearranged V, D, and J segments comprise recombination signal sequences (RSS) that allow for V_{L}/J_{L} recombination or V_{H}/D_{H}/J_{H} recombination according to the 12/23 rule.

The terms "antigen-binding protein," "immunoglobulin, "antibody," "antibodies," "binding protein" and the like refer to monoclonal antibodies, multispecific antibodies, human antibodies, humanized antibodies, chimeric antibodies, single-chain Fvs (scFv), single chain antibodies, Fab fragments, F(ab') fragments, disulfide-linked Fvs (sdFv), intrabodies, minibodies, diabodies and anti-idiotypic (anti-Id) antibodies (including, e.g., anti-Id antibodies to antigen-specific TCR), and epitope-binding fragments of any of the above. The terms "antibody" and "antibodies" also refer to covalent diabodies such as those disclosed in U.S. Pat. Appl. Pub. 20070004909, and Ig-DARTS such as those disclosed in U.S. Pat. Appl. Pub. 20090060910. A pMHC-binding protein refers to an antigen-binding protein, immunoglobulin, antibody, or the like that specifically binds a pMHC complex.

The term "specifically binds," "binds in a specific manner," "antigen-specific" or the like, indicates that the molecules involved in the specific binding are (1) able to stably bind, e.g., associate, e.g., form intermolecular non-covalent bonds, under physiological conditions, and are (2) unable to form stably bind under physiological conditions to other molecules outside the specified binding pair. Accordingly, an antigen-binding protein (e.g., immunoglobulin, antibody, and the like) that binds in a specific manner to a pMHC complex indicates that the pMHC-binding protein forms stable intermolecular non-covalent bonds with the pMHC complex. Accordingly, a pMHC-binding protein that specifically binds, e.g., binds in a specific manner, to a specific pMHC complex comprising a first peptide complexed with a first MHC molecule
(i) does not stably bind under physiological conditions to a pMHC complex comprising a second peptide presented in the context of the first MHC molecule, wherein the first and second peptides are not identical, e.g., display different idiotypes (conformational structures) upon association with the peptide binding groove of the first MHC molecule,
(ii) may interfere with (e.g., block) the interaction between (a) a first TCR that specifically recognizes the first peptide presented in the context of the first MHC molecule and (b) the pMHC complex comprising the first peptide and first MHC molecule, but
(iii) will not interfere with the interaction between (a) a second TCR that specifically recognizes a second peptide presented in the context of the first MHC molecule or (b) the pMHC complex comprising the second peptide presented in the context of the first MHC molecule, wherein the first and second peptides are different, e.g., display different idiotypes (conformational structures) upon association with the peptide binding groove of the first MHC molecule. A pMHC-binding protein that specifically binds a pMHC complex comprising a first peptide and first MHC molecule may also, or independently, bind a first cell presenting the first peptide in the context of the first MHC molecule but will not bind a second cell presenting a second peptide in the context of the first MHC molecule, wherein the first and second peptides are different, display different idiotypes (conformational structures) upon association with the peptide binding groove of the MHC molecule. Specific binding may also be characterized by an equilibrium dissociation constant (K_{D}) of in the low micromolar to picomolar range. High specificity may be in the low nanomolar range, with very high specificity being in the picomolar range. Methods for determining whether two molecules specifically bind are well known in the art and include, for example, equilibrium dialysis, surface plasmon resonance, and the like.

An "individual" or "subject" or "animal" refers to humans, veterinary animals (e.g., cats, dogs, cows, horses, sheep, pigs, etc.) and experimental animal models of diseases (e.g., mice, rats). In one aspect, the subject is a human.

The term "protein" is used herein encompasses all kinds of naturally occurring and synthetic proteins, including protein fragments of all lengths, fusion proteins and modified proteins, including without limitation, glycoproteins, as well as all other types of modified proteins (e.g., proteins resulting from phosphorylation, acetylation, myristoylation, palmitoylation, glycosylation, oxidation, formylation, amidation, polyglutamylation, ADP-ribosylation, pegylation, biotinylation, etc.).

The terms "nucleic acid" and "nucleotide" encompass both DNA and RNA unless specified otherwise.

The term "titer," "antibody titer," "titer of antibodies" or the like refers to the ability of a sample of antibodies (e.g., in serum obtained from a subject) that share a characteristic, e.g., isotype, ability to bind to an antigen in a specific or non-specific manner, etc. Methods of determination of the titer of antibodies in sera collected from a subject are known in the art. In some aspects, the titer of antibodies in serum collected from a subject is calculated as the interpolated serum dilution factor of which the binding signal is 2-fold over background. In some embodiments, a non-human animal that expresses and is tolerized to a human(ized) MHC molecule can or does generate, a specific response (e.g., a specific immune response, e.g., a specific B-cell response, e.g., a specific antibody response) when immunized with a pMHC complex of interest (e.g., peptide of interest presented in the context of the HLA molecule from which the human(ized) MHC molecule is derived). In some embodiments, a response is considered a "specific response" or the like where the non-human animal generates, when immunized with a pMHC complex of interest, an antibody titer to the pMHC complex of interest that is greater than the antibody titer to an irrelevant pMHC complex. In some embodiments, a response is considered a specific response or the like, where the non-human animal generates, when immunized with a pMHC complex of interest, an antibody titer to the pMHC complex of interest that is at least 2-fold greater than the antibody titer to an irrelevant pMHC complex. In some embodiments, a response is considered a specific response or the like, where the non-human animal generates, when immunized with a pMHC complex of interest, an antibody titer to the pMHC complex of interest that is at least 5-fold greater than the antibody titer to an irrelevant pMHC complex. In some embodiments, a response is considered a specific response or the like, where the non-human animal generates, when immunized with a pMHC complex of interest, an antibody titer to the pMHC complex of interest that is at least 10-fold greater than the antibody titer to an irrelevant pMHC complex. In some embodiments, a response is considered a specific response or the like, where the non-human animal generates, when immunized with a pMHC complex of interest, an antibody titer to the pMHC complex of interest that is significantly greater than the antibody titer to an irrelevant pMHC complex.

The term "operably linked" or the like refers to a juxtaposition wherein the components described are in a relationship permitting them to function in their intended manner. For example, unrearranged variable region gene segments are "operably linked' to a contiguous constant region gene if the unrearranged variable region gene segments are capable of rearranging to form a rearranged variable region gene that is expressed in conjunction with the constant region gene as a polypeptide chain of an antigen binding protein. A control sequence "operably linked" to a coding sequence is ligated in such a way that expression of the coding sequence is achieved under conditions compatible with the control sequences. "Operably linked" sequences include both expression control sequences that are contiguous with a gene of interest and expression control sequences that act in trans or at a distance to control a gene of interest (or sequence of interest). The term "expression control sequence" includes polynucleotide sequences, which are necessary to affect the expression and processing of coding sequences to which they are ligated. "Expression control sequences" include: appropriate transcription initiation, termination, promoter and enhancer sequences; efficient RNA processing signals such as splicing and polyadenylation signals; sequences that stabilize cytoplasmic mRNA; sequences that enhance translation efficiency (i.e., Kozak consensus sequence); sequences that enhance polypeptide stability; and when desired, sequences that enhance polypeptide secretion. The nature of such control sequences differs depending upon the host organism. For example, in prokaryotes, such control sequences generally include promoter, ribosomal binding site and transcription termination sequence, while in eukaryotes typically such control sequences include promoters and transcription termination sequence. The term "control sequences" is intended to include components whose presence is essential for expression and processing, and can also include additional components whose presence is advantageous, for example, leader sequences and fusion partner sequences.

The term "administration" and the like refers to and includes the administration of a composition to a subject or system (e.g., to a cell, organ, tissue, organism, or relevant component or set of components thereof). The skilled artisan will appreciate that route of administration may vary depending, for example, on the subject or system to which the composition is being administered, the nature of the composition, the purpose of the administration, etc. For example, in certain aspects, administration to an animal subject (e.g., to a human or a rodent) may be bronchial (including by bronchial instillation), buccal, enteral, interdermal, intra-arterial, intradermal, intragastric, intramedullary, intramuscular, intranasal, intraperitoneal, intrathecal, intravenous, intraventricular, mucosa!, nasal, oral, rectal, subcutaneous, sublingual, topical, tracheal (including by intratracheal instillation), transdermal, vaginal and/or vitreal. In some embodiments, administration may involve intermittent dosing. In some embodiments, administration may involve continuous dosing ( e.g., perfusion) for at least a selected period of time.

The term "derived from", when used concerning a rearranged variable region gene or a variable domain "derived from" an unrearranged variable region and/or unrearranged variable region gene segments, refers to the ability to trace the sequence of the rearranged variable region gene or variable domain back to a set of unrearranged variable region gene segments that were rearranged to form the rearranged variable region gene that expresses the variable domain (accounting for, where applicable, splice differences and somatic mutations). For example, a rearranged variable region gene that has undergone somatic mutation does not change the fact that it is derived from the unrearranged variable region gene segments.

The term "endogenous locus" or "endogenous gene" refers to a genetic locus found in a parent or reference organism prior to introduction of a disruption, deletion, replacement, alteration, or modification as described herein. In some embodiments, an endogenous locus has a sequence found in nature. In some embodiments, an endogenous locus is a wild-type locus. In some embodiments, an endogenous locus is an engineered locus.

The term "heterologous" refers to an agent or entity from a different source. For example, when used in reference to a polypeptide, gene, or gene product present in a particular cell or organism, the term clarifies that the relevant polypeptide, gene, or gene product: 1) was engineered by the hand of man; 2) was introduced into the cell or organism (or a precursor thereof) through the hand of man (e.g., via genetic engineering); and/or 3) is not naturally produced by or present in the relevant cell or organism (e.g., the relevant cell type or organism type). "Heterologous" also includes a polypeptide, gene or gene product that is normally present in a particular native cell or organism, but has been altered or modified, for example, by mutation or placement under the control of non-naturally associated and, in some aspects, non-endogenous regulatory elements (e.g., a promoter).

In accordance with the disclosure herein, there may be employed conventional molecular biology, microbiology, and recombinant DNA techniques within the skill of the art. Such techniques are explained fully in the literature. See, e.g., Sambrook, Fritsch & Maniatis, Molecular Cloning: A Laboratory Manual, Second Edition. Cold Spring Harbor, NY: Cold Spring Harbor Laboratory Press, 1989 (herein "Sambrook et al., 1989"); DNA Cloning: A Practical Approach, Volumes I and II (D.N. Glover ed. 1985); Oligonucleotide Synthesis (M.J. Gait ed. 1984); Nucleic Acid Hybridization [B.D. Hames & S.J. Higgins eds. (1985)]; Transcription And Translation [B.D. Hames & S.J. Higgins, eds. (1984)]; Animal Cell Culture [R.I. Freshney, ed. (1986)]; Immobilized Cells And Enzymes [IRL Press, (1986)]; B. Perbal, A Practical Guide To Molecular Cloning (1984); Ausubel, F.M. et al. (eds.). Current Protocols in Molecular Biology. John Wiley & Sons, Inc., 1994. These techniques include site directed mutagenesis, see, e.g., in Kunkel, Proc. Natl. Acad. Sci. USA 82: 488- 492 (1985), U. S. Patent No. 5,071, 743, Fukuoka et al., Biochem. Biophys. Res. Commun. 263: 357-360 (1999); Kim and Maas, BioTech. 28: 196-198 (2000); Parikh and Guengerich, BioTech. 24: 4 28-431 (1998); Ray and Nickoloff, BioTech. 13: 342-346 (1992); Wang et al., BioTech. 19: 556-559 (1995); Wang and Malcolm, BioTech. 26: 680-682 (1999); Xu and Gong, BioTech. 26: 639-641 (1999), U.S. Patents Nos. 5,789, 166 and 5,932, 419, Hogrefe, Strategies 14. 3: 74-75 (2001), U. S. Patents Nos. 5,702,931, 5,780,270, and 6,242,222, Angag and Schutz, Biotech. 30: 486-488 (2001), Wang and Wilkinson, Biotech. 29: 976-978 (2000), Kang et al., Biotech. 20: 44-46 (1996), Ogel and McPherson, Protein Engineer. 5: 467-468 (1992), Kirsch and Joly, Nucl. Acids. Res. 26: 1848-1850 (1998), Rhem and Hancock, J. Bacteriol. 178: 3346-3349 (1996), Boles and Miogsa, Curr. Genet. 28: 197-198 (1995), Barrenttino et al., Nuc. Acids. Res. 22: 541-542 (1993), Tessier and Thomas, Meths. Molec. Biol. 57: 229-237, and Pons et al., Meth. Molec. Biol. 67: 209-218.

### Tolerizing Human or Humanized MHC Molecules

In exemplary aspects, genetically modified non-human animals, i.e. rats or mice, express and are tolerized to at least one empty human or humanized MHC molecule, or at least the empty human peptide binding groove thereof, but can generate antigen-binding proteins, e.g., antigen-binding proteins comprising human or humanized variable domains, to the human(ized) MHC molecule when it is complexed with an antigenic, e.g., heterologous, peptide. In some aspects, tolerization of a non-human animal to an empty human(ized) MHC molecule is achieved by genetically modifying the non-human animal to comprise in its genome a nucleotide sequence encoding the human(ized) MHC molecule, or at least the human peptide binding groove thereof, such that the non-human animal expresses the human(ized) MHC molecule, or at least the human peptide binding groove thereof, as an empty human(ized) MHC molecule, or empty human peptide binding groove thereof. The same animal genetically modified to comprise the nucleotide encoding the human(ized) MHC molecule may be further modified to comprise humanized immunoglobulin heavy and/or light chain loci that express human or humanized antigen-binding proteins, e.g., antigen-binding proteins having human or humanized variable domains.

MHC molecules are generally classified into two categories: class I and class II MHC molecules. An MHC class I molecule is an integral membrane protein comprising a glycoprotein heavy chain, also referred to herein as the α chain, which has three extracellular domains (i.e., α1, α2 and α3) and two intracellular domains (i.e., a transmembrane domain (TM) and a cytoplasmic domain (CYT)). The heavy chain is noncovalently associated with a soluble subunit called β2 microglobulin (β2m or β2M). An MHC class II molecule or MHC class II protein is a heterodimeric integral membrane protein comprising one α chain and one β chain in noncovalent association. The α chain has two extracellular domains (α1 and α2), and two intracellular domains (a TM domain and a CYT domain). The β chain contains two extracellular domains (β1 and β2), and two intracellular domains (a TM domain and CYT domain).

The domain organization of class I and class II MHC molecules forms the antigenic determinant binding site, e.g., the peptide-binding portion or peptide binding groove, of the MHC molecule. A peptide binding groove refers to a portion of an MHC protein that forms a cavity in which a peptide, e.g., antigenic determinant, can bind. The conformation of a peptide binding groove is capable of being altered upon binding of a peptide to enable proper alignment of amino acid residues important for TCR binding to the peptide-MHC (pMHC) complex.

In some embodiments, MHC molecules include fragments of MHC chains that are sufficient to form a peptide binding groove. For example, a peptide binding groove of a class I protein can comprise portions of the α1 and α2 domains of the heavy chain capable of forming two β-pleated sheets and two α helices. Inclusion of a portion of the β2 microglobulin chain stabilizes the MHC class I molecule. While for most versions of MHC Class II molecules, interaction of the α and β chains can occur in the absence of a peptide, the two-chain molecule of MHC Class I is unstable until the binding groove is filled with a peptide. A peptide binding groove of a class II protein can comprise portions of the α1 and β1 domains capable of forming two β-pleated sheets and two α helices. A first portion of the α1 domain forms a first β-pleated sheet and a second portion of the α1 domain forms a first α helix. A first portion of the β1 domain forms a second β-pleated sheet and a second portion of the β1 domain forms a second α helix. The X-ray crystallographic structure of class II protein with a peptide engaged in the binding groove of the protein shows that one or both ends of the engaged peptide can project beyond the MHC protein (Brown et al., pp. 33-39, 1993, Nature, Vol. 364). Thus, the ends of the α1 and β1 α helices of class II form an open cavity such that the ends of the peptide bound to the binding groove are not buried in the cavity. Moreover, the X-ray crystallographic structure of class II proteins shows that the N-terminal end of the MHC β chain apparently projects from the side of the MHC protein in an unstructured manner since the first 4 amino acid residues of the β chain could not be assigned by X-ray crystallography.

Many human and other mammalian MHCs are well known in the art.

In some embodiments, a non-human animal comprises at least one of a first, second, and/or third nucleotide sequence, each of which encodes a different human or humanized MHC polypeptide selected from the group consisting of a human or humanized MHC II α polypeptide, a human or humanized MHC II β polypeptide, and a human or humanized MHC I α polypeptide; the non-human animal may also comprise a human or humanized β2 microglobulin, e.g., in those embodiments when it comprises a nucleotide sequence encoding a human or humanized MHC I α polypeptide. Use of the first, second, and third designations herein is not to be construed as limiting the non-human animals disclosed herein as requiring all three nucleotide sequences or the presence of any of the human or humanized MHC polypeptides in any specific order.

In various aspects, described herein is a genetically modified non-human animal, i.e. a mouse or a rat comprising in its genome a nucleotide sequence encoding a human or humanized MHC I polypeptide and/or a nucleotide sequence encoding human or humanized MHC II protein, or at least the human peptide binding grooves thereof. The MHC I nucleotide sequence may encode an MHC I polypeptide that is fully human (e.g., a human HLA class I molecule), or a humanized MHC I polypeptide that is partially human and partially non-human (e.g., chimeric human/non-human MHC I polypeptide), and the MHC II nucleotide sequence may encode an MHC II protein that is fully human (e.g., a human HLA class II molecule), or a humanized MHC class II protein that is partially human and partially non-human, (e.g., chimeric human/non-human MHC II protein, e.g., comprising chimeric human/non-human MHC II α and β polypeptides).

In the Examples herein, it is shown that genetically modified animals expressing from an endogenous locus a chimeric human/non-human MHC I molecule comprising a human extracellular portion (that comprises a human peptide binding domain) of a human HLA class I molecule operably linked to transmembrane and cytoplasmic domains of a non-human MHC I molecule are tolerized to the human extracellular portion, e.g., human peptide binding domain, of the human HLA class I molecule when it is empty, and are able to produce a specific immune response to the human peptide binding domain when it is complexed with an antigen, e.g., a peptide heterologous to the non-human animal. *See, e.g., Examples.* As such, in some aspects, the rats, or mice,
(1) express, e.g., from an endogenous MHC locus, a chimeric human/non-human MHC molecule comprising (a) at least a human peptide-binding groove, e.g., a human extracellular portion of a human HLA molecule operably linked to (b) non-human transmembrane and cytoplasmic domains of a non-human MHC I molecule, and
(2) are tolerized to at least the extracellular portion of the human HLA molecule.

A genetically modified non-human animal comprising in its genome, e.g., at the endogenous locus, a nucleotide sequence encoding a chimeric human/non-human MHC I polypeptide is disclosed in U.S. Patent Nos. 9,591,835 and 9,615,550. A genetically modified non-human animal comprising in its genome, e.g., at the endogenous locus, a nucleotide sequence encoding humanized, e.g., chimeric human/non-human, MHC II polypeptides is disclosed in U.S. Patent Nos. 8,847,005 and 9,043,996. A genetically modified non-human animal comprising in its genome, e.g., at the endogenous locus, a nucleotide sequence encoding a humanized, e.g., a chimeric human/non-human, MHC I polypeptide and comprising in its genome, e.g., at the endogenous locus, a nucleotide sequence encoding humanized, e.g., chimeric human/non-human, MHC II polypeptides, is disclosed in U.S. Patent No. 10,154,658.

In various aspects described herein is a genetically modified non-human animal comprising in its genome, e.g., in its germline genome, e.g., at one or more endogenous MHC loci:
(i) a first nucleotide sequence encoding a chimeric human/non-human MHC I polypeptide comprising a human portion that comprises an extracellular portion (or part thereof, e.g., one or more extracellular domains, e.g., the peptide binding groove) of a human MHC I polypeptide operably linked to a non-human portion that comprises transmembrane and cytoplasmic domains of a non-human MHC I polypeptide, e.g., an endogenous MHC I polypeptide; and/or
(ii) a second nucleotide sequence encoding a chimeric human/non-human MHC II α polypeptide comprising a human portion operably linked to a non-human portion, wherein the human portion of the chimeric MHC II α polypeptide comprises an extracellular portion (or part thereof, e.g., one or more extracellular domains, e.g., the α1 domain) of an α polypeptide of a human MHC class II molecule and a third nucleotide sequence encoding a chimeric human/non-human MHC II β polypeptide comprising a human portion operably linked to a non-human portion, wherein the human portion of the chimeric MHC II β polypeptide comprises an extracellular portion (or part thereof, e.g., one or more extracellular domains, e.g., at least the β1 domain) of a β polypeptide of the human MHC class II molecule;
wherein the non-human animal expresses the chimeric human/non-human MHC I and/or MHC II proteins and is tolerized to the chimeric human/non-human MHC I and/or MHC II proteins. In one aspect, the first, second, and/or third nucleotide sequences are respectively located the endogenous non-human MHC I, MHC II α and MHC II β loci. In one embodiment, wherein the non-human animal is a mouse, the first, second, and/or third nucleotide sequences are located at the endogenous mouse MHC locus on mouse chromosome 17. In one embodiment, the first nucleotide sequence is located at the endogenous non-human MHC I locus. In one embodiment, the second nucleotide sequence is located at the endogenous non-human MHC II α locus. In one embodiment, the third nucleotide sequence is located at the endogenous non-human MHC II β locus.

In one embodiment, the chimeric human/non-human MHC I polypeptide comprises a human portion operably linked to a non-human portion, wherein the human portion comprises at least a peptide binding groove of a human MHC I polypeptide. In one embodiment, the human portion of the chimeric polypeptide comprises an extracellular portion of a human MHC I molecule. In this embodiment, the human portion of the chimeric polypeptide comprises an extracellular domain of an α chain of a human MHC I molecule. In one embodiment, the human portion of the chimeric polypeptide comprises α1 and α2 domains of a human MHC I molecule. In another embodiment, the human portion of the chimeric polypeptide comprises α1, α2, and α3 domains of a human MHC I molecule.

In one embodiment, a human portion of the chimeric MHC II α polypeptide and/or a human portion of the chimeric MHC II β polypeptide comprises a peptide binding domain of a human MHC II α polypeptide and/or human MHC II β polypeptide, respectively, e.g., the MHC II α and β polypeptides of a human MHC II protein. In one embodiment, a human portion of the chimeric MHC II α and/or β polypeptide comprises an extracellular portion of a human MHC II α and/or β polypeptide, respectively, e.g., the MHC II α and β polypeptides of a human MHC II protein. In one embodiment, a human portion of the chimeric MHC II α polypeptide comprises α1 domain of a human MHC II α polypeptide; in another embodiment, a human portion of the chimeric MHC II α polypeptide comprises α1 and α2 domains of a human MHC II α polypeptide. In an additional embodiment, a human portion of the chimeric MHC II β polypeptide comprises β1 domain of a human MHC II β polypeptide; in another embodiment, a human portion of the chimeric MHC II β polypeptide comprises β1 and β2 domains of a human MHC II β polypeptide.

Non-human animals in exemplary aspects comprise, express, and are tolerized to the chimeric human/non-human MHC molecules. In one embodiment, wherein the human portion respectively comprises the extracellular portion of a human MHC I, MHC II α, and/or MHC II β molecule, the human portion is operably linked to a non-human portion, wherein the non-human portion of a chimeric human/non-human MHC I, MHC II α and/or MHC II β polypeptide(s) comprises transmembrane and/or cytoplasmic domains of an endogenous non-human (e.g., rodent, e.g., mouse, rat, etc.) MHC I, MHC II α and/or MHC II β polypeptide(s), respectively. Thus, the non-human portion of the chimeric human/non-human MHC I polypeptide may comprise transmembrane and/or cytoplasmic domains of an endogenous non-human MHC I polypeptide. The non-human portion of a chimeric MHC II α polypeptide may comprise transmembrane and/or cytoplasmic domains of an endogenous non-human MHC II α polypeptide. The non-human portion of a chimeric human/non-human MHC II β polypeptide may comprise transmembrane and/or cytoplasmic domains of an endogenous non-human MHC II β polypeptide. In one embodiment, the non-human animal is mouse, and a non-human portion of the chimeric MHC I polypeptide is derived from a mouse H-2K protein. In one embodiment, the non-human animal is a mouse, and non-human portions of the chimeric MHC II α and β polypeptides are derived from a mouse H-2E protein. Thus, a non-human portion of the chimeric MHC I polypeptide may comprise transmembrane and cytoplasmic domains derived from a mouse H-2K, and non-human portions of the chimeric MHC II α and β polypeptides may comprise transmembrane and cytoplasmic domains derived from a mouse H-2E protein. Although specific H-2K and H-2E sequences are contemplated in the Examples, any suitable sequences, e.g., polymorphic variants, conservative/non-conservative amino acid substitutions, etc., are encompassed herein.

In one aspect, a human portion of the chimeric human/mouse MHC I polypeptide comprises a peptide binding domain or an extracellular domain of a human MHC I (e.g., human HLA-A, e.g., human HLA-A2, e.g., human HLA-A2.1). The peptide binding groove of the human MHC I may comprise α1 and α2 domains. Alternatively, the peptide binding groove of the human MHC I may comprise α1, α2, and α3 domains. In one embodiment, the extracellular domain of the human MHC I comprises an extracellular domain of a human MHC I α chain. In one embodiment, the endogenous mouse MHC I locus is an H-2K (e.g., H-2Kb) locus, and the mouse portion of the chimeric MHC I polypeptide comprises transmembrane and cytoplasmic domains of a mouse H-2K (e.g., H-2Kb) polypeptide. Thus, in one embodiment, the mouse of the invention comprises at its endogenous mouse MHC I locus a nucleotide sequence encoding a chimeric human/mouse MHC I, wherein a human portion of the chimeric polypeptide comprises an extracellular domain of a human HLA-A2 (e.g., HLA-A2.1) polypeptide and a mouse portion comprises transmembrane and cytoplasmic domains of a mouse H-2K (e.g., H-2Kb) polypeptide (see, e.g., SEQ ID NO:24), and a mouse expresses a chimeric human/mouse HLA-A2/H-2K protein. In other embodiments, the mouse portion of the chimeric MHC I polypeptide may be derived from other mouse MHC I, e.g., H-2D, H-2L, etc.; and the human portion of the chimeric MHC I polypeptide may be derived from other human MHC I, e.g., HLA-B, HLA-C, etc.

In one aspect, a human portion of the chimeric human/mouse MHC II α polypeptide comprises a human MHC II α peptide binding or extracellular domain and a human portion of the chimeric human/mouse MHC II β polypeptide comprises a human MHC II β peptide binding or extracellular domain. The peptide-binding domain of the human MHC II α polypeptide may comprise α1 domain and the peptide-binding domain of the human MHC II β polypeptide may comprise a β1 domain; thus, the peptide-binding domain of the chimeric MHC II molecule may comprise human α1 and β1 domains. The extracellular domain of the human MHC II α polypeptide may comprise α1 and α2 domains and the extracellular domain of the human MHC II β polypeptide may comprise β1 and β2 domains; thus, the extracellular domain of the chimeric MHC II molecule may comprise human α1, α2, β1 and β2 domains. In one embodiment, the mouse portion of the chimeric MHC II molecule comprises transmembrane and cytosolic domains of mouse MHC II, e.g. mouse H-2E (e.g., transmembrane and cytosolic domains of mouse H-2E α and β chains). Thus, in one embodiment, the mouse of the invention comprises at its endogenous mouse MHC II locus a nucleotide sequence encoding a chimeric human/mouse MHC II α, wherein a human portion of the chimeric MHC II α polypeptide comprises an extracellular domain derived from an α chain of a human MHC II (e.g., α chain of HLA-DR2) and a mouse portion comprises transmembrane and cytoplasmic domains derived from an α chain of a mouse MHC II (e.g., H-2E); and a mouse comprises at its endogenous mouse MHC II locus a nucleotide sequence encoding a chimeric human/mouse MHC II β, wherein a human portion of the chimeric MHC II β polypeptide comprises an extracellular domain derived from a β chain of a human MHC II (e.g., β chain of HLA-DR2) and a mouse portion comprises transmembrane and cytoplasmic domains derived from a β chain of a mouse MHC II (e.g., H-2E); e.g., wherein the mouse expresses a chimeric human/mouse HLA-DR2/H-2E protein. In other embodiment, the mouse portion of the chimeric MHC II protein may be derived from other mouse MHC II, e.g., H-2A, etc.; and the human portion of the chimeric MHC II protein may be derived from other human MHC II, e.g., HLA-DQ, etc.

In some embodiments, a chimeric human/non-human polypeptide may be such that it comprises a human or a non-human leader (signal) sequence. In one embodiment, the chimeric MHC I polypeptide comprises a non-human leader sequence of an endogenous MHC I polypeptide. In one embodiment, the chimeric MHC II α polypeptide comprises a non-human leader sequence of an endogenous MHC II α polypeptide. In one embodiment, the chimeric MHC II β polypeptide comprises a non-human leader sequence of an endogenous MHC II β polypeptide. In an alternative embodiment, the chimeric MHC I, MHC II α and/or MHC II β polypeptide(s) comprises a non-human leader sequence of MHC I, MHC II α and/or MHC II β polypeptide(s), respectively, from another non-human animal, e.g., another rodent or another mouse strain. Thus, the nucleotide sequence encoding the chimeric MHC I, MHC II α and/or MHC II β polypeptide may be operably linked to a nucleotide sequence encoding a non-human MHC I, MHC II α and/or MHC II β leader sequence, respectively. In yet another embodiment, the chimeric MHC I, MHC II α and/or MHC II β polypeptide(s) comprises a human leader sequence of human MHC I, human MHC II α and/or human MHC II β polypeptide, respectively (e.g., a leader sequence of human HLA-A2, human HLA-DRα and/or human HLA-DRβ1*1501, respectively).

In some embodiments, a chimeric human/non-human MHC I, MHC II α and/or MHC II β polypeptide may comprise in its human portion a complete or substantially complete extracellular domain of a human MHC I, human MHC II α and/or human MHC II β polypeptide, respectively. Thus, a human portion may comprise at least 80%, at least 85%, at least 90%, e.g., 95% or more of the amino acids encoding an extracellular domain of a human MHC I, human MHC II α and/or human MHC II β polypeptide (e.g., human HLA-A2, human HLA-DRα and/or human HLA-DRβ1*1501). In one example, substantially complete extracellular domain of the human MHC I, human MHC II α and/or human MHC II β polypeptide lacks a human leader sequence. In another example, the chimeric human/non-human MHC I, chimeric human/non-human MHC II α and/or the chimeric human/non-human MHC II β polypeptide comprises a human leader sequence.

Moreover, in some embodiments, the chimeric MHC I, MHC II α and/or MHC II β polypeptide may be operably linked to (e.g., be expressed under the regulatory control of) endogenous non-human promoter and regulatory elements, e.g., mouse MHC I, MHC II α and/or MHC II β regulatory elements, respectively. Such arrangement will facilitate proper expression of the chimeric MHC I and/or MHC II polypeptides in the non-human animal, e.g., during immune response in the non-human animal.

Although the Examples provided herein show tolerization to human peptide binding domains of chimeric human/non-human MHC molecules expressed from endogenous MHC loci, such tolerization also occurs in non-human animals expressing a human MHC molecule (or the functional peptide binding domain thereof) from an ectopic locus (data not shown). Additionally, non-human animals expressing and tolerized against an empty human MHC molecule (or the empty peptide binding domain thereof) from an ectopic locus are able to generate a specific immune response against the human HLA molecule (or peptide binding domain thereof, or derivative thereof) from which the expressed human MHC molecule is derived when the non-human animal is immunized with the human HLA molecule (or peptide binding domain thereof and/or derivative thereof) complexed with an antigenic peptide, e.g., a peptide heterologous to the non-human animal (data not shown).

Without wishing to be bound by theory, it is believed that tolerization of the non-human animal occurs upon expression of the human or humanized MHC molecules. As such, it is not necessary that the human or humanized MHC molecules are expressed from an endogenous locus. Accordingly, in various aspects described herein is a genetically modified non-human animal comprising in its genome a first nucleotide sequence encoding a human(ized) MHC I polypeptide (or portions and/or derivatives thereof), a second nucleotide sequence encoding a human(ized) MHC II α polypeptide (or portions and/or derivatives thereof), and/or a third nucleotide sequence encoding a human(ized) MHC II β polypeptide (or portions and/or derivatives thereof); wherein the non-human animal expresses the human(ized) MHC I, MHC II α, and/or MHC II β polypeptides (or portions and/or derivatives thereof) and is tolerized to the human(ized) polypeptides or portions and/or derivatives. In one aspect, the first, second, and/or third nucleotide sequence(s) do(es) not respectively disrupt endogenous non-human MHC I, MHC II α and MHC II β loci, e.g., is located at an ectopic locus, e.g., the ROSA26 locus. In some embodiments, a genetically modified mouse comprises at an ectopic locus, e.g., a ROSA26 locus, a nucleotide sequence encoding a chimeric human/mouse MHC I, wherein a human portion of the chimeric polypeptide comprises an extracellular domain of a human HLA-A2 (e.g., HLA-A2.1) polypeptide and a mouse portion comprises transmembrane and cytoplasmic domains of a mouse H-2K (e.g., H-2Kb) polypeptide (see, e.g., SEQ ID NO:24), and the mouse expresses and is tolerized to a chimeric human/mouse HLA-A2/H-2K protein.

Moreover, if from an ectopic locus, fully human MHC molecules or fully human portions and/or derivatives thereof may be expressed. Accordingly, in various aspects described herein is a genetically modified non-human animal comprising in its genome a first nucleotide sequence encoding a fully human MHC I polypeptide (or fully human portions and/or derivatives thereof), a second nucleotide sequence encoding a fully human MHC II α polypeptide (or fully human portions and/or derivatives thereof), and/or a third nucleotide sequence encoding a fully human MHC II β polypeptide (or fully human portions and/or derivatives thereof); wherein the non-human animal expresses the fully human MHC I, MHC II α, and/or MHC II β polypeptides (or fully human portions and/or derivatives thereof) and is tolerized to the polypeptides or fully human portions and/or derivatives thereof. In one aspect, the first, second, and/or third nucleotide sequence(s) do(es) not respectively disrupt endogenous non-human MHC I, MHC II α and MHC II β loci, optionally does not disrupt any endogenous loci, e.g., is located at an ectopic locus, e.g., the ROSA26 locus

In some aspects, the human or humanized MHC I polypeptide may be derived from, e.g., the human or humanized MHC I polypeptide is encoded by a nucleic acid that encodes or comprises a portion of a nucleotide sequence that encodes, a functional human HLA molecule selected from the group consisting of HLA-A, HLA-B, HLA-C, HLA-E, HLA-F, HLA-G, and a combination thereof. A human or humanized MHC II α or β polypeptide may be derived from the α or β polypeptides of a functional human HLA molecule encoded by an of HLA-DP, -DQ, and -DR loci. A list of commonly used HLA antigens and alleles is described in Shankarkumar et al. ((2004) The Human Leukocyte Antigen (HLA) System, Int. J. Hum. Genet. 4(2):91-103). Shankarkumar et al. also present a brief explanation of HLA nomenclature used in the art. Additional information regarding HLA nomenclature and various HLA alleles can be found in Holdsworth et al. (2009) The HLA dictionary 2008: a summary of HLA-A, -B, -C, - DRB1/3/4/5, and DQB1 alleles and their association with serologically defined HLA-A, -B, -C, - DR, and -DQ antigens, Tissue Antigens 73:95-170, and a recent update by Marsh et al. (2010) Nomenclature for factors of the HLA system, 2010, Tissue Antigens 75:291-455. In some embodiments, the MHC I or MHC II polypeptides may be derived from any functional human HLA-A, B, C, DR, or DQ molecules. Thus, the human or humanized MHC I and/or II polypeptides may be derived from any functional human HLA molecules in exemplary embodiments. In some aspects, all MHC I and MHC II polypeptides expressed on a cell surface comprise a portion derived from human HLA molecules.

Of particular interest are polymorphic human HLA alleles, known to be associated with a number of human diseases, e.g., human autoimmune diseases. In fact, specific polymorphisms in HLA loci have been identified that correlate with development of rheumatoid arthritis, type I diabetes, Hashimoto's thyroiditis, multiple sclerosis, myasthenia gravis, Graves' disease, systemic lupus erythematosus, celiac disease, Crohn's disease, ulcerative colitis, and other autoimmune disorders. See, e.g., Wong and Wen (2004) What can the HLA transgenic mouse tell us about autoimmune diabetes?, Diabetologia 47:1476-87; Taneja and David (1998) HLA Transgenic Mice as Humanized Mouse Models of Disease and Immunity, J. Clin. Invest. 101:921-26; Bakker et al. (2006), A high-resolution HLA and SNP haplotype map for disease association studies in the extended human MHC, Nature Genetics 38:1166-72 and Supplementary Information; and International MHC and Autoimmunity Genetics Network (2009) Mapping of multiple susceptibility variants within the MHC region for 7 immune-mediated diseases, Proc. Natl. Acad. Sci. USA 106:18680-85. Thus, in some embodiments, the human or humanized MHC I and/or II polypeptides may be derived from a human HLA molecule known to be associated with a particular disease, e.g., autoimmune disease.

In one specific embodiment, the human or humanized MHC I polypeptide is derived from human HLA-A. In a specific embodiment, the HLA-A polypeptide is an HLA-A2 polypeptide (e.g., and HLA-A2.1 polypeptide). In one embodiment, the HLA-A polypeptide is a polypeptide encoded by an HLA-A*0201 allele, e.g., HLA-A*02:01:01:01 allele. The HLA-A*0201 allele is commonly used amongst the North American population. Although the present Example describe this particular HLA sequence, any suitable HLA-A sequence is encompassed herein, e.g., polymorphic variants of HLA-A2 exhibited in human population, sequences with one or more conservative or non-conservative amino acid modifications, nucleotide sequences differing from the sequence in exemplary embodiments herein due to the degeneracy of genetic code, etc.

In another specific embodiment, the human or humanized MHC I polypeptide is derived from human MHC I selected from HLA-B and HLA-C. In one embodiment, it is derived from HLA-B, e.g., HLA-B27. In another embodiment, it is derived from HLA-A3, -B7, -Cw6, etc.

In one specific embodiment, the human or humanized MHC II α and β polypeptides are derived from human HLA-DR, e.g., HLA-DR2. Typically, HLA-DR α chains are monomorphic, e.g., the α chain of HLA-DR protein is encoded by HLA-DRA gene (e.g., HLA-DRα*01 gene). On the other hand, the HLA-DR β chain is polymorphic. Thus, HLA-DR2 comprises an α chain encoded by HLA-DRA gene and a β chain encoded by HLA-DR1β*1501 gene. Any suitable HLA-DR sequences are encompassed herein, e.g., polymorphic variants exhibited in human population, sequences with one or more conservative or non-conservative amino acid modifications, etc.

In some embodiments, the human or humanized MHC II α and/or β polypeptide may be encoded by nucleotide sequences, or portions thereof, of HLA alleles known to be associated with common human diseases. Such HLA alleles include, but are not limited to, HLA-DRB1*0401, -DRB1*0301, -DQA1*0501, -DQB1*0201, DRB1*1501, -DRB1*1502, - DQB1*0602, -DQA1*0102, -DQA1*0201, -DQB1 *0202, -DQA1*0501, and combinations thereof. For a summary of HLA allele/disease associations, see Bakker et al. (2006), supra.

In further aspects, a non-human animal described herein, e.g., a rodent, e.g., a rat or a mouse, comprises (e.g., at an endogenous β2 microglobulin locus) a nucleotide sequence encoding a human or humanized β2 microglobulin. β2 microglobulin or the light chain of the MHC class I protein (also abbreviated "β2M") is a small (12 kDa) non-glycosylated protein, that functions primarily to stabilize the MHC I α chain. Generation of human or humanized β2 microglobulin animals is described in detail in U.S. Patent No. 9,615,550.

In some embodiments, the nucleotide sequence encoding the human or humanized β2 microglobulin polypeptide may comprise nucleotide residues corresponding to only a portion of the human β2 microglobulin gene, e.g., at least a portion that helps stabilize the human(ized) MHC I molecule. In some embodiments, the nucleotide sequence encoding the human β2 microglobulin polypeptide comprises the entire human β2 microglobulin gene. Alternatively, in some embodiments, the nucleotide sequence may comprise nucleotide residues encoding amino acid sequence set forth in amino acids 21-119 of a human β2 microglobulin protein (i.e., amino acid residues corresponding to the mature human β2 microglobulin). In an alternative embodiment, the nucleotide sequence may comprise nucleotide residues encoding amino acid sequence set forth in amino acids 23-115 of a human β2 microglobulin protein, for example, amino acid sequence set forth in amino acids 23-119 of a human β2 microglobulin protein. The nucleic and amino acid sequences of human β2 microglobulin are described in Gussow et al., (1987) The β2-Microglobulin Gene. Primary Structure and Definition of the Transcriptional Unit, J. Immunol. 139:3131-38.

Thus, in some embodiments, the human or humanized β2 microglobulin polypeptide may comprise amino acid sequence set forth in amino acids 23-115 of a human β2 microglobulin polypeptide, e.g., amino acid sequence set forth in amino acids 23-119 of a human β2 microglobulin polypeptide, e.g., amino acid sequence set forth in amino acids 21-119 of a human β2 microglobulin polypeptide. Alternatively, the human β2 microglobulin may comprise amino acids 1-119 of a human β2 microglobulin polypeptide.

In some embodiments, the nucleotide sequence encoding a human or humanized β2 microglobulin comprises a nucleotide sequence set forth in exon 2 to exon 4 of a human β2 microglobulin gene. Alternatively, the nucleotide sequence comprises nucleotide sequences set forth in exons 2, 3, and 4 of a human β2 microglobulin gene. In this embodiment, the nucleotide sequences set forth in exons 2, 3, and 4 are operably linked to allow for normal transcription and translation of the gene. Thus, in one embodiment, the human sequence comprises a nucleotide sequence corresponding to exon 2 to exon 4 of a human β2 microglobulin gene. In a specific embodiment, the human sequence comprises a nucleotide sequence corresponding to exon 2 to about 267 bp after exon 4 of a human β2 microglobulin gene. In a specific embodiment, the human sequence comprises about 2.8 kb of a human β2 microglobulin gene.

Thus, in some embodiments, the human or humanized β2 microglobulin polypeptide may be encoded by a nucleotide sequence comprising nucleotide sequence set forth in exon 2 to exon 4 of a human β2 microglobulin, e.g., nucleotide sequence corresponding to exon 2 to exon 4 of a human β2 microglobulin gene. Alternatively, in some embodiments, the polypeptide may be encoded by a nucleotide sequence comprising nucleotide sequences set forth in exons 2, 3, and 4 of a human β2 microglobulin gene. In a specific embodiment, the human or humanized β2 microglobulin polypeptide is encoded by a nucleotide sequence corresponding to exon 2 to about 267 bp after exon 4 of a human β2 microglobulin gene. In another specific embodiment, the human or humanized polypeptide is encoded by a nucleotide sequence comprising about 2.8 kb of a human β2 microglobulin gene. As exon 4 of the β2 microglobulin gene contains the 5' untranslated region, the human or humanized polypeptide may be encoded by a nucleotide sequence comprising exons 2 and 3 of the β2 microglobulin gene.

In addition, in some aspects, a non-human animal comprising a nucleotide sequence encoding a human or humanized β2 microglobulin protein also comprises a nucleotide sequence set forth in exon 1 of a non-human β2 microglobulin gene. Thus, in a specific aspect, the non-human animal comprises in its genome a nucleotide sequence encoding a human or humanized β2 microglobulin wherein the nucleotide sequence comprises exon 1 of a non-human β2 microglobulin and exons 2, 3, and 4 of a human β2 microglobulin gene. Thus, the human or humanized β2 microglobulin polypeptide is encoded by exon 1 of a non-human β2 microglobulin gene and exons 2, 3, and 4 of a human β2 microglobulin gene (e.g., exons 2 and 3 of a human β2 microglobulin gene).

In some aspects, the nucleotide sequence encoding the human or humanized β2 microglobulin is at the endogenous non-human animal β2 microglobulin locus. In some aspects, the nucleotide sequence of a human β2 microglobulin replaces the corresponding nucleotide sequences encoding the endogenous non-human β2 microglobulin at the endogenous non-human β2 microglobulin locus. For example, in some embodiments, a nucleotide sequence corresponding to exon 2 to exon 4 of a human β2 microglobulin gene replaces an endogenous mouse sequence corresponding to exon 2 to exon 4 of a mouse β2 microglobulin gene at the endogenous mouse β2 microglobulin locus (See, Fig. 1C). In some embodiments, a nucleotide sequence comprising nucleotide sequences set forth in exons 2, 3, and 4 of a human β2 microglobulin gene replaces nucleotide sequences set forth in exons 2, 3, and 4 of a mouse β2 microglobulin gene, etc.

In some aspects, the nucleotide sequence encoding the human or humanized β2 microglobulin does not disrupt an endogenous non-human β2 microglobulin locus, e.g., is located at an ectopic locus, e.g., the ROSA26 locus.

In some aspects, a genetically modified non-human animal comprises in its genome, at an ectopic locus, e.g., the ROSA26 locus, a first nucleotide sequence and/or second nucleotide sequence, wherein the first nucleotide sequence encodes a single chain polypeptide comprising a functional peptide binding portion of an HLA class I molecule (e.g., at least the α1 and α2 domains of a human MHC class I molecule), optionally operably linked, e.g., fused, with human or humanized β2 microglobulin (or a portion thereof), and wherein the second nucleotide sequence encodes a single chain polypeptide comprising a functional peptide binding portion of an HLA class II molecule (e.g., at least the α1 and β1 domains of a human MHC class II molecule). In some aspects, a genetically modified non-human animal comprises a first nucleotide sequence encoding a single chain polypeptide comprising at least a functional peptide binding portion of a human HLA-A2 polypeptide (e.g., a full-length mature HLA-A2 polypeptide) fused with a human β2 microglobulin, e.g., at an ectopic locus (see, e.g., FIG. 2). In some aspects, a single chain polypeptide comprising at least a functional peptide binding portion of a human HLA-A2 polypeptide (e.g., a full-length mature HLA-A2 polypeptide) fused with a human β2 microglobulin comprises an amino acid sequence set forth as SEQ ID NO: 18, SEQ ID NO:20, or SEQ ID NO:23. In some aspects, a genetically modified non-human animal as described herein comprises at an ectopic locus, e.g., at a ROSA26 locus, a nucleotide sequence encoding a human(ized) MHC class I molecule comprising at least a functional peptide binding portion of a human HLA-A2 molecule fused with a human(ized) β2 microglobulin, e.g., a nucleotide sequence set forth as SEQ ID NO: 17, SEQ ID NO: 19, SEQ ID NO:22, or a degenerate variant thereof. In some aspects, a genetically modified non-human animal as described herein comprises as an ectopic locus, e.g., at a ROSA26 locus, a nucleotide sequence encoding a chimeric MHC polypeptide, e.g., HLA-A2/H2-K polypeptide set forth as SEQ ID NO:24.

It would be understood by those of ordinary skill in the art that although some aspects include specific nucleotide and amino acid sequences to generate genetically engineered animals, sequences of one or more conservative or non-conservative amino acid substitutions, or sequences differing from the aspects herein due to the degeneracy of the genetic code, may also be considered.

Therefore, in some aspects, a non-human animal that expresses a human(ized) MHC class I α polypeptide nucleic acid sequence is described, wherein the human(ized) MHC class I α polypeptide nucleic acid sequence, or a portion thereof, is not identical to a human MHC class I α polypeptide nucleic acid sequence due to the degeneracy of the genetic code, but which is at least 85%, 90%, 95%, 96%, 97%, 98%, or 99% identical. In a specific aspect, the human(ized) MHC class I α polypeptide nucleic acid sequence is at least 90%, 95%, 96%, 97%, 98%, or 99% identical to the human MHC class I α polypeptide nucleic acid sequence of aspects exemplified herein. In one aspect, the expressed human(ized) MHC class I α polypeptide sequence comprises one or more conservative substitutions. In one aspect, the human(ized) MHC class I α polypeptide sequence comprises one or more non-conservative substitutions.

Additionally, in some aspects, a non-human animal that expresses a human(ized) β2 microglobulin sequence is described, wherein the human(ized) β2 microglobulin sequence, or a portion thereof, is not identical to a human β2 microglobulin sequence due to the degeneracy of the genetic code, but which is at least 85%, 90%, 95%, 96%, 97%, 98%, or 99% identical. In a specific aspect, the human(ized) β2 microglobulin sequence is at least 90%, 95%, 96%, 97%, 98%, or 99% identical to the human β2 microglobulin sequence of aspects exemplified herein. In one aspect, the human(ized) β2 microglobulin sequence comprises one or more conservative substitutions. In one aspect, the human(ized) β2 microglobulin sequence comprises one or more non-conservative substitutions.

In some aspects, a non-human animal that expresses a human(ized) MHC class II α polypeptide nucleic acid sequence is described, wherein the human(ized) MHC class II α polypeptide nucleic acid sequence, or a portion thereof, is not identical to a human MHC class I α polypeptide nucleic acid sequence due to the degeneracy of the genetic code, but which is at least 85%, 90%, 95%, 96%, 97%, 98%, or 99% identical. In one aspect, the expressed human(ized) MHC class II α polypeptide sequence comprises one or more conservative substitutions. In one aspect, the human(ized) MHC class II α polypeptide sequence comprises one or more non-conservative substitutions.

In some aspects, a non-human animal that expresses a human(ized) MHC class II β polypeptide nucleic acid sequence is described, wherein the human(ized) MHC class II β polypeptide nucleic acid sequence, or a portion thereof, is not identical to a human MHC class I α polypeptide nucleic acid sequence due to the degeneracy of the genetic code, but which is at least 85%, 90%, 95%, 96%, 97%, 98%, or 99% identical. In one aspect, the expressed human(ized) MHC class II α polypeptide sequence comprises one or more conservative substitutions. In one aspect, the human(ized) MHC class II α polypeptide sequence comprises one or more non-conservative substitutions

In some aspects, a non-human animal is heterozygous for a first, second, and/or third nucleotide sequence, each of which encodes a different human or humanized MHC polypeptide selected from the group consisting of a human or humanized MHC II α polypeptide, a human or humanized MHC II β polypeptide, and a human or humanized MHC I α polypeptide, or portions thereof. In some aspects, a non-human animal is homozygous for a first, second, and/or third nucleotide sequence, each of which encodes a different human or humanized MHC polypeptide selected from the group consisting of a human or humanized MHC II α polypeptide, a human or humanized MHC II β polypeptide, and a human or humanized MHC I α polypeptide, or portions thereof.

### Human or Humanized B cell immune responses

In some aspects, genetically modified non-human animal, in addition to comprising a first, second, and/or third nucleotide sequence, each of which encodes a different human or humanized MHC polypeptide selected from the group consisting of a human or humanized MHC II α polypeptide, a human or humanized MHC II β polypeptide, and a human or humanized MHC I α polypeptide, or portions thereof, e.g., also comprises a human or humanized immunoglobulin heavy and/or light chain loci, such that the non-human animal is capable of providing human or humanized antigen-binding proteins comprising a human or humanized antigen-binding domain, e.g., human or humanized variable domains.

Immunoglobulin loci comprising human variable region gene segments are known in the art and can be found, for example, in U. S. Pat. Nos. 5,633,425; 5,770,429; 5,814,318; 6,075,181; 6,114,598; 6,150,584; 6,998,514; 7,795,494; 7,910,798; 8,232,449; 8,502,018; 8,697,940; 8,703,485; 8,754,287; 8,791,323; 8,809,051; 8,907,157; 9,035,128; 9,145,588;9,206,263; 9,447,177; 9,551,124; 9,580,491 and 9,475,559, as well as in U.S. Pat. Pub. Nos. 20100146647, 20110195454, 20130167256, 20130219535, 20130326647, 20130096287, and 2015/0113668, and in PCT Pub. Nos. WO2007117410, WO2008151081, WO2009157771, WO2010039900, WO2011004192, WO2011123708 and WO2014093908.

In some aspects, non-human animals as disclosed herein comprise, in addition to the nucleotide sequence encoding a human or humanized MHC molecule, exogenously introduced fully human immunoglobulin transgenes, which are able to rearrange in precursor B cells in mice (Alt et al., 1985, Immunoglobulin genes in transgenic mice, Trends Genet 1:231-236). In these aspects, fully human immunoglobulin transgenes may be (randomly) inserted and endogenous immunoglobulin genes may also be knocked-out (Green et al., 1994, Antigen-specific human monoclonal antibodies from mice engineered with human Ig heavy and light chain YACs, Nat Genet 7:13-21; Lonberg et al., 1994, Antigen-specific human antibodies from mice comprising four distinct genetic modifications, Nature 368:856-859; Jakobovits et al., 2007, From XenoMouse technology to panitumumab, the first fully human antibody product from transgenic mice, Nat Biotechnol 25:1134-1143) e.g., wherein endogenous immunoglobulin heavy chain and κ light chain loci are inactivated, e.g., by targeted deletion of small but critical portions of each endogenous locus, followed by introduction of human immunoglobulin gene loci as randomly integrated large transgenes, or minichromosomes (Tomizuka et al., 2000, Double trans-chromosomic mice: maintenance of two individual human chromosome fragments containing Ig heavy and kappa loci and expression of fully human antibodies, PNAS USA 97:722-727).

In some embodiments, human or humanized immunoglobulin heavy and light chain loci are at endogenous immunoglobulin heavy and light chain loci, respectively. A method for a large in situ genetic replacement of the mouse germline immunoglobulin variable gene loci with human germline immunoglobulin variable gene loci while maintaining the ability of the mice to generate offspring has been previously described. *See, e.g.,* U.S. Patent Nos. 6,596,541 and 8,697,940. Specifically, the precise replacement of six megabases of both the mouse heavy chain and κ light chain immunoglobulin variable gene loci with their human counterparts while leaving the mouse constant regions intact is described. As a result, mice have been created that have a precise replacement of their entire germline immunoglobulin variable repertoire with equivalent human germline immunoglobulin variable sequences, while maintaining mouse constant regions. The human variable regions are linked to mouse constant regions to form chimeric human-mouse immunoglobulin loci that rearrange and express at physiologically appropriate levels. The antibodies expressed are "reverse chimeras," i.e., they comprise human variable region sequences and mouse constant region sequences. These mice having humanized immunoglobulin variable regions that express antibodies having human or humanized variable regions and mouse constant regions are called VELOCIMMUNE^{®} mice.

VELOCIMMUNE^{®} humanized mice exhibit a fully functional humoral immune system that is essentially indistinguishable from that of wild-type mice. They display normal cell populations at all stages of B cell development. They exhibit normal lymphoid organ morphology. Antibody sequences of VELOCIMMUNE^{®} mice exhibit normal V(D)J rearrangement and normal somatic hypermutation frequencies. Antibody populations in these mice reflect isotype distributions that result from normal class switching (e.g., normal isotype cis-switching). Immunizing VELOCIMMUNE^{®} mice results in robust humoral immune responses that generate large, diverse antibody repertoires having human immunoglobulin variable domains suitable for use as therapeutic candidates. This platform provides a plentiful source of naturally affinity-matured human immunoglobulin variable region sequences for making pharmaceutically acceptable antibodies and other antigen-binding proteins. It has also been shown that replacement of even a single endogenous V_{H} gene segment with a human V_{H} gene segment can result in an immune response comprising humanized immunoglobulin variable domain. *See, e.g.,* Tien et al. (2016) Cell 166: 1471-84. It is the precise replacement of mouse immunoglobulin variable sequences with human immunoglobulin variable sequences such that the human immunoglobulin variable sequences are operably linked with endogenous non-human constant region gene sequence(s) in a reverse chimeric manner that allows for making VELOCIMMUNE^{®} mice.

Mice modified in a reverse chimeric manner include mice modified to comprise at an endogenous immunoglobulin locus a human(ized) variable region (e.g., comprising (D), J, and one or more human V gene segments) operably linked to an endogenous constant region, e.g.,
(a) at an endogenous heavy chain locus:
   (i) an unrearranged human(ized) immunoglobulin heavy chain variable region in operable linkage to an endogenous heavy chain constant region, wherein the unrearranged human(ized) immunoglobulin heavy chain variable region comprises a plurality of unrearranged human heavy chain variable region V_{H} gene segments (e.g., all functional human unrearranged human V_{H} gene segments), one or more unrearranged immunoglobulin heavy chain D_{H} gene segments and one or more unrearranged immunoglobulin heavy chain J_{H} gene segments,
      optionally wherein the one or more unrearranged immunoglobulin heavy chain D_{H} gene segments and one or more unrearranged immunoglobulin heavy chain J_{H} gene segments are one or more unrearranged human immunoglobulin heavy chain D_{H} gene segments (e.g., all functional human D_{H} gene segments) and/or one or more unrearranged human immunoglobulin heavy chain J_{H} gene segments (e.g., all functional human J_{H} gene segments);
   (ii) a restricted unrearranged human(ized) heavy chain variable region in operable linkage to an endogenous heavy chain constant region, wherein the restricted unrearranged human(ized) heavy chain variable region consists essentially of a single unrearranged human heavy chain variable region V_{H} gene segment operably linked with one or more unrearranged immunoglobulin heavy chain D_{H} gene segments and one or more unrearranged immunoglobulin heavy chain J_{H} gene segments, optionally wherein the one or more unrearranged immunoglobulin heavy chain D_{H} gene segments and one or more unrearranged immunoglobulin heavy chain J_{H} gene segments are one or more unrearranged human immunoglobulin heavy chain D_{H} gene segments and/or one or more unrearranged human immunoglobulin heavy chain J_{H} gene segments, respectively;
   (iii) a common heavy chain encoding sequence comprising a rearranged human(ized) heavy variable region sequence in operable linkage to an endogenous heavy chain constant region, wherein the rearranged human(ized) heavy chain variable region sequence comprises a human heavy chain variable region V_{H} gene segment rearranged with an immunoglobulin heavy chain D_{H} gene segment, which is rearranged with an immunoglobulin heavy chain J_{H} gene segment,
      optionally wherein the immunoglobulin heavy chain J_{H} gene segment or the immunoglobulin heavy chain D_{H} gene segment are a human immunoglobulin heavy chain D_{H} gene segment and/or a human immunoglobulin heavy chain J_{H} gene segment, respectively;
   (iv) a histidine modified unrearranged human(ized) heavy chain variable region in operable linkage to an endogenous heavy chain constant region, wherein the histidine modified unrearranged human(ized) heavy chain variable region comprises an unrearranged immunoglobulin heavy chain variable gene sequence comprising in a complementarity determining region 3 (CDR3) encoding sequence a substitution of at least one non-histidine codon with a histidine codon or an insertion of at least one histidine codon;
   (v) a heavy chain only immunoglobulin encoding sequence comprising an unrearranged human(ized) heavy chain variable region in operable linkage to an endogenous heavy chain constant region, wherein the endogenous heavy chain constant region comprises (1) an intact endogenous IgM gene that encodes an IgM isotype that associates with light chain and (2) a non-IgM gene, e.g., an IgG gene, lacking a sequence that encodes a functional CH1 domain, wherein the non-IgM gene encodes a non-IgM isotype lacking a CH1 domain capable of covalently associating with a light chain constant domain; or
   (vi) an unrearranged human(ized) hybrid heavy chain sequence encoding a hybrid immunoglobulin chain, wherein the unrearranged human(ized) hybrid heavy chain sequence comprises unrearranged human light chain variable (V_{L}) and unrearranged joining (J_{L}) gene segments in operable linkage to an endogenous heavy chain constant region,
   optionally wherein the endogenous heavy chain constant region comprises (1) an intact endogenous IgM gene that encodes an IgM isotype that associates with light chain and (2) a non-IgM gene, e.g., an IgG gene, lacking a sequence that encodes a functional CH1 domain, wherein the non-IgM gene encodes a non-IgM isotype lacking a CH1 domain capable of covalently associating with a light chain constant domain;
   and/or
(b) at an endogenous light chain locus:
   (i) an unrearranged human(ized) immunoglobulin light chain variable region in operable linkage to an endogenous light chain constant region, wherein the unrearranged human(ized) immunoglobulin light chain variable region comprises a plurality of unrearranged human light chain variable region V_{L} gene segments (e.g., all functional human unrearranged human V_{L} gene segments) and one or more unrearranged immunoglobulin light chain J_{L} gene segments,
      optionally wherein the one or more unrearranged immunoglobulin light chain J_{L} gene segments are one or more unrearranged human immunoglobulin light chain J_{L} gene segments (e.g., all functional human J_{H}L gene segments),
      optionally wherein the endogenous immunoglobulin light chain locus is an endogenous immunoglobulin light chain kappa (κ) locus, the unrearranged human(ized) immunoglobulin light chain variable region comprises human variable κ (V_{κ}) and joining κ (J_{κ}) gene segments, and wherein the endogenous light chain constant region is an endogenous κ chain constant region sequence and/or wherein the endogenous immunoglobulin light chain locus is an endogenous immunoglobulin light chain lambda (λ), the unrearranged human(ized) immunoglobulin light chain variable region comprises human variable λ (V_{λ}) and joining λ (J_{λ}) gene segments, and the endogenous light chain constant region is an endogenous λ chain constant region sequence, optionally wherein the endogenous immunoglobulin light chain λ locus comprises (a) one or more human V_{λ} gene segments, (b) one or more human J_{λ} gene segments, and (c) one or more human C_{λ} gene segments, wherein (a) and (b) are operably linked to (c) and a rodent immunoglobulin light chain constant (C_{λ}) gene segment, and wherein the endogenous immunoglobulin λ light chain locus further comprises: one or more rodent immunoglobulin λ light chain enhancers (Eλ), and one or more human immunoglobulin λ light chain enhancers (Eλ), optionally comprising three human Eλs;
   (ii) a common light chain encoding sequence comprising a rearranged human(ized) light chain variable region sequence in operable linkage to an endogenous light chain constant region, wherein the rearranged human(ized) light chain variable region sequence comprises a human light chain variable region V_{L} gene segment rearranged with an immunoglobulin light chain J_{L} gene segment;
   (iii) a restricted unrearranged human(ized) light chain variable region in operable linkage to an endogenous light chain constant region, wherein the restricted unrearranged human(ized) light chain variable region comprises no more than two unrearranged human immunoglobulin light chain variable (V_{L}) gene segments operably linked to one or more unrearranged human immunoglobulin light chain joining (J_{L}) gene segments;
   (iv) a histidine modified unrearranged human(ized) light chain variable region in operable linkage to an endogenous light chain constant region, wherein the histidine modified unrearranged human(ized) light chain variable region comprises an unrearranged human(ized) immunoglobulin light chain variable gene sequence comprising in a complementarity determining region 3 (CDR3) encoding sequence a substitution of at least one non-histidine codon with a histidine codon or an insertion of at least one histidine codon; or
   (v) a histidine modified rearranged human(ized) light chain variable region in operable linkage to an endogenous light chain constant region, wherein the histidine modified rearranged human(ized) light chain variable region comprises a rearranged human(ized) immunoglobulin light chain variable gene sequence comprising in a complementarity determining region 3 (CDR3) encoding sequence a substitution of at least one non-histidine codon with a histidine codon or an insertion of at least one histidine codon,

optionally wherein the mouse further comprises
   (i) a human(ized) immunoglobulin heavy chain locus comprising a functional ADAM6 gene such that the mouse exhibits wildtype fertility of the non-human animal; and/or
   (ii) an exogenous terminal deoxynucleotidyl transferase (TdT) gene for increased antigen receptor diversity, optionally such that at least 10% of the rearranged variable region genes comprise non-template additions,
have been previously described. *See, e.g.,* U.S. Patent Nos. 8,697,940; 8,754,287; 9,204,624; 9,334,334; 9,801,362; 9,332,742; and 9,516,868; U.S. Patent Publications 20110195454, 20120021409, 20120192300, 20130045492; 20150289489; 20180125043; 20180244804; PCT Publication No. WO2017210586, and WO2011163314; Lee et al. (2014) Nature Biotechnology 32:356.

In some aspects, the present disclosure includes a genetically modified non-human animal whose genome, e.g., germline genome, comprises:
an endogenous immunoglobulin locus comprising an immunoglobulin heavy chain variable region comprising a human V_{H} gene segment, a human D_{H} gene segment, and a human J_{H} gene segment, wherein the immunoglobulin heavy chain variable region is operably linked to a constant region, and/or
an endogenous chain locus comprising an immunoglobulin light chain variable region comprising a human VL gene segment and a human JL gene segments, wherein the immunoglobulin light chain variable region is operably linked to a constant region.

In some aspects, the present disclosure includes a genetically modified non-human animal whose genome, e.g., germline genome, comprises:
an endogenous immunoglobulin heavy chain locus comprising an immunoglobulin heavy chain variable region comprising a human V_{H} gene segment, a human D_{H} gene segment, and a human J_{H} gene segment, wherein the immunoglobulin heavy chain variable region is operably linked to a constant region, and/or
an endogenous immunoglobulin light chain locus comprising an immunoglobulin light chain variable region comprising a human VL gene segment and a human JL gene segments, wherein the immunoglobulin light chain variable region is operably linked to a constant region.

In some aspects, a non-human animal, e.g., a rodent, e.g., a rat or a mouse, in addition to the nucleotide sequence encoding a human or humanized MHC, comprises in its genome a replacement of one or more endogenous V_{H}, D_{H}, and J_{H} segments at an endogenous immunoglobulin heavy chain locus with one or more human V_{H}, D_{H}, and J_{H} segments, wherein the one or more human V_{H}, D_{H}, and J_{H} segments are operably linked to an endogenous immunoglobulin heavy chain gene; and optionally an unrearranged or rearranged human V_{L} and human J_{L} segment operably linked to a non-human, e.g., rodent, e.g., a mouse or rat, or human immunoglobulin light chain constant (C_{L}) region gene, e.g., at an endogenous non-human light chain locus, such that the non-human animal is tolerized against the human or humanized MHC molecule and produces reverse chimeric antibodies in response to an immunogen, e.g., an antigenic pMHC complex.

In certain aspects, the genetically modified non-human animals that express and are tolerized to a human or humanized MHC molecule also comprise in their genome, e.g., germline genome, an immunoglobulin locus (exogenous or endogenous) containing an immunoglobulin variable region comprising one or more unrearranged human immunoglobulin variable region gene segments and an immunoglobulin constant region comprising an immunoglobulin constant region gene and in which the one or more unrearranged human immunoglobulin variable region gene segments are operably linked to the immunoglobulin constant region gene. In some aspects, the non-human animals that express and are tolerized to a human or humanized MHC molecule comprise in their genome, e.g., germline genome multiple such immunoglobulin loci. For example, in some aspects, the genetically modified non-human animals comprise in their genome, e.g., germline genome, a nucleotide sequence encoding a human or humanized MHC molecule and one or more immunoglobulin loci (including genetically modified rearranged or unrearranged immunoglobulin loci) such that the mice make human, humanized, partially human, and/or reverse chimeric (human variable and non-human constant regions) antibodies.

Generally, a genetically modified immunoglobulin locus comprises an immunoglobulin variable region (comprising immunoglobulin variable region gene segments) operably linked to an immunoglobulin constant region. In some aspects, the genetically modified immunoglobulin locus comprises one or more human unrearranged immunoglobulin heavy chain variable region gene segments operably linked to a heavy chain constant region gene. In some aspects, the genetically modified immunoglobulin locus comprises human unrearranged immunoglobulin light chain, e.g., κ, gene segments operably linked to a heavy chain constant region gene, see, e.g., U.S. Patent No. 9,516,868. In some aspects, the genetically modified immunoglobulin locus comprises human unrearranged immunoglobulin heavy chain variable region gene segments operably linked to a κ chain constant region gene. In some aspects, the genetically modified immunoglobulin locus comprises human unrearranged immunoglobulin variable region κ gene segments operably linked to a κ chain constant region gene. In some aspects, the genetically modified immunoglobulin locus comprises human unrearranged immunoglobulin variable region λ gene segments operably linked to a κ chain constant region gene. In some aspects, the genetically modified immunoglobulin locus comprises human unrearranged immunoglobulin variable region λ gene segments operably linked to a λ chain constant region gene.

In certain aspects, the non-human animal comprises at an endogenous heavy chain locus an unrearranged human(ized) immunoglobulin heavy chain variable region in operable linkage to an endogenous heavy chain constant region, wherein immunoglobulin variable region contains one or more unrearranged human Ig heavy chain variable region gene segments. In some aspects, the one or more unrearranged human Ig variable region gene segments comprises at least one human immunoglobulin heavy chain variable (V_{H}) segment, one or more immunoglobulin heavy chain diversity (D_{H}) segments (optionally one or more unrearranged human D_{H} segments), and one or more immunoglobulin heavy chain joining (J_{H}) segments (optionally one or more unrearranged human J_{H} segments). In some aspects, the unrearranged human Ig variable region gene segments comprise a plurality of unrearranged human V_{H} segments, one or more unrearranged (human) D_{H} segments and one or more unrearranged (human) J_{H} segments. In some aspects, the unrearranged human Ig variable region gene segments comprise at least 3 V_{H} gene segments, at least 18 V_{H} gene segments, at least 20 V_{H} gene segments, at least 30 V_{H} gene segments, at least 40 V_{H} gene segments, at least 50 V_{H} gene segments, at least 60 V_{H} gene segments, at least 70 V_{H} gene segments, or at least 80 V_{H} gene segments. In some aspects, the unrearranged human Ig gene segments include all of the functional human D_{H} gene segments. In some aspects, the unrearranged human Ig gene segments include all of the functional human J_{H} gene segments. Exemplary variable regions comprising Ig heavy chain gene segments are provided, for example, in Macdonald et al, Proc. Natl. Acad. Sci. USA 111 :5147-52 and supplemental information.

In some aspects, the non-human animals described herein comprise at an endogenous heavy chain locus a restricted unrearranged human(ized) heavy chain variable region in operable linkage to an endogenous heavy chain constant region comprising at least a non-human IgM gene, wherein the restricted unrearranged human(ized) heavy chain variable region is characterized by a single human V_{H} gene segment, a plurality of D_{H} gene segments (e.g., human D_{H} gene segments) and a plurality of J_{H} gene segments (e.g. human J_{H} gene segments), wherein the restricted immunoglobulin heavy chain locus is capable of rearranging and forming a plurality of distinct rearrangements, wherein each rearrangement is derived from the single human V_{H} gene segment, one of the D_{H} segments, and one of the J_{H} segments, and wherein each rearrangement encodes a different heavy chain variable domain (e.g., as described in U.S. Pat. Pub. No. 20130096287). In some aspects the single human V_{H} gene segment is V_{H}1-2 or V_{H}1-69.

In certain aspects, a non-human animal comprises at an endogenous light chain locus an unrearranged human(ized) immunoglobulin light chain variable region in operable linkage to an endogenous light chain constant region. In some aspects the unrearranged human(ized) immunoglobulin light chain variable region contains unrearranged human Ig κ variable region gene segments. In some aspects, the unrearranged human(ized) immunoglobulin variable region comprises a plurality of unrearranged human Vκ segments and one or more unrearranged human J_{K} segments. In some aspects, the unrearranged human immunoglobulin variable region gene segments comprise all of the human Jκ segments. In some aspects, the immunoglobulin variable region gene segments comprise four functional Vκ segments and all human Jκ segments. In some aspects, the immunoglobulin variable region gene segments comprise 16 functional Vκ segments and all human Jκ segments (e.g., all functional human Vκ segments and Jκ segments). In some aspects, the unrearranged human immunoglobulin variable region gene segments comprise all of the human Vκ segments and all human Jκ segments. Exemplary variable regions comprising Ig κ gene segments are provided, for example, in Macdonald et al, Proc. Natl. Acad. Sci. USA 1 11 :5147-52 and supplemental information.

In some aspects, a restricted unrearranged human(ized) light chain variable region in operable linkage to an endogenous light chain constant region is characterized in that the unrearranged human(ized) light chain variable region comprises no more than two human V_{L} gene segments and a plurality of J_{L} gene segments (e.g., dual light chain mice, or DLC, as described in U. S. Pat. No. 9,796,788). In some aspects the V_{L} gene segments are Vκ gene segments. In some aspects the V_{L} gene segments are Vλ gene segments. In some aspects the Vκ gene segments are IGKV3-20 and IGKV1 -39. In some aspects, a non-human animal comprises exactly two unrearranged human Vκ gene segments and five unrearranged human Jκ gene segments operably linked to a mouse light chain constant region at the endogenous κ light chain loci of the mouse, optionally wherein the exactly two unrearranged human Vκ gene segments are a human Vκ1-39 gene segment and a human Vκ3-20 gene segment, wherein the five unrearranged human Jκ gene segments are a human Jκ1 gene segment, a human Jκ2 gene segment, a human Jκ3 gene segment, a human Jκ4 gene segment, and a human Jκ5 gene segment, wherein the unrearranged human kappa light chain gene segments are capable of rearranging and encoding human variable domains of an antibody, and optionally further wherein the non-human animal does not comprise an endogenous Vκ gene segment that is capable of rearranging to form an immunoglobulin light chain variable region.

In certain aspects, the unrearranged human(ized) immunoglobulin light chain variable region in operable linkage to an endogenous light chain constant region contains unrearranged human Igλ variable region gene segments. In some aspects, the unrearranged human immunoglobulin variable region gene segments comprise a plurality of human Vλ segments and one or more human Jλ segments. In some aspect, the unrearranged human immunoglobulin variable region gene segments comprise one or more human Vλ segments, one or more human Jλ segments, and one or more human Cλ constant region sequences. In some aspects, the unrearranged human immunoglobulin variable region gene segments comprise all of the human Vλ segments. In some aspects, the unrearranged human immunoglobulin variable region gene segments comprise all of the human Jλ segments. Exemplary variable regions comprising Ig λ gene segments are provided, for example, U. S. Pat. Nos. 9,035,128 and 6,998,514. In some aspects, the unrearranged human(ized) immunoglobulin light chain variable region in operable linkage to an endogenous light chain constant region comprises (a) one or more human Vλ gene segments, (b) one or more human Jλ gene segments, and (c) one or more human Cλ gene segments, wherein (a) and (b) are operably linked to (c) and an endogenous (e.g., rodent) Cλ gene segment, and wherein the endogenous immunoglobulin λ light chain locus further comprises: one or more rodent immunoglobulin λ light chain enhancers (Eλ), and one or more human immunoglobulin λ light chain enhancers (Eλ), optionally comprising three human Eλ.

In certain aspects, the unrearranged human(ized) immunoglobulin light chain variable region in operable linkage to an endogenous light chain constant region comprises an unrearranged human Igλ variable region gene segments operably linked to an endogenous (e.g., rodent, e.g., rat or mouse) Cκ gene such that the non-human animal expresses an immunoglobulin light chain that comprises a human λ variable domain sequence derived from the Vλ and Jλ gene segments fused with an endogenous κ constant domain, see, e.g., US Patent No. 9,226,484.

In some aspects, the immunoglobulin variable region comprising unrearranged human immunoglobulin variable region gene segments also includes human immunoglobulin variable region intergenic sequences. In some aspects, the immunoglobulin variable region includes non-human (e.g., rodent, rat, mouse) Ig variable region intergenic sequences. In some aspects, the intergenic sequence is of endogenous species origin.

In some aspects, the immunoglobulin variable region is a rearranged heavy chain variable region (a universal heavy chain variable region or a common heavy chain encoding sequence). In some aspects, the rearranged Ig heavy chain variable region gene is a human rearranged Ig heavy chain variable region gene. Exemplary rearranged Ig heavy chain variable regions are provided in U.S. Patent Pub. No. 20140245468 and U.S. Patent Nos.: 9,204,624 and 9,930,871. In some aspects, the non-human organism comprising a universal heavy chain variable region is used to produce bispecific antibodies. In some aspects, a non-human animal comprises at an endogenous immunoglobulin heavy chain locus a common heavy chain encoding sequence, e.g., a rearranged human immunoglobulin heavy chain variable region nucleotide sequence operably linked to an endogenous immunoglobulin constant region gene sequence, e.g., a rearranged human immunoglobulin heavy chain variable region nucleotide sequence operably linked to an endogenous heavy chain constant region gene sequence, wherein the rearranged heavy chain variable region nucleotide sequence encodes the sequence of V_{H}3-23/X₁X₂/J_{H}, wherein X₁ is any amino acid, and X₂ is any amino acid, wherein the non-human animal expresses an immunoglobulin heavy chain variable domain that is derived from the rearranged human immunoglobulin heavy chain variable region nucleotide sequence, e.g., the V_{H}3-23/X₁X₂/J_{H} gene, linked to the endogenous heavy chain constant region gene sequence, and optionally cognate with a human immunoglobulin light chain variable domain.

In some aspects, the immunoglobulin variable region is a rearranged light variable region (a universal light chain variable region). In some aspects, the rearranged Ig light chain variable region gene is a human rearranged Ig light chain variable region gene. Exemplary rearranged Ig light chain variable regions are provided in, e.g., U.S. Patent Nos.: 9,969,814; 10,130,181, and 10,143,186 and U.S. Patent Pub. Nos. 20120021409, 20120192300, 20130045492, 20130185821, 20130302836, and 20150313193. In some aspects, the non-human organism ("universal light chain" organism) comprising a universal light chain variable region is used to produce bispecific antibodies. In some aspects, a common light chain encoding sequence comprises a single rearranged human immunoglobulin light chain Vκ/Jκ sequence operably linked to an endogenous light chain constant region, wherein the single rearranged human immunoglobulin light chain Vκ/Jκ sequence is either (i) a human Vκ1-39/Jκ5 sequence comprising a human Vκ1-39 gene segment fused to a human Jκ5 gene segment, or (ii) a human Vκ3-20/Jκ1 sequence comprising a human Vκ3-20 gene segment fused to a human Jκ1 gene segment.

In some aspects, the immunoglobulin variable region is a light chain and/or a heavy chain immunoglobulin variable region that includes insertions and/or replacements of histidine codons designed to introduce pH- dependent binding properties to the antibodies generated in such non-human organism. In some of such aspects, the histidine codons are inserted and/or replaced in the nucleic acid sequences encoding CDR3. Various such light and/or heavy immunoglobulin loci are provided in U.S. Patent Nos. 9,301,510; 9,334,334; and 9,801,362 and U.S. Patent Application Publication No. 20140013456. In some aspects, the histidine modified rearranged human(ized) light chain variable region in operable linkage to an endogenous light chain constant region comprises a single rearranged human immunoglobulin light chain variable region gene sequence comprising human Vκ and Jκ segment sequences, optionally wherein the Vκ segment sequence is derived from a human Vκ1-39 or Vκ3-20 gene segment, and wherein the single rearranged human immunoglobulin light chain variable region gene sequence comprises a substitution of at least one non-histidine codon of the Vκ segment sequence with a histidine codon that is expressed at a position selected from the group consisting of 105, 106, 107, 108, 109, 111 and a combination thereof (according to IMGT numbering). In some aspects, the histidine modified unrearranged human(ized) heavy chain variable region in operable linkage to an endogenous heavy chain constant region comprises an unrearranged human(ized) immunoglobulin heavy chain variable gene sequence comprising in a complementarity determining region 3 (CDR3) encoding sequence a substitution of at least one non-histidine codon with a histidine codon or an insertion of at least one histidine codon. In some aspects, the unrearranged human(ized) immunoglobulin heavy chain variable gene sequence comprises unrearranged human V_{H}, unrearranged human D_{H} or synthetic D_{H}, and unrearranged human J_{H} gene segments, optionally wherein the unrearranged human D_{H} or synthetic D_{H} gene segment comprises the substitution of at least one non-histidine codon with a histidine codon or an insertion of at least one histidine codon. In some aspects, the histidine modified unrearranged human(ized) light chain variable region in operable linkage to an endogenous heavy chain constant region comprises unrearranged V_{L} and unrearranged J_{L} gene segments. In some aspects, the histidine modified unrearranged human(ized) light chain variable region comprises no more than two unrearranged human V_{L} (e.g., no more than two Vκ gene segments) and one or more unrearranged human J_{L} (e.g., Jκ) gene segment(s), wherein each of the no more than two human V_{L} gene segments comprises in a CDR3 encoding sequence a substitution of at least one non-histidine codon with a histidine codon or an insertion of at least one histidine codon. In some aspects, the no more than two unrearranged human Vκ gene segments are human Vκ1-39 and Vκ3-20 gene segments each comprising one or more substitutions of a non-histidine codon with a histidine codon, and wherein the human Vκ and Jκ gene segments are capable of rearranging and the human Vκ and Jκ gene segments encode a human light chain variable domain comprising one or more histidines at a position selected from the group consisting of 105, 106, 107, 108, 109, 111 (according to IGMT numbering), and a combination thereof, wherein the one or more histidines are derived from the one or more substitutions.

In some aspects, the immunoglobulin constant region comprises a heavy chain constant region gene. In some aspects, the heavy chain constant region gene is a human heavy chain constant region gene. In some aspects, the heavy chain constant region gene is of endogenous species origin. In some aspects, the heavy chain constant region gene is a mouse constant region gene or a rat constant region gene. In some aspects, the constant region gene is a mixture of human and non-human sequence. For example, in some aspects, the constant region gene encodes a human CH1 region and a non-human (e.g., endogenous species origin, mouse, rat) CH2 and/or CH3 region. In some aspects, the heavy chain constant region gene is an Cµ, Cδ, Cy (Cyl, Cy2, Cy3, Cy4), Cα or Cε constant region gene. In some aspects, the constant region gene is an endogenous constant region gene. In some aspects, the constant region gene encodes a mutated CH1 region so that the non-human animal expresses heavy chain only antibodies (see., e.g., U.S. Patent No. 8,754,287, U.S. Patent Application Publication No. 2015/0289489). In some aspects, e.g., where the goal is to generate heavy chains to make bispecific antibodies (e.g., in universal or dual light chain organisms), the Fc domains of the heavy chains comprise modifications to facilitate heavy chain heterodimer formation and/or to inhibit heavy chain homodimer formation. Such modifications are provided, for example, in U.S. Pat. Nos. 5,731,168; 5,807,706; 5,821,333; 7,642,228 and 8,679,785 and in U.S. Pat. Pub. No. 2013/0195849.

In some aspects, the immunoglobulin constant region comprises a light chain constant region gene. In some aspects, the light chain constant region gene is a κ constant region gene. In some aspects, the light chain constant region gene is a λ constant region gene. In some aspects, the light chain constant region gene is of endogenous species origin. In some aspects, the light chain constant region gene is a mouse constant region gene or a rat constant region gene. In some aspects, the light chain constant region gene is a mixture of human and non-human sequence.

In some aspects, the immunoglobulin variable region comprising human variable region gene segments and the immunoglobulin constant region gene to which the variable region gene segments are operably linked are located at an endogenous immunoglobulin locus. In some aspects, the endogenous immunoglobulin locus is an endogenous heavy chain locus. In some aspects, the endogenous immunoglobulin locus is an endogenous κ locus. In some aspects, the endogenous immunoglobulin locus is an endogenous λ locus. In some aspects, the constant region gene to which the human variable region gene segments are operably linked is an endogenous constant region gene.

In some aspects, one or more of the endogenous immunoglobulin loci or a portion of the one or more endogenous loci (e.g., a variable region and/or a constant region) in the genome of the non-human animal described herein is inactivated. Endogenous immunoglobulin variable region gene loci and portions thereof can be inactivated using any method known in the art, including, but not limited to, the deletion of the locus or a portion thereof from the genome of the organism, the replacement of a locus or a portion thereof with a different nucleic acid sequence, the inversion of a portion of the locus and/or the displacement of a portion of the locus to another position in the genome of the non-human organism. In some aspects the inactivation of the locus is only a partial inactivation. In some aspects, the variable region of the locus is inactivated but the constant region remains functional (e.g., because it is operably linked to non-endogenous variable region gene segments).

In some aspects, the genetically modified non-human animal includes an inactivated endogenous immunoglobulin heavy chain locus. In some aspects, the endogenous immunoglobulin heavy chain locus or a portion thereof is inactivated by deletion, replacement, displacement and/or inversion of at least part of the endogenous variable region of the endogenous heavy chain locus. In some aspects, the at least part of the variable region of the endogenous heavy chain locus that is deleted, replaced, displaced, and/or inverted comprises the J segments of the variable region. In some aspects, the endogenous immunoglobulin heavy chain locus or portion thereof is inactivated by deletion, replacement, displacement and/or inversion of at least part of the endogenous constant region of the endogenous heavy chain locus. In some aspects, the at least part of the constant region of the endogenous heavy chain locus that is deleted, replaced, displaced, and/or inverted comprises the Oµ gene of the endogenous constant region.

In some aspects, the genetically modified non-human animal includes an inactivated endogenous immunoglobulin κ chain locus. In some aspects, the endogenous immunoglobulin κ chain locus or a portion thereof is inactivated by deletion, replacement, displacement and/or inversion of at least part of the endogenous variable region of the endogenous κ chain locus. In some aspects, the at least part of the variable region of the endogenous κ chain locus that is deleted, replaced, displaced, and/or inverted comprises the J segments of the variable region. In some aspects, the endogenous immunoglobulin κ chain locus or portion thereof is inactivated by deletion, replacement, displacement and/or inversion of at least part of the endogenous constant region of the endogenous κ chain locus. In some aspects, the at least part of the constant region of the endogenous κ chain locus that is deleted, replaced, displaced, and/or inverted comprises the Cκ gene of the endogenous constant region.

In some aspects, the genetically modified non-human animal includes an inactivated endogenous immunoglobulin λ chain locus. In some aspects, the endogenous immunoglobulin λ chain locus or a portion thereof is inactivated by deletion, replacement, displacement and/or inversion of at least part of an endogenous variable region of the endogenous λ chain locus. In some aspects, the at least part of at least one V-J-C gene cluster in the endogenous λ chain locus is deleted, replaced, displaced, and/or inverted. In some aspects, the endogenous immunoglobulin λ chain locus or portion thereof is inactivated by deletion, replacement, displacement and/or inversion of at least part of an endogenous constant region of the endogenous λ chain locus. In some aspects, the at least part of the constant region of the endogenous λ chain locus that is deleted, replaced, displaced, and/or inverted comprises a C gene of the endogenous constant region.

In various aspects, the immunoglobulin locus modifications do not affect fertility of the non-human animal. In some aspects, the heavy chain locus comprises a functional, e.g., endogenous ADAM6a gene, ADAM6b gene, or both, and the genetic modification does not affect the expression and/or function of the endogenous ADAM6a gene, ADAM6b gene, or both. In some aspects, the genome of the genetically modified non-human animal further comprises an ectopically located functional, e.g., endogenous ADAM6a gene, ADAM6b gene, or both. Exemplary non-human animals expressing exogenous ADAM6a and/or ADAM6b are described in U.S. Pat. Nos. 8,642,835 and 8,697,940.

In some aspects, the genetically modified non-human animal further comprises and expresses an exogenous terminal deoxynucleotidyl transferase (TdT) for increased antigen receptor diversity. Exemplary non-human animals expressing exogenous TdT are described in PCT Publication WO 2017210586.

In some aspects, the genetically modified non-human animal comprises and expresses a nucleotide sequence encoding a human or humanized MHC molecule, and expresses antibodies having human variable domains (e.g., a human variable domain derived from (e.g., encoded by) rearranged human variable region gene segments), but lacks antibodies that specifically bind an empty human or humanized MHC. In some aspects, the human or humanized variable domain is a human or humanized heavy chain variable domain. In some aspects, the antibodies are heavy chain only antibodies. In some aspects, the human or humanized variable domain is a human or humanized light chain variable domain. In some aspects, the antibodies produced by the non-human animals have both human or humanized heavy chain variable domains, and human or humanized light chain variable domains. In some aspects, the antibodies have human or humanized heavy chain constant domains. In some aspects, the antibodies have human or humanized light chain constant domains. In some aspects, the heavy and/or light chain constant domain is of non-human origin. For example, in some aspects, the heavy chain constant domain is of endogenous species origin. In some aspects, the heavy chain constant domain is of mouse or rat origin. In some aspects, the light chain constant domain is of endogenous species origin. In some aspects, the light chain constant domain is of mouse or rat origin.

### Non-Human Animals, Tissues and Cells

In some aspects, a genetically modified non-human animal described herein may be selected from a group consisting of a mouse, rat, rabbit, pig, bovine (e.g., cow, bull, buffalo), deer, sheep, goat, chicken, cat, dog, ferret, primate (e.g., marmoset, rhesus monkey). For the non-human animals where suitable genetically modifiable ES cells are not readily available, other methods are employed to make a non-human animal comprising the genetic modification. Such methods include, e.g., modifying a non-ES cell genome (e.g., a fibroblast or an induced pluripotent cell) and employing nuclear transfer to transfer the modified genome to a suitable cell, e.g., an oocyte, and gestating the modified cell (e.g., the modified oocyte) in a non-human animal under suitable conditions to form an embryo.

According to the invention, the genetically modified animal is a mouse or rat. In one embodiment, the non-human animal is a rat. In one embodiment, the non-human animal is a mouse.

In a specific embodiment, the non-human animal is a rodent that is a mouse of a C57BL strain selected from C57BL/A, C57BL/An, C57BL/GrFa, C57BL/KaLwN, C57BL/6, C57BL/6J, C57BL/6ByJ, C57BL/6NJ, C57BL/10, C57BL/10ScSn, C57BL/10Cr, and C57BL/O1a. In another embodiment, the mouse is a 129 strain selected from the group consisting of a strain that is 129P1, 129P2, 129P3, 129X1, 129S1 (e.g., 129S1/SV, 129Sl/SvIm), 129S2, 129S4, 129S5, 129S9/SvEvH, 129S6 (129/SvEvTac), 129S7, 129S8, 129T1, 129T2 (see, e.g., Festing *et al.* (1999) Revised nomenclature for strain 129 mice, Mammalian Genome 10:836, *see also,* Auerbach et al (2000) Establishment and Chimera Analysis of 129/SvEv- and C57BL/6-Derived Mouse Embryonic Stem Cell Lines). In an embodiment, the genetically modified mouse is a mix of an aforementioned 129 strain and an aforementioned C57BL/6 strain. In another specific embodiment, the mouse is a mix of aforementioned 129 strains, or a mix of aforementioned BL/6 strains. In a specific embodiment, the 129 strain of the mix is a 129S6 (129/SvEvTac) strain. In another embodiment, the mouse is a BALB strain, e.g., BALB/c strain. In yet another embodiment, the mouse is a mix of a BALB strain and another aforementioned strain. Non-human animals as provided herein may be a mouse derived from any combination of the aforementioned strains.

Germline transmission of a targeted modified allele in rat ES cells was established in the last decade. *See, e.g.,* US Pub. Nos. 20140235933 and 20140310828; Tong et al. (2010) Nature 467:211-215, Tong et al. (2011) Nat Protoc. 6(6): doi:10.1038/nprot.2011.338. As such, in one embodiment, the rat is selected from a Wistar rat, an LEA strain, a Sprague Dawley strain, a Fischer strain, F344, F6, and Dark Agouti. In one embodiment, the rat strain is a mix of two or more strains selected from the group consisting of Wistar, LEA, Sprague Dawley, Fischer, F344, F6, and Dark Agouti.

Thus, in one aspect of the disclosure, a genetically modified mouse is provided, wherein the mouse comprises, e.g., in its genome, e.g., in its germline genome,
(a) a nucleotide sequence encoding a human or humanized MHC molecule or at least a peptide binding portion thereof, and
(b) an (un)rearranged human or humanized immunoglobulin heavy locus and/or an (un)rearranged human or humanized immunoglobulin light chain locus, optionally wherein at least one of the (un)rearranged human or humanized immunoglobulin heavy locus and/or an (un)rearranged human or humanized immunoglobulin light chain locus is unrearranged,

wherein the genetically modified non-human animal expresses the human or humanized MHC molecule or at least a peptide binding portion thereof,
wherein the genetically modified non-human animal expresses immunoglobulins comprising a human or humanized heavy chain variable domain and/or a human or humanized light chain variable domain, and
wherein the non-human animal is tolerized to the human or humanized MHC molecule or at least a peptide binding portion thereof such that it generates a specific B-cell response when immunized with an antigenic peptide-MHC (pMHC) complex that comprises (i) a peptide that is heterologous to the non-human animal complexed with (ii) human HLA molecule from which the human or humanized MHC molecule is derived or a portion thereof.

In some aspects, the mouse comprises a first nucleotide sequence encoding a first fully human or chimeric human/murine MHC polypeptide (e.g., MHC II α), a second nucleotide sequence encoding a second fully human or chimeric human/murine MHC polypeptide (e.g., MHC II β), and/or a third nucleotide sequence encoding a third fully human or a chimeric human/murine MHC polypeptide (e.g., MHC I) and optionally a β2 microglobulin locus encoding a human or humanized β2 microglobulin, and
(a) at an endogenous heavy chain locus:
   (i) an unrearranged human(ized) immunoglobulin heavy chain variable region in operable linkage to an endogenous heavy chain constant region;
   (ii) a restricted unrearranged human(ized) heavy chain variable region in operable linkage to an endogenous heavy chain constant region;
   (iii) a common heavy chain encoding sequence;
   (iv) a histidine modified unrearranged human(ized) heavy chain variable region in operable linkage to an endogenous heavy chain constant region;
   (v) a heavy chain only immunoglobulin encoding sequence; or
   (vi) an unrearranged human(ized) hybrid heavy chain sequence encoding a hybrid immunoglobulin chain;
   and/or
(b) at an endogenous light chain locus:
   (i) an unrearranged human(ized) immunoglobulin light chain variable region in operable linkage to an endogenous light chain constant region;
   (ii) a common light chain encoding sequence;
   (iii) a restricted unrearranged human(ized) light chain variable region in operable linkage to an endogenous light chain constant region;
   (iv) a histidine modified unrearranged human(ized) light chain variable region in operable linkage to an endogenous light chain constant region; or
   (v) a histidine modified rearranged human(ized) light chain variable region in operable linkage to an endogenous light chain constant region,
optionally wherein the mouse further comprises
(i) a human(ized) immunoglobulin heavy chain locus comprising a functional ADAM6 gene such that the mouse exhibits wildtype fertility ; and/or
(ii) an exogenous terminal deoxynucleotidyl transferase (TdT) gene for increased antigen receptor diversity, optionally such that at least 10% of the rearranged variable region genes comprise non-template additions.

In addition to genetically modified animals (mice or rats), also described is a tissue or cell, wherein the tissue or cell is derived from a non-human animal of some aspects, e.g., wherein the tissue or cell comprises (a) a first nucleotide sequence encoding a first fully human or chimeric human/murine MHC polypeptide (e.g., MHC II α), a second nucleotide sequence encoding a second fully human or chimeric human/murine MHC polypeptide (e.g., MHC II β) and/or a third nucleotide sequence encoding a third fully human or a chimeric human/murine MHC polypeptide (e.g., MHC I) and optionally a β2 microglobulin locus encoding a human or humanized β2 microglobulin, and (b) wherein when the cell is not a B cell, an (un)rearranged human or humanized immunoglobulin heavy locus and/or an (un)rearranged human or humanized immunoglobulin light chain locus, optionally wherein at least one of the (un)rearranged human or humanized immunoglobulin heavy locus and/or an (un)rearranged human or humanized immunoglobulin light chain locus is unrearranged.

In some aspects, the tissue or cell expresses the human or humanized MHC molecule and a human or humanized antigen-binding protein and/or a nucleic acid encoding one or more variable domains of the human or humanized antigen-binding protein, wherein the antigen-binding protein specifically binds a pMHC complex that is antigenic to the non-human animal from which the tissue or cell is derived, e.g., wherein the pMHC complex comprises an antigenic peptide complexed with a human MHC (or portion thereof), against the latter of which the non-human animal is generally tolerized. In some aspects, the cell is a B cell. In some aspects, the cell is a hybridoma or quadroma, derived from the fusion of a B cell isolated from a non-human animal of some aspects, and a myeloma cell. In some aspects, the tissue is an antigen-binding protein, or nucleic acid sequence encoding same, wherein the antigen-binding protein specifically binds a pMHC complex that is antigenic to the non-human animal from which the antigen-binding protein is derived, e.g., wherein the pMHC complex comprises an antigenic peptide complexed with a human MHC (or portion thereof), against the latter of which the non-human animal is generally tolerized.

In addition to a genetically engineered non-human animal, a non-human embryo (e.g., a rodent, e.g., a mouse or a rat embryo) is also described, wherein the non-human embryo comprises a donor ES cell that may be used to generate a non-human animal (e.g., a rodent, e.g., a mouse or a rat) in exemplary aspects. In one aspect, the non-human embryo comprises an ES donor cell that comprises a human or humanized MHC I (e.g., MHC I α) nucleotide sequence, a human or humanized MHC II (e.g., MHC II α and/or MHC II β) nucleotide sequence, an (un)rearranged human or humanized immunoglobulin locus (e.g., heavy and/or light chain variable loci), and/or human or humanized β2 microglobulin gene sequence and host embryo cells.

In some aspects, non-human animal (e.g., rodent, e.g., rat or mouse) cells or genomes may be used to generate the non-human animals, e.g., pluripotent cells, embryonic stem (ES) cells, germ cells, etc., wherein the cell or genome comprises
(a) a nucleotide sequence encoding a human or humanized MHC molecule or at least a peptide binding portion thereof, and
(b) an (un)rearranged human or humanized immunoglobulin heavy locus and/or an (un)rearranged human or humanized immunoglobulin light chain locus, optionally wherein at least one of the (un)rearranged human or humanized immunoglobulin heavy locus and/or an (un)rearranged human or humanized immunoglobulin light chain locus is unrearranged,

such that the resulting genetically modified non-human animal expresses the human or humanized MHC molecule or at least a peptide binding portion thereof,
such that the resulting genetically modified non-human animal expresses immunoglobulins comprising a human or humanized heavy chain variable domain and/or a human or humanized light chain variable domain, and
such that the resulting non-human animal is tolerized to the human or humanized MHC molecule or at least a peptide binding portion thereof such that it generates a specific B-cell response when immunized with an antigenic peptide-MHC (pMHC) complex that comprises (i) a peptide that is heterologous to the non-human animal complexed with (ii) human HLA molecule from which the human or humanized MHC molecule is derived or a portion thereof.

In some exemplary aspects, the cell or genome comprises a first nucleotide sequence encoding a first fully human or chimeric human/murine MHC polypeptide (e.g., MHC II α), a second nucleotide sequence encoding a second fully human or chimeric human/murine MHC polypeptide (e.g., MHC II β), and/or a third nucleotide sequence encoding a third fully human or a chimeric human/murine MHC polypeptide (e.g., MHC I) and optionally a β2 microglobulin locus encoding a human or humanized β2 microglobulin, and
(a) at an endogenous heavy chain locus:
   (i) an unrearranged human(ized) immunoglobulin heavy chain variable region in operable linkage to an endogenous heavy chain constant region;
   (ii) a restricted unrearranged human(ized) heavy chain variable region in operable linkage to an endogenous heavy chain constant region;
   (iii) a common heavy chain encoding sequence;
   (iv) a histidine modified unrearranged human(ized) heavy chain variable region in operable linkage to an endogenous heavy chain constant region;
   (v) a heavy chain only immunoglobulin encoding sequence; or
   (vi) an unrearranged human(ized) hybrid heavy chain sequence encoding a hybrid immunoglobulin chain;
   and/or
(b) at an endogenous light chain locus:
   (i) an unrearranged human(ized) immunoglobulin light chain variable region in operable linkage to an endogenous light chain constant region;
   (ii) a common light chain encoding sequence;
   (iii) a restricted unrearranged human(ized) light chain variable region in operable linkage to an endogenous light chain constant region;
   (iv) a histidine modified unrearranged human(ized) light chain variable region in operable linkage to an endogenous light chain constant region; or
   (v) a histidine modified rearranged human(ized) light chain variable region in operable linkage to an endogenous light chain constant region,
optionally wherein the cell or genome further comprises
(i) a human(ized) immunoglobulin heavy chain locus comprising a functional ADAM6 gene such that the mouse exhibits wildtype fertility; and/or
(ii) an exogenous terminal deoxynucleotidyl transferase (TdT) gene for increased antigen receptor diversity such that, optionally, at least 10% of the rearranged variable region genes comprise non-template additions.

Although the Examples that follow describe a genetically engineered animal whose genome comprises a replacement of a nucleic acid sequence encoding mouse H-2K proteins with a nucleic acid sequence encoding a chimeric human/mouse HLA-A2/H-2K, one skilled in the art would understand that a similar strategy may be used to introduce other human(ized) MHC I and II genes (other HLA-A, HLA-B, and HLA-C; and other HLA-DR, HLA-DP and HLA-DQ genes, e.g., at endogenous loci or ectopic loci, e.g., at the ROSA26 locus). Such animals comprising multiple chimeric human/non-human (e.g., human/rodent, e.g., human/mouse) MHC I and MHC II genes at endogenous MHC loci are also provided. Examples of such chimeric MHC I and MHC II proteins are described in U.S. Publication Nos. 20130111617, 20130185819, 20130185820 and 20140245467 and U.S. Patent No. 8,847,005.

Also described is a non-human cell comprising a chromosome or fragment thereof of a non-human animal of aspects of the present disclosure. In one aspect, the non-human cell comprises a nucleus of a non-human animal aspect of the present disclosure. In one aspect, the non-human cell comprises the chromosome or fragment thereof as the result of a nuclear transfer.

### Making Genetically Modified Non-Human Animals

Also described is a method for making a genetically engineered non-human animal (e.g., a genetically engineered rodent, e.g., a mouse or rat). Generally, the methods comprise modifying the genome, e.g., germline genome, of the non-human animal to comprise (a) a first nucleic acid sequence encoding a first chimeric human/non-human MHC polypeptide, a second nucleic acid sequence encoding a second chimeric human/non-human MHC polypeptide, a third nucleic acid sequence encoding a third chimeric human/non-human MHC polypeptide and/or a β2 microglobulin locus encoding a human or humanized β2 microglobulin polypeptide and (b) immunoglobulin heavy and light chain loci that encode for human or humanized antibodies. In some aspects, modifying comprises targeting sequences encoding an endogenous MHC polypeptide extracellular domain, all or a portion of the β2 microglobulin, and immunoglobulin variable regions and replacing them with a human MHC extracellular domain(s), all or a portion of human β2 microglobulin, and human immunoglobulin variable region, respectively.

In some aspects described herein, modifying may comprise breeding, e.g., mating, animals of the same species. In other aspects, modifying comprises sequential homologous recombination in one or more ES cells. In some aspects, the ES cells are derived from non-human animals genetically modified to comprise one or more, but not all, of the genetic modifications desired, and homologous recombination in such ES cells completes the genetic modification. In other aspects, modifying may comprise a combination of breeding and homologous recombination in ES cells, e.g., breeding an animal to another (or more) animal of the same species, wherein some or all of the non-human animals may be generated from ES cells genetically modified via a single homologous recombination or sequential homologous recombination events, and wherein some ES cell may be isolated from a non-human animal comprising one or more of the genetic modifications disclosed herein. In some aspects, modifying comprises sequential homologous recombination in a single ES cell.

In some embodiments, the method utilizes a targeting construct made using VELOCIGENE^{®} technology, introducing the construct into ES cells, and introducing targeted ES cell clones into a mouse embryo using VELOCIMOUSE^{®} technology (see, e.g., U.S. Pat. No. 7,294,754 and Poueymirou et al. (2007) Nature Biotech 25:91-99). Targeting construct may comprise 5' and/or 3' homology arms that target the endogenous sequence to be replaced, an insert sequence (that replaces the endogenous sequence) and one or more selection cassettes. A selection cassette is a nucleotide sequence inserted into a targeting construct to facilitate selection of cells (e.g., ES cells) that have integrated the construct of interest. A number of suitable selection cassettes are known in the art. Commonly, a selection cassette enables positive selection in the presence of a particular antibiotic (e.g., Neo, Hyg, Pur, CM, SPEC, etc.). In addition, a selection cassette may be flanked by recombination sites, which allow deletion of the selection cassette upon treatment with recombinase enzymes. Commonly used recombination sites are *lox*P and *Frt*, recognized by Cre and Flp enzymes, respectively, but others are known in the art. A selection cassette may be located anywhere in the construct outside the coding region. In one embodiment, the selection cassette is located at the 5' end the human DNA fragment. In another embodiment, the selection cassette is located at the 3' end of the human DNA fragment. In another embodiment, the selection cassette is located within the human DNA fragment. In another embodiment, the selection cassette is located within an intron of the human DNA fragment. In another embodiment, the selection cassette is located at the junction of the human and mouse DNA fragment. F0 generation mice that are essentially fully derived from the donor gene-targeted ES cells allowing immediate phenotypic analyses. VELOCIMICE^{®} (F0 mice fully derived from the donor ES cell) independently bearing a human or humanized MHC class I gene, human or humanized β2 microglobulin gene, and/or a human or humanized MHC class II gene, as well as humanized immunoglobulin loci are identified by genotyping using a modification of allele assay (Valenzuela et al. (2003) High-throughput engineering of the mouse genome coupled with high-resolution expression analysis, Nature Biotech. 21(6):652-659) that detects the presence of these unique gene sequences. Heterozygous mice generated by this method may be bred to homozygosity.

In some aspects described herein, a non-human animal comprising a human or humanized MHC I, β2 microglobulin, MHC II molecules is bred with a second non-human animal of the same species, wherein the second non-human animal comprises an unrearranged human or humanized immunoglobulin heavy chain locus and/or an unrearranged human or humanized immunoglobulin light chain locus. For example, a mouse comprising a human or humanized MHC I, β2 microglobulin, MHC II molecules may be bred to a second mouse comprising
(a) at an endogenous heavy chain locus:
   (i) an unrearranged human(ized) immunoglobulin heavy chain variable region in operable linkage to an endogenous heavy chain constant region;
   (ii) a restricted unrearranged human(ized) heavy chain variable region in operable linkage to an endogenous heavy chain constant region;
   (iii) a common heavy chain encoding sequence;
   (iv) a histidine modified unrearranged human(ized) heavy chain variable region in operable linkage to an endogenous heavy chain constant region;
   (v) a heavy chain only immunoglobulin encoding sequence; or
   (vi) an unrearranged human(ized) hybrid heavy chain sequence encoding a hybrid immunoglobulin chain;
   and/or
(b) at an endogenous light chain locus:
   (i) an unrearranged human(ized) immunoglobulin light chain variable region in operable linkage to an endogenous light chain constant region;
   (ii) a common light chain encoding sequence;
   (iii) a restricted unrearranged human(ized) light chain variable region in operable linkage to an endogenous light chain constant region;
   (iv) a histidine modified unrearranged human(ized) light chain variable region in operable linkage to an endogenous light chain constant region; or
   (v) a histidine modified rearranged human(ized) light chain variable region in operable linkage to an endogenous light chain constant region,
optionally wherein the second mouse further comprises
(i) a human(ized) immunoglobulin heavy chain locus comprising a functional ADAM6 gene such that the mouse exhibits wildtype fertility; and/or
(ii) an exogenous terminal deoxynucleotidyl transferase (TdT) gene for increased antigen receptor diversity such that, optionally, at least 10% of the rearranged variable region genes comprise non-template additions.

In another aspect, constructs inserting human or humanized MHC I, MHC II, and/or β2 microglobulin participate in homologous recombination (at an endogenous MHC I, MHC II, and/or β2 microglobulin locus or at an ectopic locus) in a non-human animal ES cell genetically modified to comprise an unrearranged human or humanized immunoglobulin heavy chain locus and/or an unrearranged human or humanized immunoglobulin light chain locus. Alternatively, constructs for inserting comprise an unrearranged human or humanized immunoglobulin heavy chain locus and/or an unrearranged human or humanized immunoglobulin light chain locus participate in homologous recombination in a non-human animal ES cell genetically modified to comprise nucleotide sequences encoding human or humanized MHC I, MHC II, and/or β2 microglobulin. In one aspect, constructs for targeting and replacing endogenous MHC I, MHC II, and/or β2 microglobulin sequences with nucleic acid sequences encoding chimeric human/mouse MHC I, MHC II, and/or β2 microglobulin; or constructs for inserting a single chain MHC I/β2 microglobulin and/or a single chain MHC II protein participate in homologous recombination in an ES cell, which may also comprise
(a) at an endogenous heavy chain locus:
   (i) an unrearranged human(ized) immunoglobulin heavy chain variable region in operable linkage to an endogenous heavy chain constant region;
   (ii) a restricted unrearranged human(ized) heavy chain variable region in operable linkage to an endogenous heavy chain constant region;
   (iii) a common heavy chain encoding sequence;
   (iv) a histidine modified unrearranged human(ized) heavy chain variable region in operable linkage to an endogenous heavy chain constant region;
   (v) a heavy chain only immunoglobulin encoding sequence; or
   (vi) an unrearranged human(ized) hybrid heavy chain sequence encoding a hybrid immunoglobulin chain;
   and/or
(b) at an endogenous light chain locus:
   (i) an unrearranged human(ized) immunoglobulin light chain variable region in operable linkage to an endogenous light chain constant region;
   (ii) a common light chain encoding sequence;
   (iii) a restricted unrearranged human(ized) light chain variable region in operable linkage to an endogenous light chain constant region;
   (iv) a histidine modified unrearranged human(ized) light chain variable region in operable linkage to an endogenous light chain constant region; or
   (v) a histidine modified rearranged human(ized) light chain variable region in operable linkage to an endogenous light chain constant region,
optionally wherein the ES cell further comprises
(i) a human(ized) immunoglobulin heavy chain locus comprising a functional ADAM6 gene; and/or
(ii) an exogenous terminal deoxynucleotidyl transferase (TdT) gene for increased antigen receptor diversity such that.

In various aspects of the disclosure, the sequence(s) encoding a chimeric human/non-human MHC I and MHC II polypeptides are located at an endogenous non-human MHC locus (e.g., mouse H-2K and/or H-2E locus). In one embodiment, this results in placement, e.g., replacement, of an endogenous MHC gene(s) or a portion thereof with a nucleic acid sequence(s) encoding a human or humanized MHC I polypeptides. Since the nucleic acid sequences encoding MHC I, MHC II α and MHC II β polypeptides are located in proximity to one another on the chromosome, in order to achieve the greatest success in humanization of both MHC I and MHC II in one animal, if desired, the MHC I and MHC II loci should be targeted sequentially. Thus, also described herein are methods of generating a genetically modified non-human animal comprising nucleic acid sequences encoding chimeric human/non-human MHC I, MHC II α and MHC II β polypeptides.

Thus, in some aspects, a nucleotide construct for generating genetically modified animals that comprises chimeric human/non-human MHC is described. In one aspect, the nucleic acid construct comprises: 5' and 3' non-human homology arms, a human DNA fragment comprising human MHC gene sequences (e.g., human HLA-A2 or human HLA-DRs gene sequences), and a selection cassette flanked by recombination sites. In one aspect, the human DNA fragment is a genomic fragment that comprises both introns and exons of a human MHC gene (e.g., human HLA-A2 or HLA-DR2 gene). In one aspect, the non-human homology arms are homologous to a non-human MHC locus (e.g., MHC I or MHC II locus).

In one aspect, the 5' and 3' non-human homology arms comprise genomic sequence at 5' and 3' locations, respectively, of an endogenous non-human (e.g., murine) MHC class I or class II gene locus (e.g., 5' of the first leader sequence and 3' of the α3 exon of the mouse MHC I gene, or upstream of mouse H-2Ab 1 gene and downstream of mouse H-2Ea gene). In one aspect, the endogenous MHC class I locus is selected from mouse H-2K, H-2D and H-2L. In a specific aspect, the endogenous MHC class I locus is mouse H-2K. In one aspect, the endogenous MHC II locus is selected from mouse H-2E and H-2A. In one aspect, the engineered MHC II construct allows replacement of both mouse H-2E and H-2A genes. In one aspect, the mouse does not express functional endogenous MHC polypeptides from its H-2D locus. In some aspects, the mouse is engineered to lack all or a portion of an endogenous H-2D locus. In another aspect, the mouse does not express any functional endogenous MHC I and MHC II polypeptides on a cell surface. In one aspect, the only MHC I and MHC II expressed by the mouse on a cell surface are chimeric human/mouse MHC I and MHC II.

The disclosure also relates to methods for making a genetically engineered non-human animal (e.g., a genetically engineered rodent, e.g., a mouse or a rat) whose genome comprises a β2 microglobulin locus encoding a human or humanized β2 microglobulin polypeptide. In one aspect, the methods result in a genetically engineered rodent, e.g., mouse, whose genome comprises at an endogenous β2 microglobulin locus a nucleotide sequence encoding a human or humanized β2 microglobulin polypeptide. In some instances, the mouse does not express a functional mouse β2 microglobulin from an endogenous mouse β2 microglobulin locus.

Also described is a nucleotide construct used for generating genetically engineered non-human animals. In some aspects, the nucleotide construct may comprise: 5' and 3' non-human homology arms, a human DNA fragment comprising human β2 microglobulin sequences, and a selection cassette flanked by recombination sites. In one aspect, the human DNA fragment is a genomic fragment that comprises both introns and exons of a human β2 microglobulin gene. In one aspect, the non-human homology arms are homologous to a non-human β2 microglobulin locus. The genomic fragment may comprise exons 2, 3, and 4 of the human β2 microglobulin gene. In one instance, the genomic fragment comprises, from 5' to 3': exon 2, intron, exon 3, intron, and exon 4, all of human β2 microglobulin sequence. The selection cassette may be located anywhere in the construct outside the β2 microglobulin coding region, e.g., it may be located 3' of exon 4 of the human β2 microglobulin. The 5' and 3' non-human homology arms may comprise genomic sequence 5' and 3' of endogenous non-human β2 microglobulin gene, respectively. In another aspect, the 5' and 3' non-human homology arms comprise genomic sequence 5' of exon 2 and 3' of exon 4 of endogenous non-human gene, respectively.

Another aspect of the disclosure relates to a method of modifying a β2 microglobulin locus of a non-human animal (e.g., a rodent, e.g., a mouse or a rat) to express a human or humanized β2 microglobulin polypeptide. One method of modifying a β2 microglobulin locus of a non-human animal, e.g., mouse, to express a human or humanized β2 microglobulin polypeptide, comprises replacing at an endogenous β2 microglobulin locus a nucleotide sequence encoding a mouse β2 microglobulin with a nucleotide sequence encoding the human or humanized β2 microglobulin polypeptide. In one aspect of such method, the non-human animal, e.g., mouse does not express a functional β2 microglobulin polypeptide from an endogenous non-human, e.g., mouse β2 microglobulin locus. In some specific aspects, the nucleotide sequence encoding the human or humanized β2 microglobulin polypeptide comprises nucleotide sequence set forth in exons 2 to 4 of the human β2 microglobulin gene. In other aspects, the nucleotide sequence encoding the human or humanized β2 microglobulin polypeptide comprises nucleotide sequences set forth in exons 2, 3, and 4 of the human β2 microglobulin gene.

The disclosure also relates to methods for making a genetically engineered non-human animal (e.g., a genetically engineered rodent, e.g., a mouse or a rat) whose genome comprises at an ectopic locus, e.g., a ROSA26 locus, a sequence encoding a single chain β2 microglobulin/MHC complex comprising a human(ized) β2 microglobulin and a human(ized) MHC class I α polypeptide, a human(ized) MHC class I α polypeptide and/or a human(ized) β2 microglobulin. In one aspect, the methods result in a genetically engineered rodent, e.g., a rat or a mouse, whose genome comprises at an endogenous locus that is not an endogenous MHC I or β2 microglobulin locus, e.g., an endogenous ROSA26 locus, a nucleotide sequence encoding a single chain β2 microglobulin/MHC complex comprising a human(ized) β2 microglobulin and a human(ized) MHC complex, a human(ized) MHC class I α polypeptide and/or a human(ized) β2 microglobulin. Methods for targeting the ROSA locus are well-known in the art. *See, e.g.*, Stefano Casola, Mouse Models for miRNA expression: the ROSA26 Locus, in Methods in Molecular Biology vol. 667:145-163 (S. Monticelli ed. 2010.

Also described is a nucleotide construct used for generating genetically engineered non-human animals. In some aspects, the nucleotide construct may comprise: 5' and 3' non-human homology arms, a nucleotide sequence encoding a single chain β2 microglobulin/MHC complex comprising a human(ized) β2 microglobulin and a human(ized) MHC class I α polypeptide (e.g., a single chain β2 microglobulin/HLA-A2 complex as set forth in SEQ ID NO:23), and a selection cassette flanked by recombination sites. In some aspects, the nucleotide construct may comprise: 5' and 3' non-human homology arms, a nucleotide sequence encoding a human(ized) MHC class I α polypeptide, and a selection cassette flanked by recombination sites. In some aspects, the nucleotide construct may comprise: 5' and 3' non-human homology arms, a nucleotide sequence encoding a human(ized) β2 microglobulin, and a selection cassette flanked by recombination sites. The 5' and 3' non-human homology arms may comprise genomic sequence flanking an endogenous ROSA26 intron.

In some aspects, the genetically modified non-human animal (e.g., mouse) may comprise one or two copies of the genes encoding human or humanized MHC I; human or humanized β2 microglobulin; human or humanized MHC II (e.g., MHC IIα and/or MHC IIβ); and human or humanized immunoglobulin heavy and light chains. Accordingly, in some aspects, the non-human animal may be heterozygous or homozygous for any or all of these genes. Since a non-limiting purpose of modifying the non-human animal to comprise a human or human MHC I, human or humanized β2 microglobulin; human or humanized MHC II (e.g., MHC IIα and/or MHC IIβ) is to tolerize the non-human animal to the human or humanized MHC molecule, homozygosity of these gene is not required. Accordingly, In some aspects, a non-human animal may be heterozygous for the nucleotide sequence encoding a human or humanized MHC molecule. In contrast, since a non-limiting purpose of modifying immunoglobulin loci of the non-human animal is for the generation of human or humanized antibodies against an antigenic pMHC complex of interest, in some aspects a non-human animal may be homozygous for the modified immunoglobulin locus.

Upon completion of gene targeting, ES cells or genetically modified non-human animals are screened to confirm successful incorporation of exogenous nucleotide sequence of interest or expression of exogenous polypeptide. Numerous techniques are known to those skilled in the art, and include (but are not limited to) Southern blotting, long PCR, quantitative PCR (e.g., real-time PCR using TAQMAN^{®}), fluorescence *in situ* hybridization, Northern blotting, flow cytometry, Western analysis, immunocytochemistry, immunohistochemistry, etc. In one example, non-human animals (e.g., mice) bearing the genetic modification of interest can be identified by screening for loss of mouse allele and/or gain of human allele using a modification of allele assay described in Valenzuela et al. (2003), *supra.* Other assays that identify a specific nucleotide or amino acid sequence in the genetically modified animals are known to those skilled in the art.

### Antigenic peptide-MHC complexes of interest

Mice tolerized for MHC protein described herein are immunized with antigenic peptide-MHC complex to generate pMHC-specific antigen-binding proteins. In some embodiments, MHCs useful as part of an antigenic peptide-MHC (pMHC) complex include naturally occurring full-length MHCs as well as individual chains of MHCs (e.g., MHC class I α (heavy) chain, β2 microglobulin, MHC class II α chain, and MHC class II β chain), individual subunits of such chains of MHCs (e.g., α1-α3 subunits of MHC class I α chain, α1-α2 subunits of MHC class II α chain, β1- β2 subunits of MHC class II β chain) as well as fragments, mutants and various derivatives thereof (including fusion proteins), wherein such fragments, mutants and derivatives retain the ability to display an antigenic determinant for recognition by an antigen-specific TCR.

Naturally-occurring MHCs are encoded by a cluster of genes on human chromosome 6. MHCs include, but are not limited to, HLA specificities such as A (e.g. A1-A74), B (e.g., B 1-B77), C (e.g., C1-C11), D (e.g., D1-D26), DR (e.g., DR1-DR8), DQ (e.g., DQ1-DQ9) and DP (e.g. DP1-DP6). HLA specificities include A1, A2, A3, A11, A23, A24, A28, A30, A33, B7, B8, B35, B44, B53, B60, B62, DR1, DR2, DR3, DR4, DR7, DR8, and DR 11.

Naturally occurring MHC class I molecules bind peptides derived from proteolytically degraded proteins especially endogenously synthesized proteins, by a cell. Small peptides obtained accordingly are transported into the endoplasmic reticulum where they associate with nascent MHC class I molecules before being routed through the Golgi apparatus and displayed on the cell surface for recognition by cytotoxic T lymphocytes.

Naturally occurring MHC class I molecules consist of an α (heavy) chain associated with β2 microglobulin. The heavy chain consists of subunits α1-α3. The β2 microglobulin protein and α3 subunit of the heavy chain are associated. In certain embodiments, β2 microglobulin and α3 subunit are associated by covalent binding. In certain embodiments, β2 microglobulin and α3 subunit are associated non-covalently. The α1 and α2 subunits of the heavy chain fold to form a groove for a peptide, e.g., antigenic determinant, to be displayed and recognized by TCR.

Class I molecules generally associate with, e.g., bind, peptides of about 8-9 amino acids (e.g., 7 -11 amino acids) in length. All humans have between three and six different class I molecules, which can each bind many different types of peptides.

In some aspects, a pMHC complex comprises (i) a class I MHC polypeptide or a fragment, mutant or derivative thereof, and optionally, (ii) a β2 microglobulin polypeptide or a fragment, mutant or derivative thereof. In one specific aspect, the class I MHC polypeptide is associated, e.g., linked, to the β2 microglobulin polypeptide by a peptide linker.

In one specific embodiment, the class I MHC polypeptide is a human class I MHC polypeptide selected from the group consisting of HLA-A, HLA-B, HLA-C, HLA-E, HLA-F, and HLA-G. In another specific embodiment, the class I MHC polypeptide is a murine class I MHC polypeptide selected from the group consisting of H-2K, H-2D, H-2L, H2-IA, H2-IB, H2-IJ, H2-IE, and H2-IC.

In some embodiments, the MHC class I α heavy chain of an antigenic pMHC complex is fully human. In some aspects, the MHC class I α heavy chain of antigenic pMHC complex is humanized. Humanized MHC class I α heavy chains are described, e.g., in U.S. Pat. Pub. Nos. 2013/0111617, 2013/0185819 and 2014/0245467. In some embodiments, the MHC class I α heavy chain comprises a human extracellular domain (human α1, α2, and/or α3 domains) and a cytoplasmic domain of another species. In some embodiments, the class I α heavy chain polypeptide is HLA-A, HLA-B, HLA-C, HLA-E, HLA-F, HLA-G, HLA-K, or HLA-L. In some embodiments, the HLA-A sequence can be an HLA-A*0201 sequence. In various embodiments, the peptide-MHC can include all the domains of an MHC class I heavy chain.

In some embodiments, the antigenic pMHC complex comprises a β2 microglobulin. In some embodiments, the β2 microglobulin is fully human. In some embodiments, the β2 microglobulin is humanized. Humanized β2 microglobulin polypeptides are described, e.g., in U.S. Pat. Pub. Nos. 2013/0111617 and 2013/0185819.

In some embodiments, the MHC class I molecule of an antigenic pMHC complex comprises a mutation in a human(ized) β2 microglobulin (β2m or B2M) polypeptide and in the human(ized) MHC class I α heavy chain such that a disulfide bond may form between the human(ized) B2M and the human(ized) MHC class I α heavy chain. In some embodiments, the disulfide bond links one of the following pairs of residues: human(ized) B2M residue 12, human(ized) MHC class I α heavy chain residue 236; human(ized) B2M residue 12, human(ized) MHC class I α heavy chain residue 237; human(ized) B2M residue 8, human(ized) MHC class I α heavy chain residue 234; human(ized) B2M residue 10, human(ized) MHC class I α heavy chain residue 235; human(ized) B2M residue 24, human(ized) MHC class I α heavy chain residue 236; human(ized) B2M residue 28, human(ized) MHC class I α heavy chain residue 232; human(ized) B2M residue 98, human(ized) MHC class I α heavy chain residue 192; human(ized) B2M residue 99, human(ized) MHC class I α heavy chain residue 234; human(ized) B2M residue 3, human(ized) MHC class I α heavy chain residue 120; human(ized) B2M residue 31, human(ized) MHC class I α heavy chain residue 96; human(ized) B2M residue 53, human(ized) MHC class I α heavy chain residue 35; human(ized) B2M residue 60, human(ized) MHC class I α heavy chain residue 96; human(ized) B2M residue 60, human(ized) MHC class I α heavy chain residue 122; human(ized) B2M residue 63, human(ized) MHC class I α heavy chain residue 27; human(ized) B2M residue Arg3, human(ized) MHC class I α heavy chain residue Gly 120; human(ized) B2M residue His31, human(ized) MHC class I α heavy chain residue Gln96; human(ized) B2M residue Asp53, human(ized) MHC class I α heavy chain residue Arg35; human(ized) B2M residue Trp60, human(ized) MHC class I α heavy chain residue Gln96; human(ized) B2M residue Trp60, human(ized) MHC class I α heavy chain residue Asp 122; human(ized) B2M residue Tyr63, human(ized) MHC class I α heavy chain residue Tyr27; human(ized) B2M residue Lys6, human(ized) MHC class I α heavy chain residue Glu232; human(ized) B2M residue Gln8, human(ized) MHC class I α heavy chain residue Arg234; human(ized) B2M residue Tyr 10, human(ized) MHC class I α heavy chain residue Pro235; human(ized) B2M residue Serl 1, human(ized) MHC class I α heavy chain residue Gln242; human(ized) B2M residue Asn24, human(ized) MHC class I α heavy chain residue Ala236; human(ized) B2M residue Ser28, human(ized) MHC class I α heavy chain residue Glu232; human(ized) B2M residue Asp98, human(ized) MHC class I α heavy chain residue His 192; human(ized) B2M residue Met99, human(ized) MHC class I α heavy chain residue Arg234, and/or human(ized) B2M residue Arg 12, human(ized) MHC class I α heavy chain residue Gly237. *See, e.g.,* Int. Pat. Appl. Pub. WO/2015195531.

In some embodiments, the antigenic determinant amino acid sequence can be that of a peptide which can be associated with, e.g., presented by, an MHC class I molecule. In certain embodiments, the sequence can comprise from 6 to 20 contiguous amino acids. In certain embodiments, a peptide sequence can be that of a protein fragment, wherein the protein is a derived from, e.g., a portion of, a cellular protein, such as, for example, a protein associated with an autoimmune disorder, and wherein the peptide can be bound to the MHC class I heavy chain.

In some embodiments, at least one chain of the MHC and the peptide are associated as a fusion protein. In one embodiment, the MHC and the peptide are associated by a linker sequence. For example, the single chain molecule can comprise, from amino to carboxy terminal an antigenic determinant, a β2 microglobulin sequence, and a class I α (heavy) chain sequence. Alternatively, the single chain molecule can comprise, from amino to carboxy terminal an antigenic determinant, a class I α (heavy) chain sequence, and a β2 microglobulin sequence. The generation and use of pMHC complexes as single chain trimers has previously been described. *See, e.g.* U.S. Patent No. 8,895,020; U.S. Patent No 8,992,937; Hansen et al. (2010) Trends Immunol. 31:363-69; Truscott et al. (2007) J. Immunol. 178:6280-89; Mitaksov et al. (2007) Chem Biol 14:909-22. The single-chain pMHC complex can further comprise a signal peptide sequence at the amino terminal. In certain embodiments, there can be a linker sequence between the peptide sequence and the β2 microglobulin sequence. In certain embodiments, there can be a linker sequence between the β2 microglobulin sequence and the class I α (heavy) chain sequence. A single-chain molecule can further comprise a signal peptide sequence at the amino terminal, as well as first linker sequence extending between the peptide sequence and the β2 microglobulin sequence, and/or a second linker sequence extending between the β2 microglobulin sequence and the class I heavy chain sequence.

In some embodiments, a single-chain pMHC complex can comprise a first flexible linker between the peptide ligand segment and the β2 microglobulin segment. For example, linkers can extend from and connect the carboxy terminal of the peptide ligand segment to the amino terminal of the β2 microglobulin segment. In some embodiments, the linkers are structured to allow the linked peptide ligand to fold into the binding groove resulting in a functional MHC-antigen peptide. In some embodiments, this linker can comprise at least 3 amino acids, up to about 15 amino acids (e.g., 20 amino acids). In some embodiments, a single-chain molecule can comprise a second flexible linker inserted between the β2 microglobulin and MHC I heavy chain segment. For example, linkers can extend from and connect the carboxy terminal of the β2 microglobulin segment to the amino terminal of the MHC I heavy chain segment. In certain embodiments, the β2 microglobulin and the MHC I heavy chain can fold into the binding groove resulting in a molecule which can function in promoting T cell expansion.

Suitable linkers used in the pMHC complexes can be of any of a number of suitable lengths, such as from 1 amino acid (e.g., Gly) to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids, and can be 1, 2, 3, 4, 5, 6, or 7 amino acids. Exemplary linkers include glycine polymers (G)n, glycine-serine polymers (including, for example, (GS)n, (GSGGS) (SEQ ID NO:1) and (GGGS) (SEQ ID NO:2), where n is an integer of at least one), glycine-alanine polymers, alanine-serine polymers, and other flexible linkers known in the art. Glycine and glycine-serine polymers can be used; both Gly and Ser are relatively unstructured, and therefore can serve as a neutral tether between components. Glycine polymers can be used; glycine accesses significantly more phi-psi space than even alanine, and is much less restricted than residues with longer side chains (see Scheraga, Rev. Computational Chem. 1 1173-142 (1992)). Exemplary linkers can comprise amino acid sequences including, but not limited to, GGSG (SEQ ID NO:3), GGSGG (SEQ ID NO:4), GSGSG (SEQ ID NO:5), GSGGG (SEQ ID NO:6), GGGSG (SEQ ID NO:7), GSSSG (SEQ ID NO:8), GCGASGGGGSGGGGS (SEQ ID NO:9), GCGASGGGGSGGGGS (SEQ ID NO:10), GGGGSGGGGS (SEQ ID NO:11), GGGASGGGGSGGGGS (SEQ ID NO:12), GGGGSGGGGSGGGGS (SEQ ID NO:13), or GGGASGGGGS (SEQ ID NO:14), GGGGSGGGGSGGGGS (SEQ ID NO:15), GGGGSGGGGSGGGGSGGGGS (SEQ ID NO:16), GCGGS (SEQ ID NO:21) and the like. In some embodiments, a linker polypeptide includes a cysteine residue that can form a disulfide bond with a cysteine residue present in a second polypeptide.

In certain embodiments, the single-chain pMHC complex can comprise a peptide covalently attached to an MHC class I α (heavy) chain via a disulfide bridge (*i.e.*, a disulfide bond between two cystines). See, e.g., US Patent Nos. 8,992,937 and 8,895,020. In certain embodiments, the disulfide bond comprises a first cysteine, that is positioned within a linker extending from the carboxy terminal of an antigen peptide, and a second cysteine that is positioned within an MHC class I heavy (e.g., an MHC class I α (heavy) chain which has a noncovalent binding site for the antigen peptide). In certain embodiments, the second cysteine can be a mutation (addition or substitution) in the MHC class I α (heavy) chain. In certain embodiments, the single-chain molecule can comprise one contiguous polypeptide chain as well as a disulfide bridge. In certain embodiments, the single-chain molecule can comprise two contiguous polypeptide chains which are attached via the disulfide bridge as the only covalent linkage. In some embodiments, the linking sequences can comprise at least one amino acid in addition to the cysteine, including one or more glycines, one or more, alanines, and/or one or more serines. In some embodiments, the single-chain molecule comprises from N-terminus to C-terminus an MHC class I peptide (e.g., an antigenic peptide), a first linker that comprises a first cysteine, a human(ized) β2-microglobulin sequence, a second linker, and a human(ized) MHC class I heavy chain sequence comprising a second cysteine, wherein the first cysteine and the second cysteine comprise a disulfide bridge. In some embodiments, the second cysteine is a substitution of an amino acid of the human(ized) MHC class I heavy chain selected from the group consisting of T80C, Y84C and N86C (Y84C refers to a mutation at position 108 in a mature protein, where the mature protein lacks a signal sequence. Alternatively, when the protein still includes a 24 mer signal sequence, the position is instead referred to as Y108C).

In certain embodiments, the disulfide bridge can link an antigen peptide in the class I groove of the pMHC complex if the pMHC complex comprises a first cysteine in a Gly-Ser linker extending between the C-terminus of the peptide and the β2 microglobulin, and a second cysteine in a proximal heavy chain position.

In some embodiments, the β2 microglobulin sequence can comprise a full-length (human or non-human) β2 microglobulin sequence. In certain embodiments, the β2 microglobulin sequence lacks the leader peptide sequence. As such, the β2 microglobulin sequence can comprise about 99 amino acids, and can be a human β2 microglobulin sequence (Genebank AF072097.1).

In some embodiments, the pMHC complex comprises a human HLA class I molecule fused with a human β2 microglobulin. In some embodiments, the human HLA class I molecule fused with a human β2 microglobulin comprises one or more linkers, e.g., a linker linking the HLA molecule and the β2 microglobulin and/or a linker linking the peptide to the human HLA class I molecule fused with a human β2 microglobulin. In some embodiments, the nucleotide sequence that encodes a pMHC complex may comprise a sequence encoding the human HLA and β2 microglobulin. In some embodiments, the nucleotide sequence that encodes a pMHC complex may comprise a sequence encoding the human HLA and β2 microglobulin and one or more linkers. In some embodiments, the nucleotide sequence that encodes a pMHC complex may comprise a sequence that encodes the human HLA and β2 microglobulin and a sequence encoding a label (e.g., green fluorescence protein) or tag (e.g., c-myc, a histidine tag, etc.). In some embodiments, the nucleotide sequence that encodes a pMHC complex may comprises a sequence that encodes the human HLA and β2 microglobulin, a sequence encoding a linker, and a sequence encoding a label or tag. Non-limiting examples of a nucleotide sequence encoding an exemplary a human HLA and β2 microglobulin and one or more linkers are set forth as SEQ ID NO:17 and SEQ ID NO:19. The amino acid sequences encoded therefrom are respectively set forth as SEQ ID NO: 18 and SEQ ID NO:20.

A peptide of interest may be attached to the N-terminal GCGGS linker sequence (SEQ ID NO:21) of SEQ ID NO:18 and SEQ ID NO:20, wherein the cysteine of the linker forms a disulfide bridge with the Y108C amino acid of the human HLA-A2 polypeptide. Accordingly, in some embodiments, a non-human animal is immunized and/or boosted with a pMHC complex comprising an amino acid sequence comprising the sequence set forth as SEQ ID NO:18 or the sequence set forth as SEQ ID NO:20. In some embodiments, a non- human animal is immunized with DNA encoding a pMHC complex having an amino acid sequence comprising the sequence set forth as SEQ ID NO: 18 or SEQ ID NO:20.

In some embodiments, a helper T cell epitope, e.g., PADRE may be linked to C-terminus of the single-chain pMHC complex. *See, e.g.,* US Patent No. 6,413,935 and Alexander J. et al. (1994) Immunity 1:751-61. In some embodiments, PADRE is directly linked to the C-terminus of the single-chain pMHC complex. In embodiments, PADRE is linked to the C-terminus of the single-chain pMHC complex via a linker. In some embodiments, an immunization protocol described herein comprises administering to a non-human animal a single-chain pMHC complex linked at the C-terminus to PADRE. In some embodiments, a single-chain pMHC complex linked at the C-terminus to PADRE comprises an amino acid sequence set forth as SEQ ID NO:25.

In some embodiments, the pMHC complex can be that as disclosed in U.S. Patent Nos. 4,478,82; 6,011,146; 8,895,020; 8,992,937; WO 96/04314; Mottez et al. J. Exp. Med. 181: 493-502, 1995; Madden et al. Cell 70: 1035-1048, 1992; Matsumura et al., Science 257: 927-934, 1992; Mage et al., Proc. Natl. Acad. Sci. USA 89: 10658-10662, 1992; Toshitani et al, Proc. Nat'l Acad. Sci. 93: 236-240, 1996; Chung et al, J. Immunol. 163:3699-3708, 1999; Uger and Barber, J. Immunol. 160: 1598-1605, 1998; Uger et al., J. Immunol. 162, pp. 6024-6028, 1999; White et al., J. Immunol. 162: 2671-2676, 1999.

In some aspects, the pMHC complex comprises a class II MHC polypeptide or a fragment, mutant or derivative thereof. In one specific aspect, the MHC comprises α and β polypeptides of a class II MHC molecule or a fragment, mutant or derivative thereof. In one specific embodiment, the α and β polypeptides are linked by a peptide linker. In one specific embodiment, the MHC comprises α and β polypeptides of a human class II MHC molecule selected from the group consisting of HLA-DP, HLA-DR, HLA-DQ, HLA-DM and HLA-DO.

MHC class II molecules generally consist of two polypeptide chains, α and β. The chains may come from the DP, DQ, or DR gene groups. There are about 40 known different human MHC class II molecules. All have the same basic structure but vary subtly in their molecular structure. MHC class II molecules bind peptides of 13-18 amino acids in length.

In some embodiments, an antigenic pMHC complex comprises one or more MHC class II α chains. In some embodiments, the MHC class II α chain is fully human. In some aspects, the MHC class II α chain is humanized. Humanized MHC class II α chains are described, e.g., in U.S. Pat. Nos. 8,847,005 and 9,043,996 and U.S. Pat. Pub. No. 2014/0245467. In some embodiments, the humanized MHC class II α chain polypeptide comprises a human extracellular domain and a cytoplasmic domain of another species. In some embodiments, the class II α chain is HLA-DMA, HLA-DOA, HLA-DPA, HLA-DQA or HLA-DRA. In some embodiments, the class II α chain polypeptide is humanized HLA-DMA, HLA-DOA, HLA-DPA, HLA-DQA and/or HLA-DRA.

In some aspects, the viral particle comprises one or more MHC class II β chains. In some aspects, the MHC class II β chain is fully human. In some aspects, the MHC class II β chain polypeptide is humanized. Humanized MHC class II β chain polypeptides are described, e.g., in U.S. Pat. Nos. 8,847,005 and 9,043,996 and U.S. Pat. Pub. No. 2014/0245467. In some aspects, the humanized MHC class II β chain comprises a human extracellular domain and a cytoplasmic domain of another species. In some aspects, the class II β chain is HLA-DMB, HLA-DOB, HLA-DPB, HLA-DQB or HLA-DRB. In some aspects, the class II β chain is humanized HLA-DMB, HLA-DOB, HLA-DPB, HLA-DQB and/or HLA-DRB.

In some embodiments, an antigenic determinant, e.g., the peptide, comprised in an antigenic pMHC complex can comprise any peptide that is capable of binding to an MHC protein in a manner such that the pMHC complex can bind to a TCR, e.g., in a specific manner.

Examples include peptides produced by hydrolysis and most typically, synthetically produced peptides, including randomly generated peptides, specifically designed peptides, and peptides where at least some of the amino acid positions are conserved among several peptides and the remaining positions are random.

In nature, peptides that are produced by hydrolysis undergo hydrolysis prior to binding of the antigen to an MHC protein. Class I MHC typically present peptides derived from proteins actively synthesized in the cytoplasm of the cell. In contrast, class II MHC typically present peptides derived either from exogenous proteins that enter a cell's endocytic pathway or from proteins synthesized in the ER. Intracellular trafficking permits a peptide to become associated with an MHC protein.

The binding of a peptide to an MHC peptide binding groove can control the spatial arrangement of MHC and/or peptide amino acid residues recognized by a TCR, or pMHC-binding protein produced by an animal genetically modified as disclosed herein. Such spatial control is due in part to hydrogen bonds formed between a peptide and an MHC protein. Based on the knowledge on how peptides bind to various MHCs, the major MHC anchor amino acids and the surface exposed amino acids that are varied among different peptides can be determined. In some embodiments, the length of an MHC-binding peptide is from 5 to 40 amino acid residues, e.g., from 6 to 30 amino acid residues, e.g., from 8 to 20 amino acid residues, e.g., between 9 and 11 amino acid residues, including any size peptide between 5 and 40 amino acids in length, in whole integer increments (i.e., 5, 6, 7, 8, 9 ... 40). While naturally MHC Class II-bound peptides vary from about 9-40 amino acids, in nearly all cases the peptide can be truncated to a 9-11 amino acid core without loss of MHC binding activity or T-cell recognition.

Peptides include peptides comprising at least a portion, e.g., an antigenic determinant, of a protein selected from a group consisting of a human self-protein associated with an autoimmune disorder, proteins of infectious agents (e.g., bacterial, viral or parasitic organisms), allergens, and tumor associated proteins. In one embodiment, a pMHC complex as comprises an antigenic determinant of a human self-protein associated with autoimmune disorders. In another embodiment, a pMHC complex a comprises an antigenic determinant of an allergen. In another embodiment, a pMHC complex comprises an antigenic determinant of a bacteria. In another embodiment, a pMHC complex as comprises an antigenic determinant of a virus. In another embodiment, a pMHC complex as comprises an antigenic determinant of a parasite.

Attaching the peptide to the MHC Class I or MHC Class II molecule via a flexible linker has the advantage of assuring that the peptide will occupy and stay associated with the MHC during biosynthesis, transport and display. As an alternate approach, in some embodiments, the MHC and the peptide are expressed separately.

### Antigen-Binding Proteins that specifically bind an antigenic pMHC complex of interest, Nucleic Acid Constructs, Cells and Methods of Making Same

In one aspect, described are a nucleic acid encoding a variable domain of an antigen-binding domain that specifically binds an antigenic pMHC complex, and a cell expressing the nucleic acid.

In one aspect, use of a nucleic acid sequence from a non-human animal to make a cell line for the manufacture of a human therapeutic is described. In one aspect, the human therapeutic is a binding protein comprising a human antigen-binding domain and human Fc region.

In one aspect, an expression system is described, comprising a mammalian host cell comprising a nucleic acid that encodes a polypeptide that comprises a somatically mutated human heavy chain variable domain fused with a human C_{H} region and/or a nucleic acid that encodes a polypeptide that comprises a somatically mutated human light chain variable domain fused with a human C_{L} region, wherein the V_{H} and V_{L} domains are cognate.

In one aspect, the suitable host cell is selected from a B cell, a hybridoma, a quadroma, a CHO cell, a COS cell, a 293 cell, a HeLa cell, and a human retinal cell expressing a viral nucleic acid sequence (*e.g.*, a PERC.6^{™} cell (Creative Biolabs)).

In one aspect, a method for making a binding protein is described, isolating a cell or nucleic acid from a non-human animal as disclosed herein, wherein the cell or nucleic acid comprises or encodes an antigen-binding protein that specifically binds a pMHC complex of interest. In some aspects, the method further comprises and cloning the nucleotide sequence encoding a human heavy or light chain variable region sequence (which may be encoding a histidine modified human heavy chain variable domain and/or a histidine modified human light chain variable domain, which may also or independently be a universal light chain variable domain) in frame with a gene encoding a human C_{H} or C_{L} region, region, to form a human binding protein sequence, and expressing the human binding protein sequence in a suitable cell.

In one aspect, the non-human animal has been immunized with a pMHC complex of interest, and the human antigen-binding domain specifically binds (e.g., with a K_{D} in the micromolar, nanomolar, or picomolar range) an epitope of the pMHC complex of interest. In one aspect, nucleotide sequence encoding the V_{H} and/or V_{L} domains are somatically mutated in the non-human animal.

In one aspect, a method for making an antigen-binding protein that binds a pMHC complex of interest is described, the method comprising
(1) immunizing a non-human animal with a pMHC complex of interest, wherein the non-human animal comprises in its genome
   (i) a nucleotide sequence encoding a human(ized) MHC molecule or at least a peptide binding portion thereof, and
   (ii) an (un)rearranged human or humanized immunoglobulin heavy locus and/or an (un)rearranged human or humanized immunoglobulin light chain locus, such that the non-human animal is capable of providing human or humanized antigen-binding proteins comprising a human or humanized antigen-binding domain, e.g., human or humanized variable domains,
   optionally wherein at least one of the (un)rearranged human or humanized immunoglobulin heavy locus and/or an (un)rearranged human or humanized immunoglobulin light chain locus is unrearranged;
(2) allowing the non-human animal to mount an immune response to the pMHC complex of interest or the pMHC complex of interest linked to the carrier;
(3) isolating a cell (e.g., a lymphocyte) from the immunized non-human animal, wherein the cell comprises first and second immunoglobulin variable region nucleic acid sequences that encode human heavy and light chain light chain variable domains (each of which may independently be histidine modified and the light chain variable domain of which may be a common light chain variable domain) that form an antigen-binding domain that specifically binds the pMHC complex of interest;
(4) identifying the immunoglobulin heavy and light chain variable region nucleic acid sequences that encode the immunoglobulin heavy and light chain variable domains that, when paired, specifically bind the pMHC complex of interest or the pMHC complex of interest linked to the carrier; and,
(5) expressing the nucleic acid sequences of (d) in an expression system suitable for expressing the antigen-binding protein so as to form an antigen-binding protein comprising a dimer of the heavy and light chain variable domains that bind the pMHC complex of interest.

In some aspects, a method for making an antigen-binding protein that binds a pMHC complex of interest is described, the method comprising
(1) immunizing a non-human animal with a pMHC complex of interest, wherein the non-human animal comprises in its genome
   (i) a nucleotide sequence encoding a human(ized) MHC molecule or at least a peptide binding portion thereof, and
   (ii) an (un)rearranged human or humanized immunoglobulin heavy locus and/or an (un)rearranged human or humanized immunoglobulin light chain locus, such that the non-human animal is capable of providing human or humanized antigen-binding proteins comprising a human or humanized antigen-binding domain, e.g., human or humanized variable domains,
   optionally wherein at least one of the (un)rearranged human or humanized immunoglobulin heavy locus and/or an (un)rearranged human or humanized immunoglobulin light chain locus is unrearranged;
(2) obtaining a human(ized) immunoglobulin heavy chain variable region sequence and/or a human(ized) immunoglobulin light chain variable region sequence that encodes a human(ized) immunoglobulin heavy chain variable domain and/or a human(ized) immunoglobulin light chain variable domain, respectively, of an antibody that specifically binds the pMHC of interest,
(c) employing the human(ized) immunoglobulin heavy chain variable region sequence and/or the human(ized) immunoglobulin light chain variable region sequence to produce an antibody that binds the pMHC.

In some aspects, cells (such as B cells) are recovered from the non-human animal (*e.g.*, from spleen or lymph nodes). The cells may be fused with a myeloma cell line to prepare immortal hybridoma cell lines, and such hybridoma cell lines are screened and selected to identify hybridoma cell lines that produce antibodies containing hybrid heavy chains specific to the antigen used for immunization.

In one aspect, immunization comprises priming (e.g., administering to) the non-human animal with the pMHC complex of interest, allowing the non-human animal to rest for a period of time, and re-immunizing (e.g., boosting the immune response of) the non-human animal with the pMHC complex of interest. In some aspects, the methods comprise immunizing and/or boosting the non-human animal concomitantly with a helper T cell epitope, e.g., a pan DR T helper epitope (PADRE). *See, e.g.,* US Patent No. 6,413,935 and Alexander J. et al. (1994) Immunity 1:751-61. In some aspects, the method comprises priming the non-human animal with the pMHC complex of interest and boosting the immunized animal with the pMHC complex of interest linked to a helper T cell epitope, e.g., PADRE. In some aspects, the method comprises both priming and boosting the non-human animal with the antigenic pMHC complex of interest linked to a helper T cell epitope. In aspects comprising priming and/or boosting with PADRE, the non-human animal is a mouse that comprises a C57/Bl6 genetic background, e.g., is a mouse of a C57BL strain selected from C57BL/A, C57BL/An, C57BL/GrFa, C57BL/KaLwN, C57BL/6, C57BL/6J, C57BL/6ByJ, C57BL/6NJ, C57BL/10, C57BL/10ScSn, C57BL/10C, and C57BL/Ola or is a mix of an aforementioned C57BL/6 strain and another strain, e.g., 129, BALB, etc. In some aspects, the period of time between priming the non-human animal and boosting the non-human animal is a few days, at least a week, at least two weeks, at least three weeks, at least four weeks, or at least one month.

In one aspect, an immunoglobulin variable region (VR) (e.g., comprising a rearranged human V_{H}/D_{H}/J_{H} gene sequence or a rearranged human V_{L}/J_{L} gene sequence, which may respectively and independently be a histidine-modified rearranged human V_{H}/D_{H}/J_{H} gene sequence or a rearranged human V_{L}/J_{L} gene sequence, and the latter of which may also or independently be a common rearranged human V_{L}/J_{L} gene sequence) made in a non-human animal is described. In one aspect, the rearranged V_{H}/D_{H}/J_{H} gene sequence is fused with one or more human heavy chain constant region sequences (e.g., selected from a human or mouse C_{H}1, hinge, C_{H}2, C_{H}3, and a combination thereof), or the rearranged V_{L}/J_{L} gene sequence is fused to a human light chain constant region sequence. Also described herein are immunoglobulin variable domain amino acid sequence of a binding protein made in a non-human animal of aspects of the disclosure and/or encoded by a nucleic acid sequence isolated therefrom.

In one aspect, a binding protein or antigen-binding fragment thereof (e.g., Fab, F(ab)₂, scFv) made in a non-human animal of aspects of the disclosure, or derived from a sequence made in a mouse of aspects of the disclosure, is described.

### Bispecific-Binding Proteins

Immunoglobulin-like binding proteins comprising human variable domains that specifically bind a pMHC complex of interest are described. Cells expressing such binding proteins, mice that make them, and related methods and compositions are also described.

In some aspects, binding proteins, and nucleotide sequences encoding them, can be used to make multispecific binding proteins, e.g., bispecific binding proteins. In this aspect, a first polypeptide comprising a first heavy chain variable domain can associate with a second polypeptide comprising a second heavy chain variable domain. Where the first heavy chain variable domain and the second heavy chain variable domain specifically bind a different epitope, a bispecific-binding molecule can be made using the two heavy chain variable domains. The C_{H} region can be the same or different. In one aspect, e.g., one of the C_{H} regions can be modified so as to eliminate a protein A binding determinant, whereas the other heavy chain constant region is not so modified (see U.S. Pat. No. 8,586,713 B2). This particular arrangement simplifies isolation of the bispecific binding protein from, *e.g.,* a mixture of homodimers (*e.g.*, homodimers of the first or the second polypeptides). In some aspects, a bispecific pMHC-binding protein may be heterodimeric with respect to Protein A binding, and may comprise
a. a first polypeptide comprising, from N-terminal to C-terminal, a first epitope-binding region that selectively binds a first epitope, an immunoglobulin constant region that comprises a first CH3 region of a human IgG selected from IgG1, IgG2, and IgG4, wherein the first CH3 region binds to Protein A; and
b. a second polypeptide comprising, from N-terminal to C-terminal, a second epitope-binding region that selectively binds a second epitope, an immunoglobulin constant region that comprises a second CH3 region of a human IgG selected from IgG1, IgG2, and IgG4, wherein the second CH3 region comprises a modification that reduces or eliminates binding of the second CH3 region to Protein A. In some aspects, the modification is selected from the group consisting of (a) 95R, and (b) 95R and 96F in the IMGT exon numbering system, or (a') 435R, and (b') 435R and 436F in the EU numbering system. In some aspects, the second CH3 region further comprises one to five modifications selected from the group consisting of 16E, 18M, 44S, 52N, 57M, and 82I in the IMGT exon numbering system, or 356E, 358M, 384S, 392N, 397M, and 422I In the EU numbering system.

In one aspect, the methods and compositions are used to make bispecific-binding proteins. In this aspect, a first V_{H} that is fused to a C_{H} region and a second V_{H} that is fused to a C_{H} region are each independently cloned in frame with a human IgG sequence of the same isotype *(e.g.,* a human IgG1, IgG2, or IgG4). The first V_{H} specifically binds a first pMHC complex, and the second V_{H} specifically binds a second pMHC complex. The first and second epitopes may be on different pMHCs, or on the same pMHC complex.

In one aspect, the IgG isotype of the C_{H} region fused to the first V_{H} and the IgG isotype of the C_{H} region fused to the second V_{H} are the same isotype, but differ in that one IgG isotype comprises at least one amino acid substitution. In one aspect, the at least one amino acid substitution renders the heavy chain bearing the substitution unable or substantially unable to bind protein A as compared with the heavy chain that lacks the substitution.

In one aspect, the first C_{H} region comprises a first C_{H}3 domain of a human IgG selected from IgG1, IgG2, and IgG4; and the second C_{H} region comprises a second C_{H}3 domain of a human IgG selected from IgG1, IgG2, and IgG4, wherein the second C_{H}3 domain comprises a modification that reduces or eliminates binding of the second C_{H}3 domain to protein A (see US Pat. 8,586,713 B2).

In one aspect, the second C_{H}3 domain comprises a 435R modification, numbered according to the EU numbering system. In another aspect, the second C_{H}3 domain further comprises a 436F modification, numbered according to the EU numbering system.

In one aspect, the second C_{H}3 domain is that of a human IgG1 that comprises a modification selected from the group consisting of D356E, L358M, N384S, K392N, V397M, and V422I, numbered according to the EU numbering system.

In one aspect, the second C_{H}3 domain is that of a human IgG2 that comprises a modification selected from the group consisting of N384S, K392N, and V422I, numbered according to the EU numbering system.

In one aspect, the second C_{H}3 domain is that of a human IgG4 comprising a modification selected from the group consisting of Q355R, N384S, K392N, V397M, R409K, E419Q, and V422I, numbered according to the EU numbering system.

In one aspect, the binding protein comprises C_{H} regions having one or more modifications as recited herein, wherein the constant region of the binding protein is nonimmunogenic or substantially nonimmunogenic in a human. In a specific aspect, the C_{H} regions comprise amino acid sequences that do not present an immunogenic epitope in a human. In another specific aspect, the binding protein comprises a C_{H} region that is not found in a wildtype human heavy chain, and the C_{H} region does not comprise a sequence that generates a T-cell epitope.

In one aspect, Fc domains can be modified to have altered Fc receptor binding, which in turn affects effector function. An engineered heavy chain constant region (C_{H}), which includes the Fc domain, may be chimeric. As such, a chimeric C_{H} region combines C_{H} domains derived from more than one immunoglobulin isotype. For example, a chimeric C_{H} region comprises part or all of a C_{H}2 domain derived from a human IgG1, human IgG2 or human IgG4 molecule, combined with part or all of a C_{H}3 domain derived from a human IgG1, human IgG2 or human IgG4 molecule. A chimeric C_{H} region can also contain a chimeric hinge region. For example, a chimeric hinge may comprise an "upper hinge" amino acid sequence (amino acid residues from positions 216 to 227 according to EU numbering) derived from a human IgG1, a human IgG2 or a human IgG4 hinge region, combined with a "lower hinge" sequence (amino acid residues from positions 228 to 236 according to EU numbering) derived from a human IgG1, a human IgG2 or a human IgG4 hinge region. In one aspect, the chimeric hinge region comprises amino acid residues derived from a human IgG1 or a human IgG4 upper hinge and amino acid residues derived from a human IgG2 lower hinge.

For certain therapies, the Fc domain may be engineered to activate all, some, or none of the normal Fc effector functions, without affecting the Fc-containing protein's (e.g. antibody's) desired pharmacokinetic properties. For examples of proteins comprising chimeric CH regions and having altered effector functions, see US Patent No. 9,359,437. In some aspects, a pMHC-binding protein comprises recombinant polypeptide comprising a heavy chain constant (CH) region comprising, from N-terminus to C-terminus, a CH1 domain, a chimeric hinge, a CH2 domain, and a CH3 domain wherein: (a) the CH1 domain comprises the amino acid sequence DKKV or DKRV from positions 212 to 215 (EU numbering), (b) the chimeric hinge comprises a human IgG1 or a human IgG4 upper hinge amino acid sequence from positions 216 to 227 (EU numbering) and a human IgG2 lower hinge amino acid sequence PCPAPPVA (SEQ ID NO: 29) from positions 228 to 236 (EU numbering), (c) the CH2 domain comprises a human IgG4 CH2 domain amino acid sequence from positions 237 to 340 (EU numbering) comprising the amino acid sequence of SEQ ID NO: 10, and (d) the CH3 domain comprises a human IgG1 or a human IgG4 CH3 domain sequence from positions 341 to 447 (EU numbering)

In some aspects, the bispecific antibody may have a first V_{H} and a second V_{H}, each having a common light chain as its cognate V_{L} domain.

### Breaking tolerance to endogenous peptides

Immunization of non-human animals (i.e. mice or rats) with an antigenic pMHC to obtain specific pMHC-binding proteins is dependent on a divergence in sequence between endogenous proteins in the non-human animal and the heterologous protein being presented to enable the non-human animal's immune system to recognize the pMHC complex of interest as non-self (i.e., foreign). The generation of antibodies against pMHC having a high degree of homology with self-pMHC can be a difficult task due to immunological tolerance to self-pMHC. Methods of breaking tolerance to self-peptides that are homologous to a peptide of interest are well-known to a skilled artisan, *see, e.g.*, US Publication No. 20170332610. In some aspects, a method of breaking tolerance to endogenous peptides comprises modifying a non-human animal herein to comprise a deletion, e.g., a knockout mutation, of the self-peptide that has a high degree of homology with the peptide of interest.

### Pharmaceutical Compositions

In certain aspects, described herein is a composition, e.g., a pharmaceutical composition, containing at least one pMHC-binding protein formulated together with a pharmaceutically acceptable carrier.

The pharmaceutical compositions described herein may be specially formulated for administration in solid or liquid form, including those adapted for the following: (1) oral administration, for example, drenches (aqueous or non-aqueous solutions or suspensions), tablets, e.g., those targeted for buccal, sublingual, and systemic absorption, boluses, powders, granules, pastes for application to the tongue; or (2) parenteral administration, for example, by subcutaneous, intramuscular, intravenous or epidural injection as, for example, a sterile solution or suspension, or sustained-release formulation.

In some aspects, pharmaceutical compositions described herein suitable for parenteral administration comprise one or more therapeutic agents of certain aspects of the disclosure in combination with one or more pharmaceutically-acceptable sterile isotonic aqueous or nonaqueous solutions, dispersions, suspensions or emulsions, or sterile powders which may be reconstituted into sterile injectable solutions or dispersions just prior to use, which may contain sugars, alcohols, antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions described herein include water, ethanol, polyols (such as, but not limited to, glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

In certain aspects, the compositions comprise pMHC-binding protein in a concentration resulting in a w/v appropriate for a desired dose. The pMHC-binding protein may be present in the composition at a concentration of at least 1 mg/mL, at least 5 mg/mL, at least 10 mg/mL, at least 15 mg/mL, at least 20 mg/mL, at least 25 mg/mL, at least 30 mg/mL, at least 35 mg/mL, at least 40 mg/mL, at least 45 mg/mL, at least 50 mg/mL, at least 55 mg/mL, at least 60 mg/mL, at least 65 mg/mL, at least 70 mg/mL, at least 75 mg/mL, at least 80 mg/mL, at least 85 mg/mL, at least 90 mg/mL, at least 95 mg/mL, at least 100 mg/mL, at least 105 mg/mL, at least 1 10 mg/mL, at least 115 mg/mL, at least 120 mg/mL, at least 125 mg/mL, at least 130 mg/mL, at least 135 mg/mL, at least 140 mg/mL, at least 150 mg/mL, at least 200 mg/mL, at least 250 mg/mL, or at least 300 mg/mL. In some aspects, the pMHC-binding protein may be present in the composition at a concentration of 1 mg/mL to 300 mg/mL. In some aspects, the pMHC-binding protein may be present in the composition at a concentration of 5 mg/mL to 250 mg/mL. In some aspects, the pMHC-binding protein may be present in the composition at a concentration of 10 mg/mL to 200 mg/mL. In some aspects, the pMHC-binding protein may be present in the composition at a concentration of 15 mg/mL to 150 mg/mL. In some aspects, the pMHC-binding protein may be present in the composition at a concentration of 20 mg/mL to 140 mg/mL. In some aspects, the pMHC-binding protein may be present in the composition at a concentration of 25 mg/mL to 135 mg/mL. In some aspects, the pMHC-binding protein may be present in the composition at a concentration of 30 mg/mL to 130 mg/mL. In some aspects, the pMHC-binding protein may be present in the composition at a concentration of 35 mg/mL to 125 mg/mL. In some aspects, the pMHC-binding protein may be present in the composition at a concentration of 40 mg/mL to 120 mg/mL. In some aspects, the pMHC-binding protein may be present in the composition at a concentration of 45 mg/mL to 115 mg/mL. In some aspects, the pMHC-binding protein may be present in the composition at a concentration of 50 mg/mL to 110 mg/mL.

In some aspects, the composition comprises one or more active compounds as necessary for the particular indication being treated, typically those with complementary activities that do not adversely affect each other. Such additional active compounds are suitably present in combination in amounts that are effective for the purpose intended. In some aspects, compositions are prepared by mixing a pMHC-binding protein with optional physiologically acceptable carriers, excipients or stabilizers, including, but not limited to buffering agents, saccharides, salts, surfactants, solubilizers, polyols, diluents, binders, stabilizers, salts, lipophilic solvents, amino acids, chelators, preservatives, or the like (Goodman and Gilman's The Pharmacological Basis of Therapeutics, 12th edition, L. Brunton, et al. and Remington's Pharmaceutical Sciences, 16th edition, Osol, A. Ed. (1999)), in the form of lyophilized compositions or aqueous solutions at a desired final concentration. Acceptable carriers, excipients, or stabilizers are nontoxic to recipients at the dosages and concentrations employed, and include buffers such as histidine, phosphate, citrate, glycine, acetate and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than 10 to 15 residues) polypeptide; proteins, such as serum albumin, gelatin, or immunoglobulins; hydrophilic polymers such as polyvinylpyrolidone; amino acids such as glycine, glutamine, asparagine, histidine, arginine, or lysine; monosaccharides, disaccharides, and other carbohydrates including trehalose, glucose, mannose, or dextrins; chelating agents such as EDTA; sugars such as sucrose, mannitol, trehalose or sorbitol; salt-forming counter-ions such as sodium; metal complexes (e.g. , Zn-protein complexes); and/or non-ionic surfactants such as TWEEN, polysorbate 80, PLURONICS^{®} or polyethylene glycol (PEG).

In some aspects, the buffering agent is histidine, citrate, phosphate, glycine, or acetate. The saccharide excipient may be trehalose, sucrose, mannitol, maltose or raffinose. The surfactant may be polysorbate 20, polysorbate 40, polysorbate 80, or Pluronic F68. The salt may be NaCl, KCl, MgCl₂, or CaCl₂.

In some aspects, the composition comprises a buffering or pH adjusting agent to provide improved pH control. Such a composition may have a pH of between about 3.0 and about 9.0, between about 4.0 and about 8.0, between about 5.0 and about 8.0, between about 5.0 and about 7.0, between about 5.0 and about 6.5, between about 5.5 and about 8.0, between about 5.5 and about 7.0, or between about 5.5 and about 6.5. In a further aspect, such a composition has a pH of about 3.0, about 3.5, about 4.0, about 4.5, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.5, about 8.0, about 8.5, or about 9.0. In a specific aspect, a composition has a pH of about 6.0.

In some aspects, the composition comprises a buffering or pH adjusting agent to provide improved pH control. Such a composition may have a pH of between 3.0 and 9.0, between 4.0 and 8.0, between 5.0 and 8.0, between 5.0 and 7.0, between 5.0 and 6.5, between 5.5 and 8.0, between 5.5 and 7.0, or between 5.5 and 6.5. In a further aspect, such a composition has a pH of 3.0, 3.5, 4.0, 4.5, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.5, 8.0, 8.5, or 9.0. In a specific aspect, a composition has a pH of 6.0.

One of skill in the art understands that the pH of a composition generally should not be equal to the isoelectric point of the particular pMHC-binding protein to be used in the composition. Typically, the buffering agent is a salt prepared from an organic or inorganic acid or base. Representative buffering agents include, but are not limited to, organic acid salts such as salts of citric acid, ascorbic acid, gluconic acid, carbonic acid, tartaric acid, succinic acid, acetic acid, or phthalic acid; Tris, tromethamine hydrochloride, or phosphate buffers. In addition, amino acid components can also function in a buffering capacity. Representative amino acid components which may be utilized in the composition as buffering agents include, but are not limited to, glycine and histidine. In certain aspects, the buffering agent is chosen from histidine, citrate, phosphate, glycine, and acetate. In a specific aspect, the buffering agent is histidine. In another specific aspect, the buffering agent is citrate. In yet another specific aspect, the buffering agent is glycine. The purity of the buffering agent should be at least 98%, or at least 99%, or at least 99.5%. As used herein, the term "purity" in the context of histidine and glycine refers to chemical purity of histidine or glycine as understood in the art, e.g., as described in The Merck Index, 13th ed., O'Neil et al. ed. (Merck & Co., 2001).

In certain aspects, the composition comprises histidine as a buffering agent. In certain aspects the histidine is present in the composition at a concentration of at least about 1 mM, at least about 5 mM, at least about 10 mM, at least about 20 mM, at least about 30 mM, at least about 40 mM, at least about 50 mM, at least about 75 mM, at least about 100 mM, at least about 150 mM, or at least about 200 mM histidine. In another aspect, a composition comprises between about 1 mM and about 200 mM, between about 1 mM and about 150 mM, between about 1 mM and about 100 mM, between about 1 mM and about 75 mM, between about 10 mM and about 200 mM, between about 10 mM and about 150 mM, between about 10 mM and about 100 mM, between about 10 mM and about 75 mM, between about 10 mM and about 50 mM, between about 10 mM and about 40 mM, between about 10 mM and about 30 mM, between about 20 mM and about 75 mM, between about 20 mM and about 50 mM, between about 20 mM and about 40 mM, or between about 20 mM and about 30 mM histidine. In a further aspect, the composition comprises about 1 mM, about 5 mM, about 10 mM, about 20 mM, about 25 mM, about 30 mM, about 35 mM, about 40 mM, about 45 mM, about 50 mM, about 60 mM, about 70 mM, about 80 mM, about 90 mM, about 100 mM, about 150 mM, or about 200 mM histidine. In a specific aspect, a composition may comprise about 10 mM, about 25 mM, or no histidine.

In certain aspects, the composition comprises histidine as a buffering agent. In certain aspects the histidine is present in the composition at a concentration of at least 1 mM, at least 5 mM, at least 10 mM, at least 20 mM, at least 30 mM, at least 40 mM, at least 50 mM, at least 75 mM, at least 100 mM, at least 150 mM, or at least 200 mM histidine. In another aspect, a composition comprises between 1 mM and 200 mM, between 1 mM and 150 mM, between 1 mM and 100 mM, between 1 mM and 75 mM, between 10 mM and 200 mM, between 10 mM and 150 mM, between 10 mM and 100 mM, between 10 mM and 75 mM, between 10 mM and 50 mM, between 10 mM and 40 mM, between 10 mM and 30 mM, between 20 mM and 75 mM, between 20 mM and 50 mM, between 20 mM and 40 mM, or between 20 mM and 30 mM histidine. In a further aspect, the composition comprises 1 mM, 5 mM, 10 mM, 20 mM, 25 mM, 30 mM, 35 mM, 40 mM, 45 mM, 50 mM, 60 mM, 70 mM, 80 mM, 90 mM, 100 mM, 150 mM, or 200 mM histidine. In a specific aspect, a composition may comprise 10 mM, 25 mM, or no histidine.

In some aspects, the composition comprises a carbohydrate excipient. Carbohydrate excipients can act, e.g., as viscosity enhancing agents, stabilizers, bulking agents, solubilizing agents, and/or the like. Carbohydrate excipients are generally present at between about 1% to about 99% by weight or volume, e.g., between about 0.1% to about 20%, between about 0.1% to about 15%, between about 0.1% to about 5%, between about 1% to about 20%, between about 5% to about 15%, between about 8% to about 10%, between about 10% and about 15%, between about 15% and about 20%, between 0.1% to 20%, between 5% to 15%, between 8% to 10%, between 10% and 15%, between 15% and 20%, between about 0.1% to about 5%, between about 5% to about 10%, or between about 15% to about 20%. In still other specific aspects, the carbohydrate excipient is present at 1%, or at 1.5%, or at 2%, or at 2.5%, or at 3%, or at 4%, or at 5%, or at 10%, or at 15%, or at 20%.

In some aspects, the composition comprises a carbohydrate excipient. Carbohydrate excipients can act, e.g., as viscosity enhancing agents, stabilizers, bulking agents, solubilizing agents, and/or the like. Carbohydrate excipients are generally present at between 1% to 99% by weight or volume, e.g., between 0.1% to 20%, between 0.1% to 15%, between 0.1% to 5%, between 1% to 20%, between 5% to 15%, between 8% to 10%, between 10% and 15%, between 15% and 20%, between 0.1% to 20%, between 5% to 15%, between 8% to 10%, between 10% and 15%, between 15% and 20%, between 0.1% to 5%, between 5% to 10%, or between 15% to 20%. In still other specific aspects, the carbohydrate excipient is present at 1%, or at 1.5%, or at 2%, or at 2.5%, or at 3%, or at 4%, or at 5%, or at 10%, or at 15%, or at 20%.

In some aspects, the composition comprises a carbohydrate excipient. Carbohydrate excipients suitable for use in the compositions include, but are not limited to, monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol) and the like. In certain aspects, the carbohydrate excipients for use in the compositions described herein are chosen from sucrose, trehalose, lactose, mannitol, and raffinose. In a specific aspect, the carbohydrate excipient is trehalose. In another specific aspect, the carbohydrate excipient is mannitol. In yet another specific aspect, the carbohydrate excipient is sucrose. In still another specific aspect, the carbohydrate excipient is raffinose. The purity of the carbohydrate excipient should be at least 98%, or at least 99%, or at least 99.5%.

In some aspects, the composition comprises trehalose. In certain aspects, a composition comprises at least about 1%, at least about 2%, at least about 4%, at least about 8%, at least about 20%, at least about 30%, or at least about 40% trehalose. In another aspect, a composition comprises between about 1% and about 40%, between about 1% and about 30%, between about 1% and about 20%, between about 2% and about 40%, between about 2% and about 30%, between about 2% and about 20%, between about 4% and about 40%, between about 4% and about 30%, or between about 4% and about 20% trehalose. In a further aspect, a composition comprises about 1%, about 2%, about 4%, about 6%, about 8%, about 15%, about 20%, about 30%, or about 40% trehalose. In a specific aspect, a composition comprises about 4%, about 6% or about 15% trehalose.

In some aspects, the composition comprises trehalose. In certain aspects, a composition comprises at least 1%, at least 2%, at least 4%, at least 8%, at least 20%, at least 30%, or at least 40% trehalose. In another aspect, a composition comprises between 1% and 40%, between 1% and 30%, between 1% and 20%, between 2% and 40%, between 2% and 30%, between 2% and 20%, between 4% and 40%, between 4% and 30%, or between 4% and 20% trehalose. In a further aspect, a composition comprises 1%, 2%, 4%, 6%, 8%, 15%, 20%, 30%, or 40% trehalose. In a specific aspect, a composition comprises 4%, 6% or 15% trehalose.

In certain aspects, the composition comprises an excipient. In a specific aspect, a composition comprises at least one excipient chosen from: sugar, salt, surfactant, amino acid, polyol, chelating agent, emulsifier and preservative. In certain aspects, a composition comprises a salt, e.g., a salt selected from: NaCl, KCl, CaCl₂, and MgCl₂. In a specific aspect, the composition comprises NaCl.

In some aspects, the composition comprises an amino acid, e.g., lysine, arginine, glycine, histidine or an amino acid salt. The composition may comprise at least about 1 mM, at least about 10 mM, at least about 25 mM, at least about 50 mM, at least about 100 mM, at least about 150 mM, at least about 200 mM, at least about 250 mM, at least about 300 mM, at least about 350 mM, or at least about 400 mM of an amino acid. In another aspect, the composition may comprise between about 1 mM and about 100 mM, between about 10 mM and about 150 mM, between about 25 mM and about 250 mM, between about 25 mM and about 300 mM, between about 25 mM and about 350 mM, between about 25 mM and about 400 mM, between about 50 mM and about 250 mM, between about 50 mM and about 300 mM, between about 50 mM and about 350 mM, between about 50 mM and about 400 mM, between about 100 mM and about 250 mM, between about 100 mM and about 300 mM, between about 100 mM and about 400 mM, between about 150 mM and about 250 mM, between about 150 mM and about 300 mM, or between about 150 mM and about 400 mM of an amino acid. In a further aspect, a composition comprises about 1 mM, 1.6 mM, 25 mM, about 50 mM, about 100 mM, about 150 mM, about 200 mM, about 250 mM, about 300 mM, about 350 mM, or about 400 mM of an amino acid.

In some aspects, the composition comprises an amino acid, e.g., lysine, arginine, glycine, histidine or an amino acid salt. The composition may comprise at least 1 mM, at least 10 mM, at least 25 mM, at least 50 mM, at least 100 mM, at least 150 mM, at least 200 mM, at least 250 mM, at least 300 mM, at least 350 mM, or at least 400 mM of an amino acid. In another aspect, the composition may comprise between 1 mM and 100 mM, between 10 mM and 150 mM, between 25 mM and 250 mM, between 25 mM and 300 mM, between 25 mM and 350 mM, between 25 mM and 400 mM, between 50 mM and 250 mM, between 50 mM and 300 mM, between 50 mM and 350 mM, between 50 mM and 400 mM, between 100 mM and 250 mM, between 100 mM and 300 mM, between 100 mM and 400 mM, between 150 mM and 250 mM, between 150 mM and 300 mM, or between 150 mM and 400 mM of an amino acid. In a further aspect, a composition comprises 1 mM, 1.6 mM, 25 mM, 50 mM, 100 mM, 150 mM, 200 mM, 250 mM, 300 mM, 350 mM, or 400 mM of an amino acid.

In some aspects, the composition comprises a surfactant. The term "surfactant" as used herein refers to organic substances having amphipathic structures; namely, they are composed of groups of opposing solubility tendencies, typically an oil-soluble hydrocarbon chain and a water-soluble ionic group. Surfactants can be classified, depending on the charge of the surface-active moiety, into anionic, cationic, and nonionic surfactants. Surfactants are often used as wetting, emulsifying, solubilizing, and dispersing agents for various pharmaceutical compositions and preparations of biological materials. Pharmaceutically acceptable surfactants like polysorbates (e.g., polysorbates 20 or 80); polyoxamers (e.g., poloxamer 188); Triton; sodium octyl glycoside; lauryl-, myristyl-, linoleyl-, or stearyl-sulfobetaine; lauryl-, myristyl-, linoleyl- or stearyl-sarcosine; linoleyl-, myristyl-, or cetyl-betaine; lauroamidopropyl-, cocamidopropyl-, linoleamidopropyl-, myristamidopropyl-, palmidopropyl-, or isostearamidopropyl-betaine (e.g. , lauroamidopropyl); myristamidopropyl-, palmidopropyl-, or isostearamidopropyl-dimethylamine; sodium methyl cocoyl-, or disodium methyl oleyl-taurate; and the MONAQUA^{®} series (Mona Industries, Inc., Paterson, N.J.), poly ethyl glycol, polypropyl glycol, and copolymers of ethylene and propylene glycol (e.g., PLURONICS^{®} PF68, etc.), can optionally be added to the compositions to reduce aggregation. In certain aspects, a composition comprises Polysorbate 20, Polysorbate 40, Polysorbate 60, or Polysorbate 80. Surfactants are particularly useful if a pump or plastic container is used to administer the composition. The presence of a pharmaceutically acceptable surfactant mitigates the propensity for the protein to aggregate. The compositions may comprise a polysorbate which is at a concentration ranging from between about 0.001% to about 1%, or about 0.001% to about 0.1%, or about 0.01% to about 0.1%. In other specific aspects, the compositions comprise a polysorbate which is at a concentration of 0.001%, or 0.002%, or 0.003%, or 0.004%, or 0.005%, or 0.006%, or 0.007%, or 0.008%, or 0.009%, or 0.01%, or 0.015%, or 0.02%. The compositions may comprise a polysorbate which is at a concentration ranging from between 0.001% to 1%, or 0.001% to 0.1%, or 0.01% to 0.1%. In other specific aspects, the compositions comprise a polysorbate which is at a concentration of 0.001%, or 0.002%, or 0.003%, or 0.004%, or 0.005%, or 0.006%, or 0.007%, or 0.008%, or 0.009%, or 0.01%, or 0.015%, or 0.02%.

In some aspects, the composition comprises other excipients and/or additives including, but not limited to, diluents, binders, stabilizers, lipophilic solvents, preservatives, adjuvants, or the like. Pharmaceutically acceptable excipients and/or additives may be used in the compositions described herein. Commonly used excipients/additives, such as pharmaceutically acceptable chelators (for example, but not limited to, EDTA, DTPA or EGTA) can optionally be added to the compositions to reduce aggregation. These additives are particularly useful if a pump or plastic container is used to administer the composition.

In some aspects, the composition comprises a preservative. Preservatives, such as phenol, m-cresol, p-cresol, o-cresol, chlorocresol, benzyl alcohol, phenylmercuric nitrite, phenoxyethanol, formaldehyde, chlorobutanol, magnesium chloride (for example, but not limited to, hexahydrate), alkylparaben (methyl, ethyl, propyl, butyl and the like), benzalkonium chloride, benzethonium chloride, sodium dehydroacetate and thimerosal, or mixtures thereof can optionally be added to the compositions at any suitable concentration such as between 0.001% to 5%, or any range or value therein. The concentration of preservative used in the compositions is a concentration sufficient to yield a microbial effect. Such concentrations are dependent on the preservative selected and are readily determined by the skilled artisan.

In some aspects, the composition is isotonic with human blood, wherein the compositions have essentially the same osmotic pressure as human blood. Such isotonic compositions will generally have an osmotic pressure from 250 mOSm to 350 mOSm. Isotonicity can be measured by, for example, using a vapor pressure or ice-freezing type osmometer. Tonicity of a composition is adjusted by the use of tonicity modifiers. "Tonicity modifiers" are those pharmaceutically acceptable inert substances that can be added to the composition to provide an isotonity of the composition. Tonicity modifiers suitable for the compositions described herein include, but are not limited to, saccharides, salts and amino acids.

In certain aspects, the composition is a pyrogen-free composition which is substantially free of endotoxins and/or related pyrogenic substances. Endotoxins include toxins that are confined inside a microorganism and are released only when the microorganisms are broken down or die. Pyrogenic substances also include fever-inducing, thermostable substances from the outer membrane of bacteria and other microorganisms. Both of these substances can cause fever, hypotension and shock if administered to humans. Due to the potential harmful effects, even low amounts of endotoxins must be removed from intravenously administered pharmaceutical drug solutions. The Food & Drug Administration ("FDA") has set an upper limit of 5 endotoxin units (EU) per dose per kilogram body weight in a single one-hour period for intravenous drug applications (The United States Pharmacopeial Convention, Pharmacopeial Forum 26 (1):223 (2000)). When therapeutic proteins are administered in amounts of several hundred or thousand milligrams per kilogram body weight, as can be the case with proteins of interest (e.g., antibodies), even trace amounts of harmful and dangerous endotoxin must be removed. In some aspects, the endotoxin and pyrogen levels in the composition are less than 10 EU/mg, or less than 5 EU/mg, or less than 1 EU/mg, or less than 0.1 EU/mg, or less than 0.01 EU/mg, or less than 0.001 EU/mg.

In some aspects, when used for in vivo administration, a pharmaceutical composition should be sterile. The composition may be sterilized by various sterilization methods, including sterile filtration, radiation, etc. In certain aspects, composition is filtersterilized with a presterilized 0.22-micron filter. Sterile compositions for injection can be formulated according to conventional pharmaceutical practice as described in "Remington: The Science & Practice of Pharmacy", 21st ed., Lippincott Williams & Wilkins, (2005).

Compositions comprising a pMHC-binding protein such as those disclosed herein, ordinarily will be stored in lyophilized form or in solution. It is contemplated that sterile compositions comprising the pMHC-binding protein are placed into a container having a sterile access port, for example, an intravenous solution bag or vial having an adapter that allows retrieval of the composition, such as a stopper pierceable by a hypodermic injection needle. In certain aspects, a composition is provided as a pre-filled syringe.

In certain aspects, the composition is a lyophilized formulation. The term "lyophilized" or "freeze-dried" includes a state of a substance that has been subjected to a drying procedure such as lyophilization, where at least 50% of moisture has been removed.

Regardless of the route of administration selected, agents described herein, which may be used in a suitable hydrated form, and/or the pharmaceutical compositions described herein, are formulated into pharmaceutically-acceptable dosage forms by conventional methods known to those of skill in the art.

While the invention has been particularly shown and described with reference to a number of embodiments, it would be understood by those skilled in the art that changes in the form and details may be made to the various embodiments disclosed herein.

### EXAMPLES

The following examples are provided for illustrative purposes only and are not intended to limit the scope of the invention.

### EXAMPLE 1

Mice that comprise (1) a human or humanized MHC I locus, a human or humanized β2 microglobulin locus, and/or a human or humanized MHC II locus (see, e.g., FIGs.1-2 for non-limiting examples of such humanized loci) and (2) humanized immunoglobulin heavy and/or light chain loci (see, e.g., Macdonald et al, (2014) Proc. Natl. Acad. Sci. USA 111 :5147-52) are created using either or both breeding techniques and sequential homologous recombination in an ES cell. These mice express and are tolerized to a human or humanized MHC I/β2 microglobulin molecule and/or a human or humanized MHC II molecule. Mice are immunized with a peptide-MHC (pMHC) complex of interest that comprises a peptide that is antigenic to the mice and the human or humanized MHC against which the mouse is tolerized. Mice are additionally and optionally boosted with the pMHC complex of interest, which booster is also optionally linked to a helper T cell epitope. Human or humanized antibodies expressed from the humanized immunoglobulin heavy and/or light chain loci are isolated from the serum of immunized mice are tested for specificity of binding to the pMHC complex.

Test mice that have a C57BL background and comprise nucleotide sequences encoding a humanized MHC I molecule (HLA-A2/H-2K) associated with a humanized β2 microglobulin (see, e.g., FIGs. 1A and 1C; see also U.S. Patent Nos. 9,591,835 and 9,615,550), a humanized immunoglobulin heavy chain locus (see, e.g., Macdonald (2014), *supra*), and a humanized common light chain locus (see, e.g., U.S. Patent Nos. 10,143,186; 10,130,081 and 9,969,814; U.S. Patent Pub. Nos. 20120021409, 20120192300, 20130045492, 20130185821, 20130302836, and 20150313193) were generated. These test mice, and control mice comprising a functional (e.g., murine) ADAM6 gene (see, e.g., U.S. Pat. Nos. 8,642,835 and 8,697,940) and humanized immunoglobulin heavy and light chain loci, were immunized with a single chain pMHC complex comprising a heterologous peptide (peptide B) presented in the context of an HLA-A2/β2m molecule where the immunogen was administered as a protein immunogen, set forth as SEQ ID NO:26 (FIG. 4) or as DNA encoding the single chain pMHC complex comprising an amino acid sequence set forth as SEQ ID NO:27 (FIG. 3). The mice were boosted via different routes at varying time intervals using the pMHC complex immunogen with standard adjuvants (FIGs. 3-4) or using the pMHC complex immunogen linked a T helper peptide (PADRE; **FIG. 5**). Pre-immune serum was collected from the mice prior to the initiation of immunization. The mice were bled periodically and anti-serum titers were assayed on respective antigens.

Antibody titers in serum against irrelevant (Peptide A or Peptide C) and relevant antigens presented in the context of HLA-A2 were determined using ELISA. Ninety-six well microtiter plates (Thermo Scientific) were coated with anti-myc antibody in phosphate-buffered saline (PBS, Irvine Scientific) overnight at 5 µg/ml. Plates were washed with phosphate-buffered saline containing 0.05% Tween 20 (PBS-T, Sigma-Aldrich) and blocked with 250 µl of 0.5% bovine serum albumin (BSA, Sigma-Aldrich) in PBS for 1 h at room temperature. The plates were washed with PBS-T and coated with at 2 µg/ml c-myc-tagged single chain pMHC complexes comprising relevant Peptide B or irrelevant antigens Peptide A or Peptide C presented in the context of HLA-A2.

Pre-immune and immune anti-sera were serially diluted three-fold in 0.5% BSA-PBS and added to the plates for 1 h at room temperature. The plates were washed and goat antimouse IgG-Fc- Horse Radish Peroxidase (HRP) conjugated secondary antibody (Jackson Immunoresearch) was added to the plates and incubated for 1 h at room temperature. Plates were washed and developed using 3,3',5,5'-Tetramethylbenzidine (TMB)/H₂O₂ as substrate according to manufacturer's recommended procedure and absorbance at 450nm were recorded using a spectrophotometer (Victor, Perkin Elmer). Antibody titers were computed using Graphpad PRISM software. Antibody titers were calculated as the interpolated serum dilution factor of which the binding signal is 2-fold over background.

Antibody titers that bind a peptide B containing single chain pMHC was elicited in control mice when immunized with either the DNA immunogen encoding peptide B in the context of HLA-A or the protein immunogen comprising same, **FIGs. 3-4,** which control mice included cohorts that were not tolerized to the chimeric HLA-A2/H-2K polypeptide and/or human or humanized β2 microglobulin. However, the sera of these control mice also comprised antibody titers to irrelevant peptide (peptide A or peptide C) presented in the context of HLA-A that were comparable to the antibody titer to peptide B containing single chain pMHC (FIGs. 3-4). As such, although non-tolerized animals are able to generate an immune response against pMHC complexes of interest, such immune response may be considered a non-specific immune response due to the comparable antibody titers to the peptide B containing single chain pMHC and to the irrelevant peptide containing single chain pMHC.

In contrast, sera from test mice that were tolerized to human(ized) HLA-A and β2 microglobulin molecules were immunized with a single chain peptide B/ HLA-A/β2M complex elicited an antibody titer to the peptide B containing single chain pMHC. (**FIG. 4**) The antibody titer to pMHC complexes comprising peptide B was greater than the antibody titers to HLA-A/β2M complexes comprising irrelevant peptides A or C (**FIG. 4**). Similarly, when DNA encoding the peptide B/HLA-A/β2M complex was used as the immunogen, higher antibody titers to the relevant peptide B single chain protein (in comparison to the irrelevant pMHC complexes) were observed (**FIG. 3**). Higher titers to the immunogen were also elicited in a third cohort in which test mice were administered a prime injection of the single chain pMHC complex comprising peptide B followed by boosts with the single chain pMHC complex comprising peptide B linked to a PADRE helper T cell epitope compared to the antibody titers to irrelevant pMHC complexes (**FIG. 5**). Additionally, mice expressing a single chain HLA-A2/β2M polypeptide (SEQ ID NO:23) from the ROSA26 locus (see, e.g., **FIG. 2**) were similarly tolerized to an empty HLA-A2/β2M molecule and produced higher antibody titers to pMHC complex presenting peptide B compared to the antibody titers to a pMHC complex presenting irrelevant peptide, when immunized and boosted with the pMHC/peptide B complex protein (in the presence or absence of PADRE) (data not shown.)

### Example 2

Mice expressing an MHC class I, an MHC class II, and/or β2M molecule from a locus other than the corresponding endogenous locus, e.g., the ROSA26 locus, are tolerized to an empty MHC class I, an MHC class II, and/or β2M molecule and produce higher antibody titers to an immunogen, e.g., DNA encoding the single chain pMHC complex, when compared with mice that are not tolerized to an empty MHC class I, an MHC class II, and/or a β2M molecule.

The data herein demonstrate that tolerizing a mouse or a rat to human HLA class I and human β2 microglobulin molecules or portion thereof enhances the ability of the modified mouse or rat to generate specific antibody responses to a pMHC of interest compared to control mice or rats that are not tolerized to the human HLA class I and human β2 microglobulin molecules.

## Claims

1. A genetically modified rodent whose germline genome comprises:
(a) a nucleotide sequence encoding a human or humanized MHC molecule that comprises at least a human peptide-binding groove of a human leukocyte antigen (HLA) molecule or at least the human peptide-binding groove thereof; and
(b)
(i) an unrearranged human or humanized immunoglobulin heavy chain locus, and/or
(ii) an unrearranged human or humanized immunoglobulin light chain locus,
wherein the genetically modified rodent expresses the human or humanized MHC molecule or at least the human peptide-binding groove thereof,
wherein the genetically modified rodent expresses in a B-cell a human or humanized antibody comprising:
(i) a human or humanized heavy chain variable domain, expressed from a rearranged immunoglobulin heavy chain variable region locus formed from said unrearranged human or humanized immunoglobulin heavy chain locus; and/or
(ii) a human or humanized light chain variable domain, expressed from a rearranged immunoglobulin light chain variable region locus formed from said unrearranged human or humanized immunoglobulin light chain locus,
wherein the genetically modified rodent is tolerized to:
the human or humanized MHC molecule or at least the human peptide-binding groove thereof, such that it generates a specific B-cell response that generates a human or humanized antibody that:
(A) specifically binds an antigenic peptide-MHC (pMHC) complex that comprises
(i) a peptide that is heterologous to the genetically modified rodent complexed with
(ii) the HLA molecule or at least the human peptide-binding groove thereof, and
(B) does not bind an empty HLA molecule from which the human peptide-binding groove is derived,
when immunized with said antigenic pMHC complex, and
wherein the genetically modified rodent further comprises:
(c) said antigenic pMHC complex that comprises:
(i) said peptide that is heterologous to the genetically modified rodent complexed with
(ii) said HLA molecule or at least the human peptide-binding groove thereof, and
(d) said human or humanized antibody that specifically binds said antigenic pMHC complex and does not bind the empty HLA molecule, and
wherein the genetically modified rodent is a rat or a mouse.

2. The genetically modified rodent of claim 1, wherein the human or humanized MHC molecule is selected from the group consisting of a human or humanized MHC class I molecule, a human or humanized MHC class II α molecule, a human or humanized MHC class II β molecule, or any combination thereof, and/or
wherein the genetically modified rodent comprises:
(a) at an endogenous heavy chain locus:
(i) an unrearranged human or humanized immunoglobulin heavy chain variable region in operable linkage to an endogenous heavy chain constant region;
(ii) a restricted unrearranged human or humanized heavy chain variable region in operable linkage to an endogenous heavy chain constant region;
(iii) a common heavy chain encoding sequence;
(iv) a histidine modified unrearranged human or humanized heavy chain variable region in operable linkage to an endogenous heavy chain constant region;
(v) a heavy chain only immunoglobulin encoding sequence; or
(vi) an unrearranged human or humanized hybrid heavy chain sequence encoding a hybrid immunoglobulin chain;
and/or
(b) at an endogenous light chain locus:
(i) an unrearranged human or humanized immunoglobulin light chain variable region in operable linkage to an endogenous light chain constant region;
(ii) a common light chain encoding sequence;
(iii) a restricted unrearranged human or humanized light chain variable region in operable linkage to an endogenous light chain constant region;
(iv) a histidine modified unrearranged human or humanized light chain variable region in operable linkage to an endogenous light chain constant region; or
(v) a histidine modified rearranged human or humanized light chain variable region in operable linkage to an endogenous light chain constant region.

3. The genetically modified rodent of claim 1 or claim 2, wherein the genetically modified rodent:
(i) further comprises a functional ADAM6 gene, optionally wherein the functional ADAM6 gene is an endogenous ADAM6 gene; and/or
(ii) further expresses an exogenous terminal deoxynucleotidyl transferase (TdT) gene.

4. The genetically modified rodent of any of the preceding claims, wherein the human or humanized MHC molecule is a human or humanized MHC class I molecule, optionally wherein the human peptide-binding groove is derived from an HLA class I molecule selected from the group consisting of an HLA-A molecule, an HLA-B molecule, and an HLA-C molecule, optionally wherein the genetically modified rodent further comprises in its genome a nucleotide sequence encoding a human or humanized β2 microglobulin, optionally at an endogenous β2 microglobulin locus, wherein the genetically modified rodent expresses the human or humanized β2 microglobulin such that the genetically modified rodent is tolerized to the human or humanized β2 microglobulin by itself or in association with the human or humanized MHC class I molecule.

5. The genetically modified rodent of any one of claims 1-3, wherein the human or humanized MHC molecule is a human or humanized MHC class II molecule, optionally wherein the human peptide-binding groove is derived from an α chain and/or a β chain of an HLA class II molecule selected from the group consisting of an HLA-DP molecule, an HLA-DQ molecule, and an HLA-DR molecule.

6. The genetically modified rodent of any of the preceding claims, wherein the nucleotide sequence encoding the human or humanized MHC molecule or at least the human peptide-binding groove thereof, encodes a fully human MHC molecule,
optionally wherein the nucleotide sequence encoding a fully human MHC molecule does not disrupt an endogenous rodent MHC locus, optionally wherein the nucleotide sequence encoding a fully human MHC molecule is placed into an endogenous ROSA26 locus.

7. The genetically modified rodent of any one of claims 1-5, wherein the nucleotide sequence encoding the human or humanized MHC molecule or at least the human peptide-binding groove thereof, encodes a chimeric human/rodent MHC molecule comprising extracellular domains of the HLA molecule operably linked to transmembrane and cytoplasmic domains of an endogenous MHC molecule, optionally wherein the nucleotide sequence encoding the human or humanized MHC molecule or at least the human peptide-binding groove thereof, encodes:
(i) a chimeric human/rodent MHC class I molecule comprising α1, α2, and α3 domains of an HLA class I molecule selected from the group consisting of HLA-A, HLA-B, and HLA-C operably linked to the transmembrane and cytoplasmic domains of an endogenous rodent MHC class I molecule, such as an endogenous murine H-2K polypeptide, an endogenous murine H-2D polypeptide, or an endogenous murine H-2L polypeptide, and/or
(ii) a chimeric human/rodent MHC class II molecule comprising an α1 domain and an α2 domain of an HLA class II α polypeptide operably linked to the transmembrane and cytoplasmic domains of an endogenous rodent MHC class II α molecule, such as an endogenous murine H-2A α polypeptide or an endogenous murine H-2E α polypeptide, and/or a β1 domain and a β2 domain of an HLA class II β polypeptide operably linked to the transmembrane and cytoplasmic domains of an endogenous rodent MHC class II β molecule, such as an endogenous murine H-2A β polypeptide or an endogenous murine H-2E β polypeptide.

8. The genetically modified rodent of any of the preceding claims, wherein the genetically modified rodent is heterozygous for the nucleotide sequence encoding the human or humanized MHC molecule or at least the human peptide-binding groove thereof.

9. The genetically modified rodent of any of the preceding claims, wherein the genetically modified rodent is a mouse.

10. A method of making the genetically modified rodent of any of the preceding claims comprising:
(1) modifying the genome of a rodent to comprise:
(a) a nucleotide sequence encoding a human or humanized MHC molecule that comprises at least a human peptide-binding groove of an HLA molecule or at least the human peptide-binding groove thereof, and
(b) an unrearranged human or humanized immunoglobulin heavy chain locus and/or an unrearranged human or humanized immunoglobulin light chain locus, and
(2) administering to the rodent an antigenic pMHC complex that comprises a peptide heterologous to the rodent associated with said HLA molecule or at least the human peptide-binding groove thereof,
wherein the genetically modified rodent is:
A. tolerized to the human or humanized MHC molecule or at least the human peptide-binding groove thereof such that it generates a specific B-cell response that generates a human or humanized antibody that (A) specifically binds an antigenic pMHC complex that comprises (i) a peptide that is heterologous to the genetically modified rodent complexed with (ii) the HLA molecule or at least the human peptide-binding groove thereof, and (B) does not bind an empty HLA molecule from which the human peptide-binding groove is derived, when immunized with said antigenic pMHC complex, and
B. capable of expressing from a B-cell said human or humanized antibody which comprises:
(i) a human or humanized heavy chain variable domain, expressed from a rearranged immunoglobulin heavy chain variable region locus formed from said unrearranged human or humanized immunoglobulin heavy chain locus; and/or
(ii) a human or humanized light chain variable domain, expressed from a rearranged immunoglobulin light chain variable region locus formed from said unrearranged human or humanized immunoglobulin light chain locus, and
wherein the rodent is a rat or a mouse.

11. The method of claim 10, wherein the method comprises:
(a)
(1) inserting the nucleotide sequence encoding the human or humanized MHC molecule or at least the human peptide-binding groove thereof into a first ectopic locus, or
(2) replacing:
at an endogenous rodent MHC I locus, a nucleotide sequence encoding a rodent MHC I polypeptide with a nucleotide sequence encoding a human or humanized MHC I polypeptide to form a nucleotide sequence encoding the human or humanized MHC molecule, and/or
at an endogenous rodent MHC II locus, a nucleotide sequence encoding a rodent MHC II polypeptide with a nucleotide sequence encoding a human or humanized MHC II polypeptide to form a nucleotide sequence encoding the human or humanized MHC molecule, and
(b)
(i) inserting the unrearranged human or humanized immunoglobulin heavy chain locus and/or the unrearranged human or humanized immunoglobulin light chain locus into a second ectopic locus, or
(ii) replacing:
(A) at an endogenous rodent heavy chain locus, an endogenous rodent immunoglobulin heavy chain variable (V_{H}) gene segment with an unrearranged human immunoglobulin heavy chain variable (V_{H}) gene segment, and optionally replacing an endogenous rodent immunoglobulin heavy chain diversity (D_{H}) gene segment and/or an endogenous rodent immunoglobulin heavy chain joining (J_{H}) gene segment with an unrearranged human immunoglobulin heavy chain diversity (D_{H}) gene segment and/or an unrearranged human immunoglobulin heavy chain joining (J_{H}) gene segment, respectively, wherein the unrearranged human V_{H} gene segment, and optional unrearranged human D_{H} gene segment and optional unrearranged human J_{H} gene segment, are operably linked to an endogenous heavy chain constant region gene sequence, and/or
(B) at an endogenous rodent light chain locus, an endogenous rodent immunoglobulin light chain variable (V_{L}) gene segment and an endogenous rodent immunoglobulin light chain joining (J_{L}) gene segment with a human immunoglobulin light chain variable (V_{L}) gene segment and a human immunoglobulin light chain joining (J_{L}) gene segment, which are optionally rearranged to form a V_{L}/J_{L} gene sequence, wherein the human V_{L} and human J_{L} gene segments are operably linked to an endogenous light chain constant region gene sequence,
wherein:
(A) the (a)(1) nucleotide sequence encoding the human or humanized MHC molecule or at least the human peptide-binding groove thereof and the (b)(i) unrearranged human or humanized immunoglobulin heavy chain locus and/or unrearranged human or humanized immunoglobulin light chain locus are:
(I) inserted by sequential homologous recombination in a single rodent embryonic stem (ES) cell; or
(II) inserted into a first and second ES cell, wherein said first and second ES cell are used respectively to generate a first and second genetically modified rodent; or
(B) the (a)(2) nucleotide sequence encoding the rodent MHC I polypeptide and/or nucleotide sequence encoding the rodent MHC II polypeptide, and the (b)(ii) endogenous rodent V_{H}, and optionally D_{H} and/or J_{H}, gene segments, and/or endogenous rodent V_{L} and J_{L} gene segments are:
(I) replaced by sequential homologous recombination in a single rodent embryonic stem (ES) cell; or
(II) replaced in a first and second ES cell, wherein said first and second ES cell are used respectively to generate a first and second genetically modified rodent.

12. The method of claim 10 or claim 11, wherein the antigenic pMHC complex is linked to a helper T cell epitope, optionally wherein the helper T cell epitope is PADRE.

13. The method of claim 11(a)(2), wherein a humanized MHC I molecule comprises α1, α2, and α3 domains of an HLA class I molecule and at least transmembrane and cytoplasmic domains of an endogenous rodent MHC I polypeptide, and/or wherein a humanized MHC II molecule comprises α1, α2, β1, and β2 domains of an HLA class II molecule and at least transmembrane and cytoplasmic domains of an endogenous rodent MHC II polypeptide.

14. The method of claim 11(a)(2), which comprises targeting sequences encoding an endogenous MHC polypeptide extracellular domain, endogenous β2 microglobulin, and endogenous immunoglobulin variable regions and replacing them with sequences encoding a human MHC polypeptide extracellular domain, human β2 microglobulin, and human immunoglobulin variable regions, respectively.

15. A method of generating a human or humanized antibody that specifically binds an antigenic pMHC complex of interest or a nucleic acid sequence encoding the same comprising maintaining the genetically modified rodent according to any one of claims 1-9 or made according to the method of any one of claims 10-14 in conditions sufficient for the genetically modified rodent to mount an immune response to the antigenic pMHC complex of interest, wherein the antigenic pMHC complex of interest comprises a peptide that is heterologous to the genetically modified rodent and is presented in the context of the HLA molecule or at least the human peptide-binding groove thereof, optionally
comprising as a first step(s) immunizing the genetically modified rodent with the antigenic pMHC complex of interest, and optionally further boosting the immune response of the immunized genetically modified rodent, optionally wherein immunizing and/or boosting comprises administering to the genetically modified rodent the antigenic pMHC complex of interest linked to a helper T cell epitope, optionally wherein the helper T cell epitope comprises PADRE set forth as SEQ ID NO: 28.

16. A method of obtaining a nucleic acid encoding a human immunoglobulin heavy chain variable domain and/or a human immunoglobulin light chain variable domain, comprising:
isolating from a genetically modified rodent according to any one of claims 1-9 or made according to the method of any one of claims 10-14, a nucleic acid comprising a rearranged human immunoglobulin variable region gene sequence, optionally wherein the rearranged human immunoglobulin variable region gene sequence comprises at least one somatic hypermutation, that encodes a human immunoglobulin variable domain expressed by a lymphocyte of the genetically modified rodent, or a hybridoma produced from the lymphocyte,
wherein the human immunoglobulin variable domain expressed by the lymphocyte, or hybridoma produced therefrom, associates with its cognate variable domain to form an antigen-binding domain specific for the antigenic pMHC complex.

17. A method of obtaining a cell that expresses a human immunoglobulin heavy chain variable domain and/or a human immunoglobulin light chain variable domain comprising:
isolating a lymphocyte from a genetically modified rodent according to any one of claims 1-9 or made according to the method of any one of claims 10-14,
wherein the lymphocyte expresses a human immunoglobulin variable domain that forms an antigen-binding domain specific for the antigenic pMHC complex.

18. The method of claim 17, further comprising producing a hybridoma from the isolated lymphocyte.

19. An *in vitro* method of making a human immunoglobulin variable domain comprising:
expressing, in a cell, a first nucleic acid comprising a rearranged human immunoglobulin variable region gene sequence that encodes a human immunoglobulin variable domain expressed by a lymphocyte of a genetically modified rodent according to any one of claims 1-9 or made according to the method of any one of claims 10-14, or a hybridoma produced from the lymphocyte,
wherein the human immunoglobulin variable domain expressed by the lymphocyte, or hybridoma produced therefrom, associates with its cognate variable domain to form an antigen-binding domain specific for the antigenic pMHC complex,
optionally wherein the first nucleic acid further comprises a human immunoglobulin constant region gene sequence operably linked to the rearranged human immunoglobulin variable region gene sequence,
optionally wherein the human immunoglobulin constant region gene sequence is a heavy chain constant region gene sequence and comprises a modification that increases an affinity of a C_{H}2-C_{H}3 region of an IgG heavy chain constant region amino acid sequence to neonatal Fc receptor (FcRn) at a pH ranging from 5.5 to 6.0, wherein the modification is a mutation in the IgG heavy chain constant region amino acid sequence selected from the group consisting of M428L, N434S, V259I, V308F, N434A, M252Y, S254T, T256E, T250Q, H433K, N434Y, and a combination thereof.

## Patentansprüche

1. Genetisch verändertes Nagetier, dessen Keimbahngenom Folgendes umfasst:
(a) eine Nukleotidsequenz, die ein menschliches oder humanisiertes MHC-Molekül codiert, das zumindest eine menschliche Peptidbindungsfurche eines menschlichen Leukozytenantigen (HLA)-Moleküls oder zumindest die menschliche Peptidbindungsfurche davon umfasst; und
(b)
(i) einen nicht umgelagerten menschlichen oder humanisierten Immunglobulin-Schwerketten-Locus,
(ii) einen nicht umgelagerten menschlichen oder humanisierten Immunglobulin-Leichtketten-Locus, wobei das genetisch veränderte Nagetier das menschliche oder humanisierte MHC-Molekül oder zumindest dessen menschliche Peptidbindungsfurche exprimiert,
wobei das genetisch veränderte Nagetier in einer B-Zelle einen menschlichen oder humanisierten Antikörper exprimiert, der Folgendes umfasst:
(i) eine menschliche oder humanisierte variable Domäne der schweren Kette, exprimiert von einem umgelagerten Locus der variablen Region der Immunglobulin-Schwerkette, der aus dem nicht umgelagerten menschlichen oder humanisierten Immunglobulin-Schwerketten-Locus gebildet wird; und/oder
(ii) eine menschliche oder humanisierte variable Domäne der leichten Kette, exprimiert von einem umgelagerten Locus der variablen Region der Immunglobulin-Leichtkette, der aus dem nicht umgelagerten menschlichen oder humanisierten Immunglobulin-Leichtketten-Locus gebildet wird,
wobei das genetisch veränderte Nagetier Folgendes toleriert:
das menschliche oder humanisierte MHC-Molekül oder zumindest die menschliche Peptidbindungsfurche davon, so dass es eine spezifische B-Zell-Antwort erzeugt, die einen menschlichen oder humanisierten Antikörper erzeugt, der:
(A) spezifisch einen antigenen Peptid-MHC (pMHC)-Komplex bindet, der Folgendes umfasst
(i) ein Peptid, das heterolog zu dem genetisch modifizierten Nagetier ist, komplexiert mit
(ii) dem HLA-Molekül oder zumindest der menschlichen Peptidbindungsfurche davon, und
(B) kein leeres HLA-Molekül bindet, von dem die menschliche Peptidbindungsfurche abgeleitet ist,
wenn er mit dem antigenen pMHC-Komplex immunisiert wird, und
wobei das genetisch modifizierte Nagetier ferner Folgendes umfasst:
(c) den antigenen pMHC-Komplex, der Folgendes umfasst:
(i) das Peptid, das heterolog zu dem genetisch modifizierten Nagetier ist, komplexiert mit
(ii) dem HLA-Molekül oder zumindest der menschlichen Peptidbindungsfurche davon, und
(d) den menschlichen oder humanisierten Antikörper, der spezifisch den antigenen pMHC-Komplex bindet und nicht das leere HLA-Molekül bindet, und
wobei das genetisch modifizierte Nagetier eine Ratte oder eine Maus ist.

2. Genetisch verändertes Nagetier nach Anspruch 1, wobei das menschliche oder humanisierte MHC-Molekül ausgewählt ist aus der Gruppe bestehend aus einem menschlichen oder humanisierten MHC-Klasse-I-Molekül, einem menschlichen oder humanisierten MHC-Klasse-II-α-Molekül, einem menschlichen oder humanisierten MHC-Klasse-II-β-Molekül oder einer beliebigen Kombination davon, und/oder
wobei das genetisch modifizierte Nagetier Folgendes umfasst:
(a) an einem endogenen Schwerketten-Locus:
(i) eine nicht umgelagerte menschliche oder humanisierte variable Region der schweren Kette von Immunglobulin in funktioneller Verknüpfung mit einer endogenen konstanten Region der schweren Kette;
(ii) eine eingeschränkte nicht umgelagerte menschliche oder humanisierte variable Region der schweren Kette in funktioneller Verknüpfung mit einer endogenen konstanten Region der schweren Kette;
(iii) eine für eine gemeinsame schwere Kette codierende Sequenz;
(iv) eine Histidin-modifizierte, nicht umgelagerte menschliche oder humanisierte variable Region der schweren Kette in funktionsfähiger Verknüpfung mit einer endogenen konstanten Region der schweren Kette;
(v) eine codierende Sequenz für ein Immunglobulin mit ausschließlich schwerer Kette; oder
(vi) eine nicht umgelagerte menschliche oder humanisierte Hybrid-Schwerkettensequenz, die eine Hybrid-Immunglobulinkette codiert;
und/oder
(b) an einem endogenen Leichtketten-Locus:
(i) eine nicht umgelagerte menschliche oder humanisierte variable Region der leichten Kette von Immunglobulin in funktionsfähiger Verknüpfung mit einer endogenen konstanten Region der leichten Kette;
(ii) eine für eine gemeinsame leichte Kette codierende Sequenz;
(iii) eine eingeschränkte, nicht umgelagerte menschliche oder humanisierte variable Region der leichten Kette in funktionsfähiger Verknüpfung mit einer endogenen konstanten Region der leichten Kette;
(iv) eine Histidin-modifizierte, nicht umgelagerte menschliche oder humanisierte variable Region der leichten Kette in funktionsfähiger Verknüpfung mit einer endogenen konstanten Region der leichten Kette; oder
(v) eine Histidin-modifizierte, umgelagerte menschliche oder humanisierte variable Region der leichten Kette in funktionsfähiger Verknüpfung mit einer endogenen konstanten Region der leichten Kette.

3. Genetisch verändertes Nagetier nach Anspruch 1 oder 2, wobei das genetisch veränderte Nagetier:
(i) ferner ein funktionelles ADAM6-Gen umfasst, wobei das funktionelle ADAM6-Gen optional ein endogenes ADAM6-Gen ist; und/oder
(ii) ferner ein exogenes terminales Deoxynukleotidyltransferase (TdT)-Gen exprimiert.

4. Genetisch verändertes Nagetier nach einem der vorstehenden Ansprüche, wobei das menschliche oder humanisierte MHC-Molekül ein menschliches oder humanisiertes MHC-Klasse-I-Molekül ist, wobei die menschliche Peptidbindungsfurche optional von einem HLA-Klasse-I-Molekül abgeleitet ist, das ausgewählt ist aus der Gruppe bestehend aus einem HLA-A-Molekül, einem HLA-B-Molekül und einem HLA-C-Molekül, wobei das genetisch veränderte Nagetier optional in seinem Genom ferner eine Nukleotidsequenz umfasst, die ein menschliches oder humanisiertes β2-Mikroglobulin codiert, optional an einem endogenen β2-Mikroglobulin-Locus, wobei das genetisch veränderte Nagetier das menschliche oder humanisierte β2-Mikroglobulin exprimiert, so dass das genetisch veränderte Nagetier gegenüber dem menschlichen oder humanisierten β2-Mikroglobulin allein oder in Verbindung mit dem menschlichen oder humanisierten MHC-Klasse-I-Molekül tolerant ist.

5. Genetisch verändertes Nagetier nach einem der Ansprüche 1-3, wobei das menschliche oder humanisierte MHC-Molekül ein menschliches oder humanisiertes MHC-Klasse-II-Molekül ist, wobei die menschliche Peptidbindungsfurche optional von einer α-Kette und/oder einer β-Kette eines HLA-Klasse-II-Moleküls abgeleitet ist, das ausgewählt ist aus der Gruppe bestehend aus einem HLA-DP-Molekül, einem HLA-DQ-Molekül und einem HLA-DR-Molekül.

6. Genetisch verändertes Nagetier nach einem der vorstehenden Ansprüche, wobei die Nukleotidsequenz, die das menschliche oder humanisierte MHC-Molekül oder zumindest dessen menschliche Peptidbindungsfurche codiert, ein vollständig menschliches MHC-Molekül codiert,
wobei die Nukleotidsequenz, die ein vollständig menschliches MHC-Molekül codiert, optional einen endogenen MHC-Locus eines Nagetiers nicht stört, wobei die Nukleotidsequenz, die ein vollständig menschliches MHC-Molekül codiert, optional in einen endogenen ROSA26-Locus platziert wird.

7. Genetisch verändertes Nagetier nach einem der Ansprüche 1-5, wobei die Nukleotidsequenz, die das menschliche oder humanisierte MHC-Molekül oder zumindest dessen menschliche Peptidbindungsfurche codiert, ein chimäres menschliches/Nagetier-MHC-Molekül codiert, das extrazelluläre Domänen des HLA-Moleküls umfasst, die funktionell mit transmembranen und zytoplasmatischen Domänen eines endogenen MHC-Moleküls verbunden sind, wobei die Nukleotidsequenz, die das menschliche oder humanisierte MHC-Molekül oder zumindest dessen menschliche Peptidbindungsfurche codiert, optional Folgendes codiert:
(i) ein chimäres menschliches/Nagetier-MHC-Klasse-I-Molekül, umfassend α1-, α2- und α3-Domänen eines HLA-Klasse-I-Moleküls, ausgewählt aus der Gruppe bestehend aus HLA-A, HLA-B und HLA-C, die funktionell mit den transmembranen und zytoplasmatischen Domänen eines endogenen Nagetier-MHC-Klasse-I-Moleküls verbunden sind, wie beispielsweise eines endogenen murinen H-2K-Polypeptids, eines endogenen murinen H-2D-Polypeptids oder eines endogenen murinen H-2L-Polypeptids, und/oder
(ii) ein chimäres menschliches/Nagetier-MHC-Klasse-II-Molekül, umfassend eine α1-Domäne und eine α2-Domäne eines HLA-Klasse-II-α-Polypeptids, die funktionell mit den transmembranen und zytoplasmatischen Domänen eines endogenen Nagetier-MHC-Klasse-II-α-Moleküls verbunden sind, wie beispielsweise eines endogenen murinen H-2A-α-Polypeptids oder eines endogenen murinen H-2E-α-Polypeptids, und/oder eine β1-Domäne und eine β2-Domäne eines HLA-Klasse-II-β-Polypeptids, die funktionell mit den transmembranen und zytoplasmatischen Domänen eines endogenen Nagetier-MHC-Klasse-II-β-Moleküls verbunden sind, wie beispielsweise eines endogenen murinen H-2A-β-Polypeptids oder eines endogenen murinen H-2E-β-Polypeptids.

8. Genetisch verändertes Nagetier nach einem der vorstehenden Ansprüche, wobei das genetisch veränderte Nagetier heterozygot für die Nukleotidsequenz ist, die das menschliche oder humanisierte MHC-Molekül oder zumindest dessen menschliche Peptidbindungsfurche codiert.

9. Genetisch verändertes Nagetier nach einem der vorstehenden Ansprüche, wobei das genetisch veränderte Nagetier eine Maus ist.

10. Verfahren zur Herstellung des genetisch modifizierten Nagetiers nach einem der vorstehenden Ansprüche, umfassend:
(1) Modifizieren des Genoms eines Nagetiers, so dass es Folgendes umfasst:
(a) eine Nukleotidsequenz, die ein menschliches oder humanisiertes MHC-Molekül codiert, das zumindest eine menschliche Peptidbindungsfurche eines HLA-Moleküls oder zumindest die menschliche Peptidbindungsfurche davon umfasst, und
(b) einen nicht umgelagerten menschlichen oder humanisierten Immunglobulin-Schwerketten-Locus und/oder einen nicht umgelagerten menschlichen oder humanisierten Immunglobulin-Leichtketten-Locus, und
(2) Verabreichen eines antigenen pMHC-Komplexes an das Nagetier, der ein Peptid umfasst, das heterolog zu dem Nagetier ist, das mit dem HLA-Molekül oder zumindest der menschlichen Peptidbindungsfurche verbunden ist,
wobei das genetisch veränderte Nagetier
A. gegenüber dem menschlichen oder humanisierten MHC-Molekül oder zumindest dessen menschlicher Peptidbindungsfurche so tolerant ist, dass es eine spezifische B-Zell-Antwort erzeugt, die einen menschlichen oder humanisierten Antikörper erzeugt, der (A) spezifisch einen antigenen pMHC-Komplex bindet, der Folgendes umfasst: (i) ein Peptid, das zu dem genetisch veränderten Nagetier heterolog ist, das komplexiert ist mit (ii) dem HLA-Molekül oder zumindest der menschlichen Peptidbindungsfurche davon, und (B) kein leeres HLA-Molekül bindet, von dem die menschliche Peptidbindungsfurche abgeleitet ist, wenn er mit dem antigenen pMHC-Komplex immunisiert wird, und
B. in der Lage ist, von einer B-Zelle den menschlichen oder humanisierten Antikörper zu exprimieren, der Folgendes umfasst:
(i) eine menschliche oder humanisierte variable Domäne der schweren Kette, exprimiert von einem umgelagerten Locus der variablen Region der schweren Kette von Immunglobulin, der von dem nicht umgelagerten menschlichen oder humanisierten Locus der schweren Kette von Immunglobulin gebildet wird; und/oder
(ii) eine menschliche oder humanisierte variable Domäne der leichten Kette, exprimiert von einem umgelagerten Locus der variablen Region der leichten Kette von Immunglobulin, der von dem nicht umgelagerten menschlichen oder humanisierten Locus der leichten Kette von Immunglobulin gebildet wird, und
wobei das Nagetier eine Ratte oder eine Maus ist.

11. Verfahren nach Anspruch 10, wobei das Verfahren Folgendes umfasst:
(a)
(1) Einfügen der Nukleotidsequenz, die das menschliche oder humanisierte MHC-Molekül oder zumindest die Peptidbindungsfurche davon codiert, in einen ersten ektopischen Locus, oder
(2) Ersetzen von:
an einem endogenen Nagetier-MHC-1-Locus, einer Nukleotidsequenz, die ein Nagetier-MHC-I-Polypeptid codiert, durch eine Nukleotidsequenz, die ein menschliches oder humanisiertes MHC-I-Polypeptid codiert, um eine Nukleotidsequenz zu bilden, die das menschliche oder humanisierte MHC-Molekül codiert, und/oder
an einem endogenen Nagetier-MHC-II-Locus, einer Nukleotidsequenz, die ein Nagetier-MHC-II-Polypeptid codiert, durch eine Nukleotidsequenz, die ein menschliches oder humanisiertes MHC-II-Polypeptid codiert, um eine Nukleotidsequenz zu bilden, die das menschliche oder humanisierte MHC-Molekül codiert, und
(b)
(i) Einfügen des nicht umgelagerten menschlichen oder humanisierten Immunglobulin-Schwerketten-Locus und/oder des nicht umgelagerten menschlichen oder humanisierten Immunglobulin-Leichtketten-Locus in einen zweiten ektopischen Locus, oder
(ii) Ersetzen von:
(A) an einem endogenen Locus der schweren Kette eines Nagetiers, einem endogenen variablen Gensegment der schweren Kette (V_{H}-Gensegment) von Immunglobulin eines Nagetiers durch ein nicht umgelagertes menschliches variables Gensegment der schweren Kette (V_{H}-Gensegment) von Immunglobulin, und optional Ersetzen von jeweils einem endogenen Diversitäts-Gensegment der schweren Kette (D_{H}-Gensegment) von Immunglobulin eines Nagetiers und/oder einem endogenen verbindenden Gensegment der schweren Kette (J_{H}-Gensegment) von Immunglobulin eines Nagetiers durch ein nicht umgelagertes menschliches Diversitäts-Gensegment der schweren Kette (D_{H}-Gensegment) von Immunglobulin und/oder ein nicht umgelagertes menschliches verbindendes Gensegment der schweren Kette (J_{H}-Gensegment) von Immunglobulin, wobei das nicht umgelagerte menschliche V_{H}-Gensegment und das optionale nicht umgelagerte menschliche D_{H}-Gensegment und das optionale nicht umgelagerte menschliche J_{H}-Gensegment funktionell mit einer endogenen Gensequenz der konstanten Region der schweren Kette verbunden sind, und/oder
(B) an einem endogenen Locus der leichten Kette eines Nagetiers, einem endogenen variablen Gensegment der leichten Kette (V_{L}-Gensegment) von Immunglobulin eines Nagetiers und einem endogenen verbindenden Gensegment der leichten Kette (J_{L}-Gensegment) von Immunglobulin eines Nagetiers durch ein menschliches variables Gensegment der leichten Kette (V_{L}-Gensegment) von Immunglobulin und ein menschliches verbindendes Gensegment der leichten Kette (J_{L}-Gensegment) von Immunglobulin, die optional umgelagert sind, um eine V_{L}-/J_{L}-Gensequenz zu bilden, wobei die menschlichen V_{L}- und menschlichen J_{L}-Gensegmente funktionell mit einer endogenen Gensequenz der konstanten Region der leichten Kette verbunden sind,
wobei:
(A) die (a)(1) Nukleotidsequenz, die das menschliche oder humanisierte MHC-Molekül oder zumindest dessen menschliche Peptidbindungsfurche codiert, und der (b)(i) nicht umgelagerte menschliche oder humanisierte Immunglobulin-Schwerketten-Locus und/oder nicht umgelagerte menschliche oder humanisierte Immunglobulin-Leichtketten-Locus
(I) durch sequenzielle homologe Rekombination in eine einzelne embryonale Stammzelle (ES-Zelle) eines Nagetiers eingefügt werden; oder
(II) in eine erste und zweite ES-Zelle eingefügt werden, wobei die erste und zweite ES-Zelle jeweils zur Erzeugung eines ersten und zweiten genetisch veränderten Nagetiers verwendet werden; oder
(B) die (a)(2) Nukleotidsequenz, die das Nagetier-MHC 1-Polypeptid codiert, und/oder die Nukleotidsequenz, die das Nagetier-MHC II-Polypeptid codiert, und die (b)(ii) endogenen Nagetier-V_{H}- und optional -D_{H}- und/oder -J_{H}-Gensegmente und/oder endogenen Nagetier-V_{L}- und -J_{L}-Gensegmente
(I) durch sequenzielle homologe Rekombination in einer einzelnen embryonalen Stammzelle (ES-Zelle) eines Nagetiers ersetzt werden; oder
(II) in einer ersten und zweiten ES-Zelle ersetzt werden, wobei die erste und zweite ES-Zelle jeweils zur Erzeugung eines ersten und zweiten genetisch modifizierten Nagetiers verwendet werden.

12. Verfahren nach Anspruch 10 oder 11, wobei der antigene pMHC-Komplex an ein Helfer-T-Zell-Epitop gebunden ist, wobei das Helfer-T-Zell-Epitop gegebenenfalls PADRE ist.

13. Verfahren nach Anspruch 11(a)(2), wobei ein humanisiertes MHC-I-Molekül α1-, α2- und α3-Domänen eines HLA-Klasse-I-Moleküls und zumindest transmembrane und zytoplasmatische Domänen eines endogenen Nagetier-MHC-I-Polypeptids umfasst und/oder wobei ein humanisiertes MHC-II-Molekül α1-, α2-, β1- und β2-Domänen eines HLA-Klasse-II-Moleküls und zumindest transmembrane und zytoplasmatische Domänen eines endogenen Nagetier-MHC-II-Polypeptids umfasst.

14. Verfahren nach Anspruch 11(a)(2), das das Targeting von Sequenzen, die eine endogene extrazelluläre Domäne eines MHC-Polypeptids, endogenes β2-Mikroglobulin und endogene variable Regionen von Immunglobulin codieren, und das Ersetzen dieser durch Sequenzen umfasst, die eine menschliche extrazelluläre Domäne eines MHC-Polypeptids, menschliches β2-Mikroglobulin bzw. menschliche variable Regionen von Immunglobulin codieren, umfasst.

15. Verfahren zur Erzeugung eines menschlichen oder humanisierten Antikörpers, der spezifisch an einen antigenen pMHC-Komplex von Interesse oder eine dafür codierende Nukleinsäuresequenz bindet, umfassend die Aufrechterhaltung des genetisch veränderten Nagetiers nach einem der Ansprüche 1-9 oder hergestellt nach dem Verfahren eines der Ansprüche 10-14 unter Bedingungen, die ausreichen, damit das genetisch veränderte Nagetier eine Immunantwort auf den antigenen pMHC-Komplex von Interesse aufbaut, wobei der antigene pMHC-Komplex von Interesse ein Peptid umfasst, das zu dem genetisch veränderten Nagetier heterolog ist und im Kontext des HLA-Moleküls oder zumindest der menschlichen Peptidbindungsfurche davon vorhanden ist, optional
umfassend als ersten Schritt/erste Schritte das Immunisieren des genetisch veränderten Nagetiers mit dem antigenen pMHC-Komplex von Interesse und optional das weitere Verstärken der Immunantwort des immunisierten genetisch veränderten Nagetiers, wobei das Immunisieren und/oder das Verstärken optional das Verabreichen des antigenen pMHC-Komplexes von Interesse, der an ein Helfer-T-Zell-Epitop gebunden ist, an das genetisch veränderte Nagetier umfasst, wobei das Helfer-T-Zell-Epitop optional PADRE umfasst, wie in SEQ ID NO: 28 dargelegt.

16. Verfahren zum Erhalten einer Nukleinsäure, die eine menschliche variable Domäne der schweren Kette von Immunglobulin und/oder eine menschliche variable Domäne der leichten Kette von Immunglobulin codiert, umfassend:
Isolieren einer Nukleinsäure, die eine umgelagerte menschliche Gensequenz der variablen Region von Immunglobulin umfasst, von einem genetisch veränderten Nagetier nach einem der Ansprüche 1-9 oder hergestellt nach einem der Ansprüche 10-14, wobei die menschliche Gensequenz der variablen Region von Immunglobulin optional zumindest eine somatische Hypermutation umfasst, die eine menschliche variable Domäne von Immunglobulin codiert, die von einem Lymphozyten des genetisch veränderten Nagetiers oder einem von dem Lymphozyten produzierten Hybridom exprimiert wird,
wobei die menschliche variable Domäne von Immunglobulin, die von dem Lymphozyten oder dem daraus produzierten Hybridom exprimiert wird, mit ihrer verwandten variablen Domäne assoziiert, um eine Antigen-bindende Domäne zu bilden, die für den antigenen pMHC-Komplex spezifisch ist.

17. Verfahren zum Erhalten einer Zelle, die eine menschliche variable Domäne der schweren Kette von Immunglobulin und/oder eine menschliche variable Domäne der leichten Kette von Immunglobulin exprimiert, umfassend:
Isolieren eines Lymphozyten aus einem genetisch veränderten Nagetier nach einem der Ansprüche 1-9 oder hergestellt nach dem Verfahren eines der Ansprüche 10-14,
wobei der Lymphozyt eine menschliche variable Domäne von Immunglobulin exprimiert, die eine Antigen-bindende Domäne bildet, die für den antigenen pMHC-Komplex spezifisch ist.

18. Verfahren nach Anspruch 17, ferner umfassend das Produzieren eines Hybridoms von dem isolierten Lymphozyten.

19. In-vitro-Verfahren zur Herstellung einer menschlichen variablen Domäne von Immunglobulin, umfassend:
Exprimieren, in einer Zelle, einer ersten Nukleinsäure, umfassend eine umgelagerte menschliche Gensequenz der variablen Region von Immunglobulin, die eine menschliche variable Domäne von Immunglobulin codiert, die von einem Lymphozyten eines genetisch veränderten Nagetiers nach einem der Ansprüche 1-9 oder hergestellt nach dem Verfahren eines der Ansprüche 10-14 oder einem von dem Lymphozyten produzierten Hybridom exprimiert wird,
wobei die menschliche variable Domäne von Immunglobulin, die von dem Lymphozyten oder dem daraus produzierten Hybridom exprimiert wird, sich mit seiner verwandten variablen Domäne assoziiert, um eine Antigen-bindende Domäne zu bilden, die für den antigenen pMHC-Komplex spezifisch ist,
wobei die erste Nukleinsäure optional ferner eine menschliche Gensequenz der konstanten Region von Immunglobulin umfasst, die funktionell mit der umgelagerten menschlichen Gensequenz der variablen Region von Immunglobulin verbunden ist,
wobei die menschliche Gensequenz der konstanten Region von Immunglobulin optional eine Gensequenz der konstanten Region der schweren Kette ist und eine Modifikation umfasst, die die Affinität einer C_{H}2-C_{H}3-Region einer Aminosäuresequenz der konstanten Region der schweren Kette von IgG zu neonatalem Fc-Rezeptor (FcRn) bei einem pH-Wert im Bereich von 5,5 bis 6,0 erhöht, wobei die Modifikation eine Mutation in der Aminosäuresequenz der konstanten Region der schweren Kette von IgG ist, ausgewählt aus der Gruppe bestehend aus M428L, N434S, V259I, V308F, N434A, M252Y, S254T, T256E, T250Q, H433K, N434Y und einer Kombination davon.

## Revendications

1. Rongeur génétiquement modifié dont le génome de lignée germinale comprend :
(a) une séquence nucléotidique codant pour une molécule de HMC humain ou humanisé qui comprend au moins un sillon de liaison peptidique humain d'une molécule d'antigène leucocytaire humain (HLA) ou au moins son sillon de liaison peptidique humain ; et
(b)
(i) un locus de chaîne lourde d'immunoglobuline humaine ou humanisée non réarrangé, et/ou
(ii) un locus de chaîne légère d'immunoglobuline humaine ou humanisée non réarrangé,
dans lequel le rongeur génétiquement modifié exprime la molécule de MHC humain ou humanisé ou au moins son sillon de liaison peptidique humain,
dans lequel le rongeur génétiquement modifié exprime dans une cellule B un anticorps humain ou humanisé comprenant :
(i) un domaine variable de chaîne lourde humain ou humanisé, exprimé à partir d'un locus de région variable de chaîne lourde d'immunoglobuline réarrangé formé à partir dudit locus de chaîne lourde d'immunoglobuline humaine ou humanisée non réarrangé ; et/ou
(ii) un domaine variable de chaîne légère humain ou humanisé, exprimé à partir d'un locus de région variable de chaîne légère d'immunoglobuline réarrangé formé à partir dudit locus de chaîne légère d'immunoglobuline humaine ou humanisée non réarrangé,
dans lequel le rongeur génétiquement modifié est rendu tolérant à :
la molécule de MHC humain ou humanisé ou au moins son sillon de liaison peptidique humain, de telle sorte qu'il génère une réponse de cellules B spécifique qui génère un anticorps humain ou humanisé qui :
(A) se lie spécifiquement à un complexe peptide-MHC (pMHC) antigénique qui comprend
(i) un peptide qui est hétérologue au rongeur génétiquement modifié complexé avec
(ii) la molécule de HLA ou au moins son sillon de liaison peptidique humain, et
(B) ne se lie pas à une molécule de HLA vide à partir de laquelle le sillon de liaison peptidique humain est dérivé,
lorsqu'il est immunisé avec ledit complexe pMHC antigénique, et
dans lequel le rongeur génétiquement modifié comprend en outre :
(c) ledit complexe pMHC antigénique qui comprend :
(i) ledit peptide qui est hétérologue au rongeur génétiquement modifié complexé avec
(ii) ladite molécule de HLA ou au moins son sillon de liaison peptidique humain, et
(d) ledit anticorps humain ou humanisé qui se lie spécifiquement audit complexe pMHC et ne se lie pas à la molécule de HLA vide, et
dans lequel le rongeur génétiquement modifié est un rat ou une souris.

2. Rongeur génétiquement modifié selon la revendication 1, dans lequel la molécule de MHC humain ou humanisé est sélectionnée dans le groupe constitué par une molécule de MHC humain ou humanisé de classe I, une molécule de MHC humain ou humanisé de classe II α, une molécule de MHC humain ou humanisé de classe II β, ou toute combinaison de celles-ci, et/ou
dans lequel le rongeur génétiquement modifié comprend :
(a) au niveau d'un locus de chaîne lourde endogène :
(i) une région variable de chaîne lourde d'immunoglobuline humaine ou humanisée non réarrangée en liaison opérationnelle avec une région constante de chaîne lourde endogène ;
(ii) une région variable de chaîne lourde humaine ou humanisée non réarrangée restreinte en liaison opérationnelle avec une région constante de chaîne lourde endogène ;
(iii) une séquence codante de chaîne lourde commune ;
(iv) une région variable de chaîne lourde humaine ou humanisée non réarrangée modifiée par histidine en liaison opérationnelle avec une région constante de chaîne lourde endogène ;
(v) une séquence codante d'immunoglobuline à chaîne lourde uniquement ; ou
(vi) une séquence de chaîne lourde hybride humaine ou humanisée non réarrangée codant pour une chaîne d'immunoglobuline hybride ;
et/ou
(b) au niveau d'un locus de chaîne légère endogène :
(i) une région variable de chaîne légère d'immunoglobuline humaine ou humanisée non réarrangée en liaison opérationnelle avec une région constante de chaîne légère endogène ;
(ii) une séquence codante de chaîne légère commune ;
(iii) une région variable de chaîne légère humaine ou humanisée non réarrangée restreinte en liaison opérationnelle avec une région constante de chaîne légère endogène ;
(iv) une région variable de chaîne légère humaine ou humanisée non réarrangée modifiée par histidine en liaison opérationnelle avec une région constante de chaîne légère endogène ; ou
(v) une région variable de chaîne légère humaine ou humanisée réarrangée modifiée par histidine en liaison opérationnelle avec une région constante de chaîne légère endogène.

3. Rongeur génétiquement modifié selon la revendication 1 ou la revendication 2, dans lequel le rongeur génétiquement modifié :
(i) comprend en outre un gène ADAM6 fonctionnel, éventuellement dans lequel le gène ADAM6 fonctionnel est un gène ADAM6 endogène ; et/ou
(ii) exprime en outre un gène de désoxynucléotidyl transférase terminale (TdT) exogène.

4. Rongeur génétiquement modifié selon l'une quelconque des revendications précédentes, dans lequel la molécule de MHC humain ou humanisé est une molécule de MHC humain ou humanisé de classe I, éventuellement dans lequel le sillon de liaison peptidique humain est dérivé d'une molécule de HLA de classe I sélectionnée dans le groupe constitué par une molécule de HLA-A, une molécule de HLA-B, et une molécule de HLA-C, éventuellement dans lequel le rongeur génétiquement modifié comprend en outre dans son génome une séquence nucléotidique codant pour une microglobuline β2 humaine ou humanisée, éventuellement au niveau d'un locus de microglobuline β2 endogène, dans lequel le rongeur génétiquement modifié exprime la microglobuline β2 humaine ou humanisée de telle sorte que le rongeur génétiquement modifié est rendu tolérant à la microglobuline β2 humaine ou humanisée par elle-même ou en association avec la molécule de MHC humain ou humanisé de classe I.

5. Rongeur génétiquement modifié selon l'une quelconque des revendications 1 à 3, dans lequel la molécule de MHC humain ou humanisé est une molécule de MHC humain ou humanisé de classe II, éventuellement dans lequel le sillon de liaison peptidique humain est dérivé d'une chaîne α et/ou d'une chaîne β d'une molécule de HLA de classe II sélectionnée dans le groupe constitué par une molécule de HLA-DP, une molécule de HLA-DQ, et une molécule de HLA-DR.

6. Rongeur génétiquement modifié selon l'une quelconque des revendications précédentes, dans lequel la séquence nucléotidique codant pour la molécule de MHC humain ou humanisé ou au moins son sillon de liaison peptidique humain, code pour une molécule de MHC pleinement humain,
éventuellement dans lequel la séquence nucléotidique codant pour une molécule de MHC pleinement humain ne perturbe pas un locus de MHC de rongeur endogène, éventuellement dans lequel la séquence nucléotidique codant pour une molécule de MHC pleinement humain est placée dans un locus ROSA26 endogène.

7. Rongeur génétiquement modifié selon l'une quelconque des revendications 1 à 5, dans lequel la séquence nucléotidique codant pour la molécule de MHC humain ou humanisé ou au moins son sillon de liaison peptidique humain, code pour une molécule de MHC humain/de rongeur chimérique comprenant des domaines extracellulaires de la molécule de HLA liés opérationnellement à des domaines transmembranaires et cytoplasmiques d'une molécule de MHC endogène, éventuellement dans lequel la séquence nucléotidique codant pour la molécule de MHC humain ou humanisé ou au moins son sillon de liaison peptidique humain, code pour :
(i) une molécule de MHC humain/de rongeur de classe I chimérique comprenant des domaines α1, α2, et α3 d'une molécule de HLA de classe I sélectionnée dans le groupe constitué par HLA-A, HLA-B, et HLA-C liée opérationnellement aux domaines transmembranaires et cytoplasmiques d'une molécule de MHC de rongeur de classe I endogène, tel qu'un polypeptide H-2K murin endogène, un polypeptide H-2D murin endogène, ou un polypeptide H-2L murin endogène, et/ou
(ii) une molécule de MHC humain/de rongeur de classe II chimérique comprenant un domaine α1 et un domaine α2 d'un polypeptide de HLA de classe II α lié opérationnellement aux domaines transmembranaires et cytoplasmiques d'une molécule de MHC de rongeur de classe II α endogène, telle qu'un polypeptide H-2A α murin endogène ou un polypeptide H-2E α murin endogène, et/ou un domaine β1 et un domaine β2 d'un polypeptide de HLA de classe II β lié opérationnellement aux domaines transmembranaires et cytoplasmiques d'une molécule de MHC de rongeur de classe II β endogène, tel qu'un polypeptide H-2A β murin endogène ou un polypeptide H-2E β murin endogène.

8. Rongeur génétiquement modifié selon l'une quelconque des revendications précédentes, dans lequel le rongeur génétiquement modifié est hétérozygote pour la séquence nucléotidique codant pour la molécule de MHC humain ou humanisé ou au moins son sillon de liaison peptidique humain.

9. Rongeur génétiquement modifié selon l'une quelconque des revendications précédentes, dans lequel le rongeur génétiquement modifié est une souris.

10. Procédé de production du rongeur génétiquement modifié de l'une quelconque des revendications précédentes comprenant :
(1) la modification du génome d'un rongeur pour comprendre :
(a) une séquence nucléotidique codant pour une molécule de HMC humain ou humanisé qui comprend au moins un sillon de liaison peptidique humain d'une molécule de HLA ou au moins son sillon de liaison peptidique humain ; et
(b) un locus de chaîne lourde d'immunoglobuline humaine ou humanisée non réarrangé et/ou un locus de chaîne légère d'immunoglobuline humaine ou humanisée non réarrangé, et
(2) l'administration au rongeur d'un complexe pMHC antigénique qui comprend un peptide hétérologue au rongeur associé à ladite molécule de HLA ou au moins son sillon de liaison peptidique humain,
dans lequel le rongeur génétiquement modifié est :
A. rendu tolérant à la molécule de MHC humain ou humanisé ou au moins son sillon de liaison peptidique humain de telle sorte qu'il génère une réponse de cellules B spécifique qui génère un anticorps humain ou humanisé qui (A) se lie spécifiquement à un complexe pMHC antigénique qui comprend (i) un peptide qui est hétérologue au rongeur génétiquement modifié complexé avec (ii) la molécule de HLA ou au moins son sillon de liaison peptidique humain, et (B) ne se lie pas à une molécule de HLA vide à partir de laquelle le sillon de liaison peptidique humain est dérivé, lorsqu'il est immunisé avec ledit complexe pMHC antigénique, et
B. capable d'exprimer à partir d'une cellule B ledit anticorps humain ou humanisé qui comprend :
(i) un domaine variable de chaîne lourde humain ou humanisé, exprimé à partir d'un locus de région variable de chaîne lourde d'immunoglobuline réarrangé formé à partir dudit locus de chaîne lourde d'immunoglobuline humaine ou humanisée non réarrangé ; et/ou
(ii) un domaine variable de chaîne légère humain ou humanisé, exprimé à partir d'un locus de région variable de chaîne légère d'immunoglobuline réarrangé formé à partir dudit locus de chaîne légère d'immunoglobuline humaine ou humanisée non réarrangé, et
dans lequel le rongeur est un rat ou une souris.

11. Procédé selon la revendication 10, dans lequel le procédé comprend :
(a)
(1) l'insertion de la séquence nucléotidique codant pour la molécule de MHC humain ou humanisé ou au moins son sillon de liaison peptidique humain dans un premier locus ectopique, ou
(2) le remplacement :
au niveau d'un locus de MHC de rongeur I, d'une séquence nucléotidique codant pour un polypeptide de MHC de rongeur I par une séquence nucléotidique codant pour un polypeptide de MHC humain ou humanisé I pour former une séquence nucléotidique codant pour la molécule de MHC humain ou humanisé, et/ou
au niveau d'un locus de MHC de rongeur II endogène, d'une séquence nucléotidique codant pour un polypeptide de MHC de rongeur II par une séquence nucléotidique codant pour un polypeptide de MHC humain ou humanisé II pour former une séquence nucléotidique codant pour la molécule de MHC humain ou humanisé, et
(b)
(i) l'insertion du locus de chaîne lourde d'immunoglobuline humaine ou humanisée non réarrangé et/ou du locus de chaîne légère d'immunoglobuline humaine ou humanisée non réarrangé dans un deuxième locus ectopique, ou
(ii) le remplacement :
(A) au niveau d'un locus de chaîne lourde de rongeur endogène, d'un segment de gène de variable de chaîne lourde (V_{H}) d'immunoglobuline de rongeur endogène par un segment de gène de variable de chaîne lourde (V_{H}) d'immunoglobuline humaine non réarrangé, et éventuellement le remplacement d'un segment de gène de diversité de chaîne lourde (D_{H}) d'immunoglobuline de rongeur endogène et/ou d'un segment de gène de j onction de chaîne lourde (J_{H}) d'immunoglobuline de rongeur endogène par un segment de gène de diversité de chaîne lourde (D_{H}) d'immunoglobuline humaine non réarrangé et/ou un segment de gène de jonction de chaîne lourde (J_{H}) d'immunoglobuline humaine non réarrangé, respectivement, dans lequel le segment de gène V_{H} humain non réarrangé et le segment de gène D_{H} humain non réarrangé éventuel et le segment de gène J_{H} humain non réarrangé éventuel, sont liés opérationnellement à une séquence de gène de région constante de chaîne lourde endogène, et/ou
(B) au niveau d'un locus de chaîne légère de rongeur endogène, d'un segment de gène de variable de chaîne légère (V_{L}) d'immunoglobuline de rongeur endogène et d'un segment de gène de jonction de chaîne légère (J_{L}) d'immunoglobuline de rongeur endogène par un segment de gène de variable de chaîne légère (V_{L}) d'immunoglobuline humaine et un segment de gène de jonction de chaîne légère (J_{L}) d'immunoglobuline humaine, qui sont éventuellement réarrangés pour former une séquence de gène V_{L}/J_{L}, dans lequel les segments de gène V_{L} humain et J_{L} humain sont liés opérationnellement à une séquence de gène de région constante de chaîne légère endogène,
dans lequel :
(A) la séquence nucléotidique (a)(1) codant pour la molécule de MHC humain ou humanisé ou au moins son sillon de liaison peptidique humain et le locus de chaîne lourde d'immunoglobuline humaine ou humanisée non réarrangé et/ou le locus de chaîne légère d'immunoglobuline humaine ou humanisée non réarrangé (b)(i) sont :
(I) insérés par recombinaison homologue séquentielle dans une seule cellule souche embryonnaire (ES) de rongeur ; ou
(II) insérés dans des première et deuxième cellules ES, dans lequel lesdites première et deuxième cellules ES sont utilisées respectivement pour générer des premier et deuxième rongeurs génétiquement modifiés ; ou
(B) la séquence nucléotidique codant pour le polypeptide de MHC de rongeur I et/ou la séquence nucléotidique codant pour le polypeptide de MHC de rongeur II (a)(2), et les segments de gène V_{H}, et éventuellement D_{H} et/ou J_{H} de rongeur endogènes (b)(ii), et/ou les segments de gène V_{L} et J_{L} sont :
(I) remplacés par recombinaison homologue séquentielle dans une seule cellule souche embryonnaire (ES) de rongeur ; ou
(II) remplacés dans des première et deuxième cellules ES, dans lequel lesdites première et deuxième cellules ES sont utilisées respectivement pour générer des premier et deuxième rongeurs génétiquement modifiés.

12. Procédé selon la revendication 10 ou la revendication 11, dans lequel le complexe pMHC antigénique est lié à un épitope de cellule T auxiliaire, éventuellement dans lequel l'épitope de cellule T auxiliaire est PADRE.

13. Procédé selon la revendication 11 (a)(2), dans lequel une molécule de MHC humanisé I comprend des domaines α1, α2, et α3 d'une molécule de HLA de classe I et au moins des domaines transmembranaires et cytoplasmiques d'un polypeptide de MHC de rongeur I endogène, et/ou dans lequel une molécule de MHC humanisé II comprend des domaines α1, α2, β1, et β2 d'une molécule de HLA de classe II et au moins des domaines transmembranaires et cytoplasmiques d'un polypeptide de MHC de rongeur II endogène.

14. Procédé selon la revendication 11(a)(2), qui comprend le ciblage de séquences codant pour un domaine extracellulaire de polypeptide de MHC endogène, la β2-microglobuline endogène, et des régions variables d'immunoglobuline endogène et leur remplacement par des séquences codant pour un domaine extracellulaire de polypeptide de MHC humain, la β2-microglobuline humaine, et des régions variables d'immunoglobuline humaine, respectivement.

15. Procédé de génération d'un anticorps humain ou humanisé qui se lie spécifiquement à un complexe pMHC antigénique d'intérêt ou une séquence d'acide nucléique codant pour celui-ci comprenant le maintien du rongeur génétiquement modifié selon l'une quelconque des revendications 1 à 9 ou produit selon le procédé de l'une quelconque des revendications 10 à 14 dans des conditions suffisantes pour permettre au rongeur génétiquement modifié de bâtir une réponse immunitaire au complexe pMHC antigénique d'intérêt, dans lequel le complexe pMHC antigénique d'intérêt comprend un peptide qui est hétérologue au rongeur génétiquement modifié et est présenté dans le contexte de la molécule de HLA ou au moins son sillon de liaison peptidique, éventuellement
comprenant en tant que première(s) étape(s) l'immunisation du rongeur génétiquement modifié avec le complexe pMHC antigénique d'intérêt, et éventuellement en outre l'amplification de la réponse immunitaire du rongeur génétiquement modifié, éventuellement dans lequel l'immunisation et/ou l'amplification comprennent l'administration au rongeur génétiquement modifié du complexe pMHC antigénique d'intérêt lié à un épitope de cellule T auxiliaire, éventuellement dans lequel l' épitope de cellule T auxiliaire comprend PADRE indiqué comme SEQ ID N° : 28.

16. Procédé d'obtention d'un acide nucléique codant pour un domaine variable de chaîne lourde d'immunoglobuline humaine et/ou un domaine variable de chaîne légère d'immunoglobuline humaine, comprenant :
l'isolement à partir d'un rongeur génétiquement modifié selon l'une quelconque des revendications 1 à 9 ou produit selon le procédé de l'une quelconque des revendications 10 à 14, d'un acide nucléique comprenant une séquence de gène de région variable d'immunoglobuline humaine réarrangée, éventuellement dans lequel la séquence de gène de région variable d'immunoglobuline humaine réarrangée comprend au moins une hypermutation somatique, qui code pour un domaine variable d'immunoglobuline humaine exprimé par un lymphocyte du rongeur génétiquement modifié, ou un hybridome produit à partir du lymphocyte,
dans lequel le domaine variable d'immunoglobuline humaine exprimé par le lymphocyte, ou l'hybridome produit à partir de celui-ci, s'associé à son domaine variable cognat pour former un domaine de liaison à l'antigène spécifique pour le complexe pMHC antigénique.

17. Procédé d'obtention d'une cellule qui exprime un domaine variable de chaîne lourde d'immunoglobuline humaine et/ou un domaine variable de chaîne légère d'immunoglobuline humaine comprenant :
l'isolement d'un lymphocyte à partir d'un rongeur génétiquement modifié selon l'une quelconque des revendications 1 à 9 ou produit selon le procédé de l'une quelconque des revendications 10 à 14,
dans lequel le lymphocyte exprime un domaine variable d'immunoglobuline humaine qui forme un domaine de liaison à l'antigène spécifique pour le complexe pMHC antigénique.

18. Procédé selon la revendication 17, comprenant en outre la production d'un hybridome à partir du lymphocyte isolé.

19. Procédé *in vitro* de fabrication d'un domaine variable d'immunoglobuline humaine comprenant :
l'expression, dans une cellule, d'un premier acide nucléique comprenant une séquence de gène de région variable d'immunoglobuline humaine réarrangée qui code pour un domaine variable d'immunoglobuline humaine exprimé par un lymphocyte d'un rongeur génétiquement modifié selon l'une quelconque des revendications 1 à 9 ou produit selon le procédé de l'une quelconque des revendications 10 à 14, ou un hybridome produit à partir du lymphocyte,
dans lequel le domaine variable d'immunoglobuline humaine exprimé par le lymphocyte, ou l'hybridome produit à partir de celui-ci, s'associe à son domaine variable cognat pour former un domaine de liaison à l'antigène spécifique pour le complexe pMHC antigénique,
éventuellement dans lequel le premier acide nucléique comprend en outre une séquence de gène de région constante d'immunoglobuline humaine liée opérationnellement à la séquence de gène de région variable d'immunoglobuline humaine,
éventuellement dans lequel la séquence de gène de région constante d'immunoglobuline humaine est une séquence de gène de région constante de chaîne lourde et comprend une modification qui accroît une affinité d'une région C_{H}2-C_{H}3 d'une séquence d'acides aminés de région constante de chaîne lourde d'IgG pour le récepteur Fc néonatal (FcRn) à un pH dans la plage de 5,5 à 6,0, dans lequel la modification est une mutation dans la séquence d'acides aminés de région constante de chaîne lourde d'IgG sélectionnée dans le groupe constitué par M428L, N434S, V259I, V308F, N434A, M252Y, S254T, T256E, T250Q, H433K, N434Y, et une combinaison de celles-ci.
